(19) Europäisches Patentamt
European Patent Office
Office européen des brevets

(11) EP 2 562 253 B1

(12) **EUROPEAN PATENT SPECIFICATION**

(45) Date of publication and mention of the grant of the patent:
**02.08.2017 Bulletin 2017/31**

(21) Application number: **11771784.3**

(22) Date of filing: **28.01.2011**

(51) Int Cl.:
***C12N 1/21*** *(2006.01)* ***C12N 5/10*** *(2006.01)*
***C12P 7/02*** *(2006.01)* ***C07D 333/16*** *(2006.01)*
***C07D 333/20*** *(2006.01)* ***C07D 453/02*** *(2006.01)*
***C12N 9/04*** *(2006.01)* ***C12P 17/12*** *(2006.01)*
***C12P 17/00*** *(2006.01)*

(86) International application number:
**PCT/JP2011/051771**

(87) International publication number:
**WO 2011/132444 (27.10.2011 Gazette 2011/43)**

(54) **MODIFIED CARBONYL REDUCTASE, GENE THEREOF, AND METHOD OF PRODUCING OPTICALLY ACTIVE ALCOHOLS USING THESE**

MODIFIZIERTE CARBONYLREDUKTASE, GEN DARAUS SOWIE VERFAHREN ZUR HERSTELLUNG OPTISCH AKTIVER ALKOHOLE DAMIT

CARBONYLE RÉDUCTASE MODIFIÉE, SON GÈNE, ET PROCÉDÉ DE PRODUCTION D'ALCOOLS OPTIQUEMENT ACTIFS LES METTANT EN UVRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2010 JP 2010098698**

(43) Date of publication of application:
**27.02.2013 Bulletin 2013/09**

(73) Proprietors:
- **KANEKA CORPORATION**
**Osaka (JP)**
- **National University Corporation Nara Institute of Science and Technology**
**Ikoma-shi, Nara 630-0192 (JP)**

(72) Inventors:
- **NISHIHACHIJYO Masakatsu**
**Takasago-shi**
**Hyogo 676-8688 (JP)**
- **IGUCHI Keita**
**Takasago-shi**
**Hyogo 676-8688 (JP)**
- **KAWANO Shigeru**
**Takasago-shi**
**Hyogo 676-8688 (JP)**
- **YASOHARA Yoshihiko**
**Takasago-shi**
**Hyogo 676-8688 (JP)**

(74) Representative: **Hoffmann Eitle**
**Patent- und Rechtsanwälte PartmbB**
**Arabellastraße 30**
**81925 München (DE)**

(56) References cited:
**WO-A1-2010/123062 WO-A1-2010/123062**
**WO-A1-2010/123067 JP-A- 2006 061 111**
**JP-A- 2006 061 111 US-A1- 2008 319 208**

- **YAMADA-ONODERA K ET AL.: "Purification and characterization of 2-aminoacetophenone reductase of newly isolated Burkholderia sp.", YT. J BIOSCI BIOENG., vol. 104, 2007, pages 416-419, XP22391309,**
- **DATABASE DDBJ/EMBL/GENBANK 22 November 2005 (2005-11-22), COPELAND, A. ET AL.: "Definition: Short-chain dehydrogenase/reductase SDR [Burkholderia sp. 383].", XP002700365, Database accession no. Q398R9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- UZURA A ET AL.: "Stereoselective synthesis of (R)-3-quinuclidinol through asymmetric reduction of 3-quinuclidinone with 3-quinuclidinone reductase of Rhodotorula rubra.", APPL MICROBIOL BIOTECHNOL., vol. 83, 21 February 2009 (2009-02-21), pages 617-626, XP19705546,
- DATABASE DDBJ/EMBL/GENBANK 31 March 2010 (2010-03-31), HOLDEN, M.T.G.: "Definition: putative short chain dehydrogenase [Burkholderia cenocepacia J2315].", XP002700665, Database accession no. B4EMW5 & M. T. G. Holden ET AL: "The Genome of Burkholderia cenocepacia J2315, an Epidemic Pathogen of Cystic Fibrosis Patients", Journal of Bacteriology, vol. 191, no. 1, 1 January 2009 (2009-01-01), pages 261-277, XP55069622, ISSN: 0021-9193, DOI: 10.1128/JB.01230-08
- DATABASE UniProt [Online] 24 March 2009 (2009-03-24), "SubName: Full=Short-chain dehydrogenase/reductase SDR;", XP002700658, retrieved from EBI accession no. UNIPROT:B9BH98 Database accession no. B9BH98 -& DATABASE UniProt [Online] 24 March 2009 (2009-03-24), "SubName: Full=Short-chain dehydrogenase/reductase SDR;", XP002700659, retrieved from EBI accession no. UNIPROT:B9AZ90 Database accession no. B9AZ90 & J. J. VARGA ET AL: "Draft Genome Sequence Determination for Cystic Fibrosis and Chronic Granulomatous Disease Burkholderia multivorans Isolates", JOURNAL OF BACTERIOLOGY, vol. 194, no. 22, 15 November 2012 (2012-11-15), pages 6356-6357, XP055068739, ISSN: 0021-9193, DOI: 10.1128/JB.01306-12
- DATABASE UniProt [Online] 20 March 2007 (2007-03-20), "SubName: Full=Dehydrogenase;", XP002700368, retrieved from EBI accession no. UNIPROT:A2WFQ0 Database accession no. A2WFQ0
- DATABASE UniProt [Online] 20 May 2008 (2008-05-20), "SubName: Full=Short-chain dehydrogenase/reductase SDR; Flags: Precursor;", XP002700369, retrieved from EBI accession no. UNIPROT:B1YY11 Database accession no. B1YY11
- LEUNG D W ET AL: "A METHOD FOR RANDOM MUTAGENESIS OF A DEFINED DNA SEGMENT USING A MODIFIED POLYMERASE CHAIN REACTION", TECHNIQUE, PHILADELPHIA, US, vol. 1, no. 1, 1 August 1989 (1989-08-01), pages 11-15, XP000614654, ISSN: 1043-4658
- YAMADA-ONODERA K ET AL.: 'Purification and characterization of 2-aminoacetophenone reductase of newly isolated Burkholderia sp.' YT. J BIOSCI BIOENG. vol. 104, no. 5, 2007, pages 416 - 419, XP022391309
- DATABASE DDBJ/EMBL/GENBANK [Online] 01 April 2010 COPELAND, A. ET AL.: 'Definition: Short-chain dehydrogenase/reductase SDR [Burkholderia sp. 383].', XP008163992 Database accession no. YP_371686
- UZURA A ET AL.: 'Stereoselective synthesis of (R)-3-quinuclidinol through asymmetric reduction of 3-quinuclidinone with 3-quinuclidinone reductase of Rhodotorula rubra.' APPL MICROBIOL BIOTECHNOL. vol. 83, 21 February 2009, pages 617 - 626, XP019705546
- NAITO R ET AL.: 'Synthesis and antimuscarinic properties of quinuclidin-3-yl 1,2,3,4-tetrahydroisoquinoline-2-carboxylate derivatives as novel muscarinic receptor antagonists.' J MED CHEM. vol. 48, 2005, pages 6597 - 6606, XP002435582
- DATABASE DDBJ/EMBL/GENBANK [Online] 31 March 2010 HOLDEN, M.T.G.: 'Definition: putative short chain dehydrogenase [Burkholderia cenocepacia J2315].', XP008163991 Database accession no. YP_002234590
- BROCK A ET AL.: 'The functional divergence of short-chain dehydrogenases involved in tropinone reduction.' PLANT J. vol. 54, 2008, pages 388 - 401, XP055068528

SEQUENCE LISTING

<110> \212\224\216®\211ï\216Ð\203J\2031\203J KANEKA CORPORATION
<110>
\215\221\227§\221å\212w\226@\2201\223Þ\227Ç\220æ\222[\211È\212w\213z\217p\221å\212w\211@\221å\212w NATIONAL UNIVERSITY CORPORATION NARA INSTITUTE OF SCIENCE AND TECHNOLOGY

<120>
\211ü\225Ï\214^\203J\203\213\203{\203j\203\213\212ò\214³\215y\221f\201A\202»
\202Ì\210â\223'\216q\201A\202¨\202æ\202Ñ\202»\202ê\202ç\202ð\227\230\227p\202
u\202½\214õ\212w\212\210\220«\203A\203\213\203R\201[\203\213\202Ì\220»\221¢ \225û\226@

<130> B100107W001

<150> JP 2010-098698
<151> 2010-04-22

<160> 13

<170> PatentIn version 3.1

<210> 1
<211> 263
<212> PRT
<213> Burkholderia sp.

<400> 1

```
Met Thr His Pro Gln Pro Arg Thr Ile Val Ile Thr Gly Ala Gly Thr
1               5                   10                  15

Gly Ile Gly Ala Ala Cys Ala Arg Arg Phe Ala Arg Arg Gly Asp Arg
            20                  25                  30

Val Val Leu Ile Gly Arg Arg Gln Ala Pro Leu Asp Ala Leu Ala Ala
        35                  40                  45

Glu Thr Gly Gly Leu Ala Leu Ala Gly Asp Ala Ala Ser Thr Ala Asp
    50                  55                  60

Trp Ala Gly Phe Leu Pro Arg Ile Ala Glu Arg Phe Gly Arg Val Asp
65                  70                  75                  80

Ala Leu Val Ala Cys Ala Gly Gly His Gly Leu Gly Arg Ala Asp Glu
                85                  90                  95

Thr Asp Asp Ala Gln Trp Arg Asp Ala Met His Ala Asn Leu Asp Thr
            100                 105                 110

Ala Phe Val Ser Ala Arg Ala Cys Leu Pro Asp Leu Ile Ala Gln Arg
        115                 120                 125

Gly Ser Ile Val Leu Val Ala Ser Ile Ala Ala Leu Ala Ala Gly Pro
    130                 135                 140
```

```
Ser Val Cys Gly Tyr Thr Val Gly Lys His Ala Leu Leu Gly Leu Ala
145                 150                 155                 160

Arg Ser Leu Ala Arg Asp Tyr Gly Pro His Gly Val Arg Ala Asn Ala
                165                 170                 175

Val Cys Pro Gly Trp Val Arg Thr Pro Met Ala Asp Ala Glu Met Glu
            180                 185                 190

Pro Leu Met Ser Ala His Gly Asp Thr Leu Asp Gly Ala Tyr Ala Arg
        195                 200                 205

Val Ser Ala Asp Val Pro Leu Arg Arg Ala Ala Asp Pro Asp Glu Ile
    210                 215                 220

Ala Ala Val Cys Ala Phe Leu Thr Ser Pro Asp Ala Ser Phe Val Thr
225                 230                 235                 240

Gly Ala Thr Leu Val Ala Asp Gly Gly Ala Met Val Val Asp Val Pro
                245                 250                 255

Thr Leu Ala Phe Asp Lys Leu
            260
```

<210> 2
<211> 792
<212> DNA
<213> Burkholderia sp.

<400> 2

atgacccacc cgcaaccccg cacgatcgtg atcaccggcg cgggcaccgg tatcggcgcc 60

gcgtgcgcgc gccgcttcgc ccgccgcggc gaccgcgtcg tgctgatcgg gcggcgccag 120

gcgccgctcg acgcgctggc cgccgaaacg ggcggcctcg cgctggccgg cgatgccgcg 180

agcaccgccg actgggccgg cttcctgccg cggatcgccg aacgcttcgg ccgcgtcgac 240

gcgctcgtcg cgtgcgcggg cggccacggc ctcggccgcg cggacgaaac cgacgacgcg 300

caatggcgcg acgcgatgca cgcgaacctc gacaccgcgt tcgtcagcgc gcgcgcgtgc 360

ctgcccgacc tgatcgcgca gcgcggcagc atcgtgctgg tcgcgtcgat cgccgcgctc 420

gcggccggcc ccagcgtgtg cggctatacg gtcggcaagc atgcgctgct cgggctcgcg 480

cggtcgctcg cacgcgacta cgggccgcac ggcgtgcggg cgaacgcggt atgccccggc 540

tgggtccgca cgccgatggc cgacgcggag atggagccgc tgatgtcggc acacggcgac 600

acgctcgacg gtgcgtatgc gcgcgtcagc gccgacgtgc cgctgcggcg cgcggcagac 660

ccggacgaga tcgcggccgt gtgcgcgttc ctcacgtcgc cggacgcgtc tttcgtgacc 720

ggcgcgacgc tcgtcgccga cggcggcgcg atggtcgtcg acgtgccgac gctcgcgttc 780

gacaagcttt ga 792

<210> 3
<211> 31
<212> DNA
<213> Artificial

<220>
<223> primer 1

<400> 3
ggataacaat ttcataagga ggttacatat g 31

<210> 4
<211> 23
<212> DNA
<213> Artificial

<220>
<223> primer 2

<400> 4
ctagaggatc cccgggtacc tta 23

<210> 5
<211> 30
<212> DNA
<213> Artificial

<220>
<223> primer 3

<400> 5
atatatacat atgacccacc cgcaaccccg 30

<210> 6
<211> 34
<212> DNA
<213> Artificial

<220>
<223> primer 4

<400> 6
tataggtacc ttatcaaagc ttgtcgaacg cgag 34

<210> 7
<211> 30
<212> DNA
<213> Artificial

<220>
<223> primer 5

<400> 7
atatatacat atgatccacc cgcaaccccg 30

<210> 8
<211> 30
<212> DNA
<213> Artificial

<220>
<223> primer 6

<400> 8
cggcgatccg cggcagggag ccggcccagt 30

<210> 9
<211> 30
<212> DNA
<213> Artificial

<220>
<223> primer 7

<400> 9
actgggccgg ctccctgccg cggatcgccg 30

<210> 10
<211> 30
<212> DNA
<213> Artificial

<220>
<223> primer 8

<400> 10
tataggtacc ttatcaaagc ttgtcgaacg 30

<210> 11
<211> 30
<212> DNA
<213> Artificial

<220>
<223> primer 9

<400> 11
atatatacat atgacccacc cgcaaccccg 30

<210> 12
<211> 43
<212> DNA
<213> Artificial

<220>
<223> primer 10

<400> 12
gccgaattct aaggaggtta acaatgtata aagatttaga agg 43

<210> 13
<211> 31
<212> DNA
<213> Artificial

<220>
<223> primer 11

<400> 13
ctgcaggtcg acttatccgc gtcctgcttg g 31

## Description

TECHNICAL FIELD

The present invention relates to a modified carbonyl reductase, to a gene therefor, to a vector comprising this gene, to a transformant transformed with this vector, and to a method for manufacturing an optically active alcohol using these.

BACKGROUND ART

One method that is known for manufacturing optically active alcohols, which are useful as synthetic raw materials and intermediates for drugs and agricultural chemicals, is by asymmetrically reducing the carbonyl groups of a carbonyl compound with a microorganism or enzyme. Enzymes that asymmetrically reduce carbonyl compounds (hereunder, carbonyl reductases) are useful in the manufacture of various optically active alcohols.

In an asymmetrical reduction reaction using a carbonyl reductase, the enzyme may be inactivated or the enzyme reaction may be inhibited by the substrate or product, by acids or alkalis used for pH adjustment, or by organic solvents or surfactants and the like that are added to improve the reaction solution properties. For example, according to Non Patent Literature 1 the activity of an asymmetrical reductase is greatly inhibited in the presence of even a small concentration of a compound containing a halogen atom.

By contrast, for example an enzyme produced by random mutagenesis and having resistance to organic solvents (Patent Literature 1) is known as a carbonyl reductase with improved functions.

UZURA A. et al., APPL MICROBIOL BIOTECHNOL., vol. 83, 2009-02-21, p. 617-626 relates to Stereoselective synthesis of (*R*) -3-quinuclidinol through asymmetric reduction of 3-quinuclidinone with 3-quinuclidinone reductase of *Rhodotorula rubra*.

Since compounds having halogen atoms are often included in substrates, products, acids, alkalis, surfactants and organic solvents, if a carbonyl reductase resistant to such enzyme inactivation and enzyme reaction inhibition could be provided, it would contribute to industrial production of optically active alcohols by helping to reduce reaction time and improve reaction yields.

CITATION LIST

- Patent Literature

- Patent Literature 1: Japanese Patent Application Publication No. 2006-61111

- Non Patent Literature

- Non Patent Literature 1: J. Biosci. Bioeng., 104, 416-419 (2007)

SUMMARY OF INVENTION

- Technical Problem

It is an object of the present invention to modify the wild-type enzyme, which has reduced reactivity in the presence of halogen atoms, so as to provide a modified carbonyl reductase having improved reactivity in the presence of halogen atoms in comparison with the wild-type enzyme. Another object is to provide a method for efficiently manufacturing an optically active alcohol using this enzyme or a transformant producing this enzyme.

- Solution to Problem

The inventors of the present invention have discovered a modified carbonyl reductase having improved reactivity in the presence of halogen atoms in comparison with the wild-type enzyme from a mutant enzyme library prepared by introducing random mutations into the wild-type enzyme gene, thereby completing the present invention.

That is, the present invention relates to a polypeptide having the following properties (a) to (c): (a) at least 97%

sequence identity with an amino acid sequence represented by SEQ ID NO:1 in the sequence listing, (b) production of (R)-3-quinuclidinol by reduction of 3-quinuclidinone or production of (S)-3-chloro-1-(2-thienyl)propan-1-ol by reduction of 3-chloro-1-(2-thienyl)propan-1-one, and (c) higher reactivity to carbonyl compounds in the presence of halogen atoms than a carbonyl reductase consisting of the amino acid sequence represented by SEQ ID NO:1 in the sequence listing.

The halogen atom is preferably a chlorine atom.

In the amino acid sequence represented by SEQ ID NO:1 in the sequence listing, preferably one or multiple amino acids selected from the group consisting of amino acids Nos. 2, 5, 8, 11, 23, 26, 28, 29, 30, 32, 33, 34, 40, 45, 46, 48, 61, 62, 64, 65, 66, 68, 71, 72, 75, 78, 79, 80, 81, 83, 88, 90, 95, 96, 100, 103, 105, 108, 115, 116, 119, 123, 128, 130, 133, 137, 141, 146, 152, 164, 168, 169, 170, 176, 177, 180, 189, 191, 196, 200, 201, 203, 211, 218, 225, 226, 229, 230, 231, 234, 240, 242 and 262 is substituted, and/or one or multiple amino acids are added to either the N or C terminus.

Preferably one or multiple amino acid substitutions and/or amino acid additions selected from the group consisting of: substitution of isoleucine or alanine for No. 2, arginine for No. 5, alanine for No. 8, valine or threonine for No. 11, threonine for No. 23, tyrosine for No. 26, histidine or leucine for No. 28, leucine for No. 29, serine for No. 30, histidine for No. 32, leucine for No. 33, methionine for No. 34, arginine for No. 40, serine, threonine, valine or proline for No. 45, methionine or isoleucine for No. 46, threonine for No. 48, glycine for No. 61, alanine or threonine for No. 62, threonine for No. 64, threonine for No. 65, valine for No. 66, serine or leucine for No. 68, glutamine for No. 71, phenylalanine for No. 72, cysteine or histidine for No. 75, histidine for No. 78, alanine for No. 79, tyrosine for No. 80, threonine for No. 81, alanine for No. 83, serine for No. 88, serine for No. 90, glycine for No. 95, aspartic acid or lysine for No. 96, threonine for No. 100, histidine for No. 103, threonine or valine for No. 105, threonine for No. 108, isoleucine for No. 115, threonine for No. 116, glutamic acid for No. 119, glycine for No. 123, histidine for No. 128, asparagine for No. 130, aspartic acid for No. 133, threonine for No. 137, valine for No. 141, alanine for No. 146, serine for No. 152, threonine for No. 164, arginine for No. 168, serine for No. 169, arginine for No. 170, valine for No. 176, alanine for No. 177, cysteine for No. 180, threonine for No. 189, isoleucine for No. 191, leucine for No. 196, valine for No. 200, alanine for No. 201, glycine for No. 203, valine for No. 211, valine for No. 218, serine for No. 225, threonine or valine for No. 226, valine for No. 229, isoleucine for No. 230, phenylalanine for No. 231, leucine for No. 234, alanine for No. 240, threonine for No. 242 and methionine for No. 262 and addition of the tryptophan and arginine to the C terminus is introduced, of the amino acid sequence represented by SEQ ID NO:1 in the sequence listing.

In addition to the properties (a) to (c) above, the polypeptide preferably has property of (d) which is greater affinity with a coenzyme than a carbonyl reductase consisting of the amino acid sequence represented by SEQ ID NO:1 in the sequence listing. From the standpoint of affinity with the coenzyme, the activity of (c) above is not necessary, and a polypeptide having the properties of (a), (b) and (d) above will have high affinity for the coenzyme.

Preferably one or multiple amino acids selected from the group consisting of amino acids Nos. 2, 46, 68 and 95 are substituted, and more preferably one or multiple amino acid substitutions selected from the group consisting of substitution of isoleucine for No. 2, methionine for No. 46, serine for No. 68 and glycine for No. 95 are introduced, of the amino acid sequence represented by SEQ ID NO:1 in the sequence listing.

The present invention also relates to a polynucleotide encoding this polypeptide.

The present invention also relates to a vector comprising this polynucleotide.

This vector preferably comprises a polynucleotide encoding a polypeptide having reductive coenzyme regeneration ability, and the polypeptide having reductive coenzyme regeneration ability is more preferably glucose dehydrogenase.

The present invention also relates to a transformant obtained by transforming a host cell with this vector.

This host cell is preferably *E. coli.*

The present invention also relates to a method of manufacturing an alcohol compound, wherein the polypeptide, the transformant and/or its treated product is applied to a carbonyl compound.

This carbonyl compound is preferably an asymmetric ketone, and the product is preferably an optically active alcohol.

The compound having a carbonyl group is preferably an asymmetric ketone represented by Formula (1) below:

[Chem. 1]

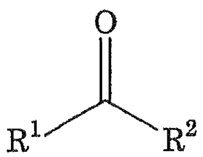

(1)

(where $R^1$ and $R^2$ are hydrogen atoms, halogen atoms, optionally substituted alkyl groups, optionally substituted aralkyl groups, optionally substituted aryl groups, optionally substituted alkoxy groups, amino groups or nitro groups, or else

$R^1$ and $R^2$ bind with each other to form a ring; but $R^1$ and $R^2$ have different structures), and the product is preferably an optically active alcohol represented by Formula (2) below:

[Chem. 2]

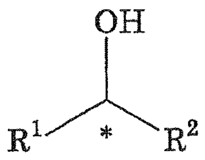

(2)

(where $R^1$ and $R^2$ are as described above, and * represents an asymmetric carbon).

This compound having a carbonyl group is preferably 3-quinuclidinone or a salt thereof, 3-chloro-1-(2-thienyl)propan-1-one, or 3-chloro-1-(2-phenyl)propan-1-one.

The present invention also relates to a method for manufacturing a bulk pharmaceutical, the method including a step in which the polypeptide, the transformant and/or its treated product is applied to a carbonyl compound to produce an optically active alcohol.

The optically active alcohol is preferably (R)-3-quinuclidinone while the bulk pharmaceutical is solifenacin, or the optically active alcohol is (S)-3-chloro-1-(2-thienyl)propan-1-ol while the bulk pharmaceutical is duloxetine.

- Advantageous Effects of Invention

The present invention provides a modified carbonyl reductase and gene therefor having improved reactivity in the presence of halogen atoms in comparison with the wild type, a vector comprising the gene, a transformant transformed with the vector, a treated product of the transformant, and a method for efficiently manufacturing an optically active alcohol using these.

(BRIEF DESCRIPTION OF DRAWINGS)

FIG. 1 shows the structures of the recombinant vectors of Reference Example 1 and Reference Example 2 of the present invention.

FIG. 2 is a Lineweaver-Burk plot showing the measurement results of Comparative Example 4.

DESCRIPTION OF EMBODIMENTS

The polypeptide of the present invention has the following properties (a) to (c): (a) having at least 97% sequence identity with an amino acid sequence represented by SEQ ID NO:1 in the sequence listing, (b) producing (R)-3-quinuclidinol by reduction of 3-quinuclidinone or producing (S)-3-chloro-1-(2-thienyl)propan-1-ol by reduction of 3-chloro-1-(2-thienyl)propan-1-one, and (c) having higher reactivity to carbonyl compounds in the presence of halogen atoms in comparison with a carbonyl reductase consisting of the amino acid sequence represented by SEQ ID NO:1 in the sequence listing.

[Mutation description conventions]

In this Description, amino acids, peptides and proteins are represented by the abbreviations shown below, which have been adopted by the IUPAC-IUB Commission on Biochemical Nomenclature (CBN). Unless otherwise indicated, the sequences of amino acid residues in peptides and proteins are represented from the N terminus on the left to the C terminus on the right. The following commonly used naming conventions are also adopted for ease of reference. One is a method of giving the original amino acid, the position and the substituted amino acid in that order, such as by using "Y84D" to represent the substitution of aspartic acid for tyrosine at the 64 position. Multiplex variations are represented by the hyphenation "-" of two words. For example, "S41A-Y64D" means that alanine has been substituted for serine at the 41 position and aspartic acid has been substituted for tyrosine at the 64 position.

[Amino acid abbreviations]

A = Ala = alanine, C = Cys = cysteine,

D = Asp = aspartic acid, E = Glu = glutamic acid, F = Phe = phenylalanine, G = Gly = glycine,
H = His = histidine, I = Ile = isoleucine,
K = Lys = lysine, L = Leu = leucine,
M = Met = methionine, N = Asn = asparagine,
P = Pro = proline, Q = Gln = glutamine,
R = Arg = arginine, S = Ser = serine,
T = Thr = threonine, V = Val = valine,
W = Trp = tryptophan, Y = Tyr = tyrosine

[Sequence identity]

To determine the "sequence identity" of polypeptides and polynucleotides, the two polypeptides or polynucleotides being compared are optimally aligned, and the number of positions at which the amino acids or nucleotides (such as A, T, C, G, U or I) are the same in both is divided by the number of compared nucleic acids, and multiplied by 100.

Sequence identity can be calculated for example using the following sequence analysis tools: GCG Wisconsin Package (University of Wisconsin), the ExPASy World Wide Web molecular biology server (Swiss Institute of Bioinformatics), BLAST (National Center for Biotechnology Information), or GENETYX (Genetyx Corp.).

In the present invention, the wild-type enzyme before mutagenesis consists of the 263 amino acid residues represented by SEQ ID NO:1 in the sequence listing, and is a polypeptide that has the ability to produce (R)-3-quinuclidinol by reduction of 3-quinuclidinone or to produce (S)-3-chloro-1-(2-thienyl)propan-1-ol by reduction of 3-chloro-1-(2-thienyl)propan-1-one.

The polypeptide is not particularly restricted as to origin, but is preferably a carbonyl reductase from a microorganism in the genus *Burkholderia,* or more preferably from the *Burkholderia sp.* YT strain. This strain is a microorganism discovered by Onodera et al., and deposited on August 18, 2008 at the National Institute of Technology and Evaluation (NITE) Patent Microorganisms Depository, (2-5-8 Kazusakamatari, Kisarazu-shi, Chiba-ken, 292-0818) under the accession number NITE P-613. The reaction of the wild-type enzyme is known to be inhibited by chlorides (J. Biosci. Bioeng., 104, 416-419 (2007)).

The wild-type enzyme of the present invention is encoded by the polynucleotide represented by SEQ ID NO:2 in the sequence listing. This polynucleotide can be obtained from *Burkholderia* and especially from the *Burkholderia sp.* YT strain by ordinary genetic engineering methods as described in Molecular Cloning 2nd Edition (Joseph Sambrook, Cold Spring Harbor Laboratory Press (1989)) and the like for example.

That is, a polynucleotide encoding the amino acid sequence represented by SEQ ID NO:1, or a polynucleotide represented by SEQ ID NO:2, can be amplified by PCR using the methods described below (Reference Example 1) from genome DNA of the *Burkholderia sp.* YT strain to prepare a wild-type enzyme gene.

The polypeptide of the present invention may also be one that has been obtained by adding modifications to the amino acid sequence represented by SEQ ID NO:1.

Modifications added to the amino acid sequence represented by SEQ ID NO:1 may be substitutions, additions, insertion and deletions, and may include only one kind of modification (such as substitution), or 2 or more kinds of modifications (such as substitution and insertion). The aforementioned "multiple amino acids" mean 40 amino acids, or preferably 20, or more preferably 10, or still more preferably 8, 5, 4, 3 or 2 amino acids.

The sequence identity between an amino acid sequence with added modifications and the amino acid sequence represented by SEQ ID NO:1 is at least 97%, at least 98%, at least 98.5% or at least 99%.

A carbonyl reductase with added modifications is preferably an enzyme consisting of a polypeptide that has the amino acid sequence represented by SEQ ID NO:1 with at least 1 or else multiple amino acids substituted, added, inserted or deleted therein, and that has 97% or greater sequence homology with a carbonyl reductase of the amino acid sequence represented by SEQ ID NO:1 in the sequence listing.

The locations of the amino acid substitutions, insertions, deletions or additions in the amino acid sequence represented by SEQ ID NO:1 in the sequence listing is not particularly limited, but preferably the polypeptide has one or more than one of amino acids Nos. 2, 5, 8, 11, 23, 26, 28, 29, 30, 32, 33, 34, 40, 45, 46, 48, 61, 62, 64, 65, 66, 68, 71, 72, 75, 78, 79, 80, 81, 83, 88, 90, 95, 96, 100, 103, 105, 108, 115, 116, 119, 123, 128, 130, 133, 137, 141, 146, 152, 164, 168, 169, 170, 176, 177, 180, 189, 191, 196, 200, 201, 203, 211, 218, 225, 226, 229, 230, 231, 234, 240, 242 and 262 substituted, and/or one or multiple amino acids added to either the N terminus or the C terminus of the amino acid sequence represented by SEQ ID NO:1 in the sequence listing.

More preferably, this is a polypeptide comprising one or multiple amino acid substitutions and/or amino acid additions selected from the group consisting of: substitution of isoleucine or alanine for No. 2, arginine for No. 5, alanine for No. 8, valine or threonine for No. 11, threonine for No. 23, tyrosine for No. 26, histidine or leucine for No. 28, leucine for No. 29, serine for No. 30, histidine for No. 32, leucine for No. 33, methionine for No. 34, arginine for No. 40, serine, threonine, valine or proline for No. 45, methionine or isoleucine for No. 46, threonine for No. 48, glycine for No. 61,

alanine or threonine for No. 62, threonine for No. 64, threonine for No. 65, valine for No. 66, serine or leucine for No. 68, glutamine for No. 71, phenylalanine for No. 72, cysteine or histidine for No. 75, histidine for No. 78, alanine for No. 79, tyrosine for No. 80, threonine for No. 81, alanine for No. 83, serine for No. 88, serine for No. 90, glycine for No. 95, aspartic acid or lysine for No. 96, threonine for No. 100, histidine for No. 103, threonine or valine for No. 105, threonine for No. 108, isoleucine for No. 115, threonine for No. 116, glutamic acid for No. 119, glycine for No. 123, histidine for No. 128, asparagine for No. 130, aspartic acid for No. 133, threonine for No. 137, valine for No. 141, alanine for No. 146, serine for No. 152, threonine for No. 164, arginine for No. 168, serine for No. 169, arginine for No. 170, valine for No. 176, alanine for No. 177, cysteine for No. 180, threonine for No. 189, isoleucine for No. 191, leucine for No. 196, valine for No. 200, alanine for No. 201, glycine for No. 203, valine for No. 211, valine for No. 218, serine for No. 225, threonine or valine for No. 226, valine for No. 229, isoleucine for No. 230, phenylalanine for No. 231, leucine for No. 234, alanine for No. 240, threonine for No. 242 and methionine for No. 262 and addition of the tryptophan and arginine to the C terminus, introduced in the amino acid sequence represented by SEQ ID NO:1 in the sequence listing.

From the standpoint of improving stability in the presence of chloride ions, the polypeptide of the present invention preferably comprises one or multiple amino acid substitutions selected from the group consisting of: substitution of isoleucine for No. 2, tyrosine for No. 26, histidine for No. 28, methionine for No. 46, glycine for No. 61, serine for No. 68, tyrosine for No. 80, threonine for No. 81, alanine for No. 83, glycine for No. 95, lysine for No. 96, glutamic acid for No. 119, glycine for No. 123, asparagine for No. 130, serine for No. 152, leucine for No. 196, valine for No. 200, isoleucine for No. 230 and leucine for No. 234, introduced into the amino acid sequence represented by SEQ ID NO:1 in the sequence listings.

A polypeptide having an introduced amino acid substitution or substitutions selected from the following (1) to (17): (1) isoleucine for No. 2 and methionine for No. 46, (2) arginine for No. 5, threonine for No. 23, threonine for No. 100 and lysine for No. 201, (3) tyrosine for No. 26, (4) histidine for No. 28, glycine for No. 61 and leucine for No. 234, (5) serine for No. 68, (6) tyrosine for No. 80 and asparagine for No. 130, (7) threonine for No. 81, (8) alanine for No. 83, (9) serine for No. 88, (10) glycine for No. 95, (11) lysine for No. 96, (12) threonine for No. 105 and valine for No. 226, (13) glutamic acid for No. 119 and leucine for No. 196, (14) glycine for No. 123, (15) serine for No. 152, (16) valine for No. 200 and (17) isoleucine for No. 230 is still more preferred.

Moreover, from the standpoint of improved resistance to reaction inhibition by chloride ions, the polypeptide of the present invention preferably has one or multiple amino acid substitutions and/or amino acid additions selected from the group consisting of the following: substitution of isoleucine or alanine for No. 2, arginine for No. 5, alanine for No. 8, threonine for No. 23, tyrosine for No. 26, histidine or leucine for No. 28, leucine for No. 29, serine for No. 30, histidine for No. 32, leucine for No. 33, methionine for No. 34, arginine for No. 40, serine, threonine, valine or proline for No. 45, methionine or isoleucine for No. 46, threonine for No. 48, glycine for No. 61, alanine or threonine for No. 62, threonine for No. 65, valine for No. 66, serine or leucine for No. 68, glutamine for No. 71, phenylalanine for No. 72, cysteine or histidine for No. 75, histidine for No. 78, alanine for No. 79, tyrosine for No. 80, threonine for No. 81, serine for No. 88, serine for No. 90, glycine for No. 95, aspartic acid or lysine for No. 96, threonine for No. 100, histidine for No. 103, valine for No. 105, threonine for No. 108, isoleucine for No. 115, threonine for No. 116, glycine for No. 123, histidine for No. 128, asparagine for No. 130, aspartic acid for No. 133, threonine for No. 137, valine for No. 141, alanine for No. 146, serine for No. 152, threonine for No. 164, arginine for No. 168, serine for No. 169, arginine for No. 170, valine for No. 176, alanine for No. 177, cysteine for No. 180, threonine for No. 189, isoleucine for No. 191, valine for No. 200, glycine for No. 203, valine for No. 211, valine for No. 218, serine for No. 225, threonine for No. 226, valine for No. 229, isoleucine for No. 230, phenylalanine for No. 231, leucine for No. 234, alanine for No. 240, threonine for No. 242 and methionine for No. 262 and addition of tryptophan and arginine to the C terminus, introduced in the amino acid sequence represented by SEQ ID NO:1 in the sequence listing.

It is more desirable to have an introduced amino acid addition and/or amino acid substitution or substitutions selected from the following (1) to (55): (1) alanine for No. 2, arginine for No. 40 and valine for No. 229, (2) isoleucine for No. 2, (3) isoleucine for No. 2, valine for No. 11, methionine for No. 46 and threonine for No. 64, (4) isoleucine for No. 2, threonine for No. 11 and methionine for No. 46, (5) isoleucine for No. 2, leucine for No. 28 and methionine for No. 46, (6) isoleucine for No. 2, leucine for No. 29, histidine for No. 32 and methionine for No. 46, (7) isoleucine for No. 2, histidine for No. 32 and methionine for No. 46, (8) isoleucine for No. 2, threonine for No. 45 and methionine for No. 46, (9) isoleucine for No. 2, valine for No. 45 and methionine for No. 46, (10) isoleucine for No. 2, proline for No. 45, methionine for No. 46 and arginine for No. 168, (11) isoleucine for No. 2, serine for No. 45, methionine for No. 46 and serine for No. 90, (12) isoleucine for No. 2 and isoleucine for No. 46, (13) isoleucine for No. 2 and methionine for No. 46, (14) isoleucine for No. 2, methionine for No. 46 and threonine for No. 48, (15) isoleucine for No. 2, methionine for No. 46 and threonine for No. 62, (16) isoleucine for No. 2, methionine for No. 46 and threonine for No. 65, (17) isoleucine for No. 2, methionine for No. 46 and valine for No. 66, (18) isoleucine for No. 2, methionine for No. 46 and serine for No. 68, (19) isoleucine for No. 2, methionine for No. 46 and histidine for No. 78, (20) isoleucine for No. 2, methionine for No. 46 and alanine for No. 177, (21) arginine for No. 5, threonine for No. 23, threonine for No. 100 and lysine for No. 201, (22) alanine for No. 8, (23) histidine for No. 28, glycine for No. 61 and leucine for No. 234, (24) histidine for No. 28, alanine for No. 62, alanine

for No. 79 and threonine for No. 164, (25) cysteine for No. 29, (26) serine for No. 30 and phenylalanine for No. 231, (27) leucine for No. 33, (28) methionine for No. 34, (29) methionine for No. 46, (30) methionine for No. 46 and serine for No. 68, (31) alanine for No. 62, isoleucine for No. 115, valine for No. 141, serine for No. 169, cysteine for No. 180, threonine for No. 242 and methionine for No. 262, (32) serine for No. 68, (33) leucine for No. 68 and valine for No. 105, (34) glutamine for No. 71, (35) phenylalanine for No. 72 and aspartic acid for No. 133, (36) cysteine for No. 75, threonine for No. 137 and glycine for No. 203, (37) histidine for No. 75 and threonine for No. 226, (38) tyrosine for No. 80 and asparagine for No. 130, (39) threonine for No. 81, (40) alanine for No. 83, (41) alanine for No. 83 and threonine for No. 176, (42) serine for No. 88, (43) serine for No. 90, (44) glycine for No. 95, (45) aspartic acid for No. 96 and arginine for No. 170, (46) histidine for No. 103, threonine for No. 189 and isoleucine for No. 191, (47) threonine for No. 108, alanine for No. 146, threonine for No. 164 and valine for No. 218, (48) threonine for No. 116 and histidine for No. 128, (49) glutamic acid for No. 119 and leucine for No. 196, (50) glycine for No. 123, (51) serine for No. 152, (52) alanine for No. 201, (53) valine for No. 211 and tryptophan for No. 264, (54) serine for No. 225 and arginine for No. 264 and (55) alanine for No. 240.

From the standpoint of improving stability in the presence of an organic chloride, the polypeptide of the present invention preferably has one or multiple amino acid substitutions and/or amino acid additions selected from the group consisting of: substitution of isoleucine for No. 2, alanine for No. 8, valine or threonine for No. 11, tyrosine for No. 26, histidine for No. 28, histidine for No. 32, leucine for No. 33, methionine for No. 34, serine or threonine for No. 45, methionine or isoleucine for No. 46, threonine for No. 48, glycine for No. 61, threonine for No. 62, threonine for No. 64, threonine for No. 65, valine for No. 66, serine for No. 68, glutamine for No. 71, cysteine or histidine for No. 75, histidine for No. 78, alanine for No. 79, tyrosine for No. 80, threonine for No. 81, alanine for No. 83, serine for No. 88, serine for No. 90, glycine for No. 95, aspartic acid or lysine for No. 96, threonine for No. 116, glutamic acid for No. 119, glycine for No. 123, histidine for No. 128, asparagine for No. 130, threonine for No. 137, serine for No. 152, arginine for No. 170, valine for No. 176, alanine for No. 177, leucine for No. 196, valine for No. 200, glycine for No. 203, serine for No. 225, threonine for No. 226, isoleucine for No. 230 and leucine for No. 234, and addition of arginine to the C terminus, introduced into the amino acid sequence represented by SEQ ID NO:1 in the sequence listing.

Moreover, it is more desirable to have an amino acid substitution selected from the following (1) to (44): (1) substitution of alanine for No. 2, arginine for No. 40 and valine for No. 229, (2) isoleucine for No. 2, valine for No. 11, methionine for No. 46 and threonine for No. 64, (3) isoleucine for No. 2, threonine for No. 11 and methionine for No. 46, (4) isoleucine for No. 2, leucine for No. 28 and methionine for No. 46, (5) isoleucine for No. 2, leucine for No. 29, histidine for No. 32 and methionine for No. 46, (6) isoleucine for No. 2, histidine for No. 32 and methionine for No. 46, (7) isoleucine for No. 2, threonine for No. 45 and methionine for No. 46, (8) isoleucine for No. 2, valine for No. 45 and methionine for No. 46, (9) isoleucine for No. 2, proline for No. 45, methionine for No. 46 and arginine for No. 168, (10) isoleucine for No. 2, serine for No. 45, methionine for No. 46 and serine for No. 90, (11) isoleucine for No. 2 and isoleucine for No. 46, (12) isoleucine for No. 2 and methionine for No. 46, (13) isoleucine for No. 2, methionine for No. 46 and threonine for No. 48, (14) isoleucine for No. 2, methionine for No. 46 and threonine for No. 62, (15) isoleucine for No. 2, methionine for No. 46 and threonine for No. 65, (16) isoleucine for No. 2, methionine for No. 46 and valine for No. 66, (17) isoleucine for No. 2, methionine for No. 46 and serine for No. 68, (18) isoleucine for No. 2, methionine for No. 46 and histidine for No. 78, (19) isoleucine for No. 2, methionine for No. 46 and alanine for No. 177, (20) alanine for No. 8, (21) histidine for No. 28, glucine for No. 61 and leucine for No. 234, (22) histidine for No. 28, alanine for No. 62, alanine for No. 79 and threonine for No. 164, (23) cysteine for No. 29, (24) leucine for No. 33, (25) methionine for No. 34, (26) methionine for No. 46 and serine for No. 68, (27) serine for No. 68, (28) glutamine for No. 71, (29) cysteine for No. 75, threonine for No. 137 and glycine for No. 203, (30) histidine for No. 75 and threonine for No. 226, (31) tyrosine for No. 80 and asparagine for No. 130, (32) threonine for No. 81, (33) alanine for No. 83, (34) alanine for No. 83 and valine for No. 176, (35) serine for No. 88, (36) serine for No. 90, (37) glycine for No. 95, (38) aspartic acid for No. 96 and arginine for No. 170, (39) threonine for No. 116 and histidine for No. 128, (40) glutamic acid for No. 119 and leucine for No. 196, (41) glycine for No. 123, (42) serine for No. 152, (43) alanine for No. 201 and (44) serine for No. 225 and arginine for No. 264, introduced into the amino acid sequence represented by SEQ ID NO:1 in the sequence listing.

The organic chloride is preferably a chloroketone, and more preferably is either chloroacetone or 3-chloro-1-(2-thienyl)propan-1-one.

From the standpoint of affinity with a coenzyme, the polypeptide of the present invention preferably has 1 or more amino acids selected from amino acids Nos. 2, 46, 68 and 95 substituted in the amino acid sequence represented by SEQ ID NO:1 in the sequence listing.

More preferably, 1 or more amino acid substitutions selected from the following group: isoleucine for No. 2, methionine for No. 46, serine for No. 68 and glycine for No. 95, are introduced into the amino acid sequence represented by SEQ ID NO:1 in the sequence listing.

Stability in the presence of chloride ions, resistance to reaction inhibition by chloride ions and stability in the presence of organic chlorides are discussed below.

The "activity of producing (R)-3-quinuclidinol by reduction of 3-quinuclinone" in the present invention means that the optical purity of (R)-3-quinuclidinol as determined by the following evaluation method may be 85% e.e. or more,

and is preferably 90% e.e. or more, or more preferably 95% e.e. or more. Most preferably it is 96, 97, 98 or 99% e.e. or more.

[Methods for evaluating ability to produce (R)-3-quinuclidinol)

The polypeptide of the present invention is added to a 0.5 M phosphate buffer (pH 6.5) containing 0.1 M 3-quinuclidinone hydrochloride and 0.1 M reduced nicotinamide adenine dinucleotide phosphate (hereunder, NADPH), and reacted at 30°C. After the reaction, extraction operations are performed with dichloromethane and other organic solvents, and the steric configuration and optical purity of the resulting 3-quinuclidinol are confirmed by analysis under the following gas chromatography conditions.

(Gas chromatography analysis conditions)

| | |
|---|---|
| Column: | Gamma DEX (30 m, i.d. 0.25 mm, Supelco) |
| Column temperature: | 150°C |
| Inlet temperature: | 220°C |
| Detector temperature: | 220°C |
| Detection: | FID |
| Carrier gas: | Helium, flow volume: 100 kPa |
| Retention times: | 3-quinuclidinone about 9.8 min., (S)-3-quinuclidinol about 11.5 min., (R)-3-quinuclidinol about 12.0 min. |

The optical purity of optically active 3-quinuclidinol can be determined by the following formula:

Optical purity of R enantiomer (% e.e.) = {(peak area of R enantiomer) - (peak area of S enantiomer)} / {peak area of R enantiomer) + (peak area of S enantiomer)} × 100.

The activity of producing (S)-3-chloro-1-(2-thienyl)propan-1-ol by reduction of 3-chloro-1-(2-thienyl)propan-1-one can be evaluated by the following method. The "activity of producing (S)-3-chloro-1-(2-thienyl)propan-1-ol by reduction of 3-chloro-1-(2-thienyl)propan-1-one" in the present invention means that the optical purity of (S)-3-chloro-1-(2-thienyl)propan-1-ol as determined by this evaluation method may be at least 85% e.e., and is preferably at least 90% e.e., or more preferably at least 95% e.e., or most preferably 96, 97, 98 or 99% e.e.

(Method of evaluating ability to produce (S)-3-chloro-1-(2-thienyl)propan-1-ol)

The polypeptide of the present invention is added to 0.5 M phosphate buffer (pH 6.5) containing 10 mM 3-chloro-1-(2-thienyl)propan-1-one, 1% dimethyl sulfoxide and 10 mM NADPH, and reacted at 30°C. After the reaction, extraction operations are performed with dichloromethane and other organic solvents, and the steric configuration and optical purity of the resulting 3-chloro-1-(2-thienyl)propan-1-ol are confirmed by analysis under the following conditions.

[Analysis conditions for high-performance liquid chromatography]

| | |
|---|---|
| Column: | Chiralcel OD-H (250 mm, i.d. 4.6 μm, Daicel Corporation) |
| Column temperature: | 30°C |
| Detection wavelength: | 240 nm |
| Mobile phase: | n-hexane/isopropanol = 97.5/2.5 |
| Retention times: | (S)-3-chloro-1-(2-thienyl)propan-1-ol about 19.9 min., (R)-3-chloro-1-(2-thienyl)propan-1-ol about 21.5 min., 3-chloro-1-(2-thienyl)propan-1-one about 12.2 min. |

The optical purity of (S)-3-chloro-1-(2-thienyl)propan-1-ol can be determined by the following formula:

Optical purity of S enantiomer (% e.e.) = {(peak area of S enantiomer) - (peak area of R enantiomer)} / {(peak area of S enantiomer) + (peak area of R enantiomer)} × 100.

"In the presence of halogen atoms" means that the enzyme of the present invention is present together with a compound containing any one halogen atom or multiple halogen atoms. Halogen atoms are fluorine atoms, chlorine atoms, bromine atoms, ioidine atoms or astatine atoms for example. Halogen atoms are preferably chlorine atoms. More preferably, they are one or more of molecular chlorine ($Cl_2$), chloride ions (Cl-), or inorganic chlorides or organic chlorides.

Preferred examples of inorganic chlorides include aluminum chloride, ammonium chloride (sodium chloride), potassium chloride, calcium chloride, tin chloride, cesium chloride, thionyl chloride, sodium chloride, nitrosyl chloride, barium chloride, benzenediazonium chloride, magnesium chloride, lithium chloride, zinc chloride, lead chloride, chloroauric (III) acid, silver chloride, mercury chloride, magnesium chloride hydroxide, copper chloride hydroxide (II), hydrogen chloride (hydrochloric acid), iron chloride, chloroplatinic (VI) acid, chloric acid, potassium chlorate, perchloric acid, lithium perchlorate and the like, and hydrochloric acid or sodium chloride is especially desirable.

An organic chloride is preferably an alkane, alkene, alkyne, cycloalkane, aryl or heteroaryl compound having one or more chlorine atoms as substituents, or more preferably a chloroalcohol compound or chloroketone compound, or most preferably 3-chloro-1-(2-thienyl)propan-1-ol, 3-chloro-1-(2-thienyl)propan-1-one, chloroacetone, phenacyl chloride or m-chlorophenacyl chloride.

When a chloride has acidity or alkalinity, it may have its pH adjusted with a suitable acid or alkali, or may be in the form of a salt of the acidic or basic chloride with an organic or inorganic compound. 3-quinuclidinone hydrochloride is preferred.

For a chloride and enzyme to be present together does not necessarily mean that the chloride must be dissolved in a liquid containing the enzyme, and instead a liquid containing the enzyme and a liquid containing the chloride, or a liquid containing the enzyme and the chloride in solid form or the like, may be in an inhomogenous state, or may have been physically agitated or mixed.

An "enzyme having high reactivity in the presence of halogen atoms" means that the reductive activity of the enzyme with respect to 3-quinuclidinone or 3-chloro-1-(2-thienyl)propan-1-one is higher in the presence of halogen atoms or after a certain period of treatment in the presence of such halogen atoms than that of the wild-type enzyme represented by SEQ ID NO:1 in the sequence listing. Preferably, the enzyme is highly stable with respect to chlorides, or is highly resistant to reaction inhibition by chlorides.

For an enzyme to have high stability with respect to chlorides means specifically that the remaining activity with respect to 3-quinuclidinone after incubation with sodium chloride is at least 1% higher or preferably at least 5% higher or more preferably at least 10% higher or most preferably at least 20% higher than that of the wild-type enzyme as measured by the methods described in Example 12 below.

For an enzyme to have high resistance to reaction inhibition by chlorides means specifically that relative activity with respect to 3-quinuclidinone in the presence of sodium chloride is at least 1% higher or preferably at least 5% higher or more preferably at least 10% higher or most preferably at least 20% higher than that of the wild-type enzyme as measured by the methods described in Example 14 below.

The stability of an enzyme with respect to chlorides can be evaluated for example by the following methods.

[Methods for evaluating enzyme stability with respect to chlorides]

A buffer (preferably pH 5 to 8, 0.01 to 1 M phosphate buffer) containing any concentration (such as 0.01% to 50%) of a chloride is added to a cell-free extract containing the enzyme, and incubated at any temperature (such as 4 to 40°C). If the chloride and buffer are not homogenous, they are shaken or agitated during incubation. This is sampled immediately after addition of the chloride (0 hours) and after any treatment time (0.1 to 48 hours) and diluted with an 0.1 M aqueous solution of potassium phosphate (pH 7.0), and enzyme activity is measured by the following "Methods for evaluating ability to reduce carbonyl compounds" using the diluted solution. Remaining activity can be calculated by the following formula:

Remaining activity (%) = [enzyme activity after any treatment time] / [enzyme activity at 0 hours] × 100.

A modified carbonyl reductase that is more stable with respect to chlorides than the carbonyl reductase represented by SEQ ID NO:1 in the sequence listing is an enzyme the remaining activity of which as evaluated by this method is at least 1% greater than that of the wild-type enzyme, or preferably at least 5% or more preferably at least 10% or most preferably at least 20% greater than that of the wild-type enzyme.

[Methods for evaluating ability to reduce carbonyl compounds]

A reaction liquid containing 0.25 mM of NADPH or reduced nicotinamide adenine dinucleotide (hereunder, NADH), 1 to 50 mM of a carbonyl compound the reduction of which is to be evaluated (such as 3-quinuclidinone or 3-chloro-1-(2-thienyl)propan-1-one) and the polypeptide of the present invention in a 100 mM potassium phosphate buffer solution containing 0.3% (v/v) dimethyl sulfoxide is reacted at 30°C, and the progress of the reducing reaction can be easily evaluated by measuring the reduction in absorbancy at 340 nm that occurs as the amount of NADPH or NADH is reduced. When absorbancy is reduced, it can be judged that the polypeptide of the present invention has the ability to reduce the carbonyl compound under evaluation. The more rapid the rate of reduction in absorbancy, the greater the reducing ability with respect to the carbonyl compound under evaluation. The reducing ability of the polypeptide can also be quantified, with 1 U of reducing ability representing the amount of enzyme that catalyzes digestion of 1 μmol of NADPH in 1 minute.

Resistance to reaction inhibition by chlorides can be determined as follows for example.

[Method of evaluating resistance to reaction inhibition by chlorides]

First, the activity of the enzyme is measured by the aforementioned "Methods for evaluating ability to reduce carbonyl compounds" under conditions with no chlorides present. The result is "enzyme activity without chloride". Next, activity is measured in the same way with 100 mM of potassium phosphate buffer (pH 6.5) containing any concentration (such as 0.01% to 50%) of a chloride, the same concentration of the carbonyl compound as in the "Methods for evaluating ability to reduce carbonyl compounds", and 0.3% (v/v) of dimethyl sulfoxide. The result is "enzyme activity with chloride".

Relative activity can be calculated from the following formula:

$$\text{Relative activity (\%)} = [\text{enzyme activity with chloride}] / [\text{enzyme activity without chloride}] \times 100.$$

In this Description, a modified carbonyl reductase having higher resistance to reaction inhibition by chlorides than the carbonyl reductase represented by SEQ ID NO:1 of the sequence listing is one the remaining activity of which as measured by the aforementioned evaluation method is at least 1% or preferably at least 5% or more preferably at least 10% or most preferably at least 20% higher than that of the wild type.

In this Description, an "enzyme having greater affinity for a coenzyme" is an enzyme that has a lower Km value with respect to a reduced coenzyme than the wild-type enzyme represented by SEQ ID NO:1 of the sequence listing. The reduced coenzyme is preferably NADPH. The greater the affinity of an enzyme for a coenzyme at the same coenzyme concentration, the higher the reactivity of the enzyme and the more useful it is. In the case of the wild-type enzyme there is competitive inhibition between the chloride and the coenzyme, and a modified carbonyl reductase with improved affinity for the coenzyme is useful because of having improved resistance to inhibition by the chloride (Comparative example 4 and Example 33).

The Km value of the reduced coenzyme can be evaluated by the following methods for example.

[Method for evaluating affinity for coenzyme]

Reductase activity with respect to any carbonyl compound (for example, 3-quinuclidinone or 3-chloro-1-(2-thienyl)propan-1-one) is measured by the same methods as in the "Method for evaluating ability to reduce carbonyl compounds" above in the presence of any concentrations (for example, 4 or more different concentrations in the range of 1 to 0.01 mM) of a reduced coenzyme (for example, NADPH). A Lineweaver-Burk plot is prepared using the measured reductase activity values, and the Km value can be calculated from this.

In this Description, a modified carbonyl reductase having greater affinity for a coenzyme than the wild-type carbonyl reductase represented by SEQ ID NO:1 in the sequence listing is an enzyme having a Km value of at most 99% or preferably at most 95% or more preferably at most 90% or most preferably at most 80% as measured by the aforementioned method, given 100% as the Km value of the wild-type enzyme.

The modified carbonyl reductase of the present invention can be discovered by the following methods.

Using a kit based on error prone PCR (Leung et al., Technique 1, 11-15 (1989)) or a similar principle, a DNA

fragment can be obtained comprising one or more substitutions, insertions, deletions or additions introduced into the nucleotide sequence represented by SEQ ID NO:2 in the sequence listing (wild-type enzyme gene). For example, using the wild-type enzyme gene as a template, a Diversify PCR Random Mutagenesis Kit (Clontech) can be used together with Primer 1: 5'-GGATAACAATTTCATAAGGAGGTTACATATG-3' (SEQ ID NO:3) and Primer 2: 5'-CTAGAGGATCCCCGGGTACCTTA-3' (SEQ ID NO:4) to obtain multiple kinds of double-stranded DNA (mutant enzyme genes) comprising mutations introduced randomly into the full-length wild-type enzyme gene, and having a NdeI recognition site added to the start codon and a new stop codon (TAA) and a KpnI recognition site added immediately after the stop codon. Each amplified fragment is digested with NdeI and KpnI and inserted between the KpnI recognition site and NdeI recognition site downstream from the lac promoter of a pUCN18 plasmid (plasmid obtained by using PCR to destroy the NdeI site of pUC18 (Takara Bio) by changing the No. 185 T to A, and introducing a new NdeI site by changing the Nos. 471 and 472 GC to TG), and the *Escherichia coli* HB101 strain (hereunder, *E. coli* HB101) is then transformed with this plasmid. The transformed *E. coli* is applied to LB plate medium containing 100 μg/mL of ampicillin, to obtain a single colony of *E. coli.* A mutant enzyme library comprising mutations introduced by the same operations can also be prepared using mutant enzyme genes obtained by the aforementioned methods in place of the wild-type gene.

The modified carbonyl reductase of the present invention can be selected from this library. The selection method is not particularly limited, but is preferably as follows.

[Selection method by plate evaluation of enzymes having improved stability with respect to chlorides]

A suitable medium (for example, 2 × YT medium (tryptone 1.6%, yeast extract 1.0%, sodium chloride 0.5%, pH 7.0) containing 200 μg/ml of ampicillin) is inoculated with recombinant bacteria of the mutant enzyme library and a recombinant bacteria that produces the wild-type enzyme (for example, the *E. coli* HB101 (pNBS) shown in Reference Example 3), and shaking cultured for 24 hours at 37°C. The resulting culture liquid is subjected to cell disruption treatment and centrifuged, and the sediment is removed to obtain a cell-free extract. A buffer solution (preferably an 0.01 to 1 M phosphate buffer solution with a pH of 5 to 8,) containing a suitable concentration of a chloride (preferably sodium chloride with a final concentration of 0.64 M, or chloroacetone with a final concentration of 0.01 to 0.02%) is added to the cell-free extracts containing each enzyme, and incubated at a suitable temperature (for example 4 to 40°C). After about 0.1 to 48 hours of incubation, the treated cell-free extract is dispensed onto a 96-well plate (AGC Technoglass), and phosphate buffer (pH 5 to 7) containing NADPH (preferably 1.5 mM) and a carbonyl compound (preferably 20 mM 3-quinuclidinone hydrochloride) is added and reacted at 10 to 40°C. NADPH fluorescence is observed over time with a FAS-III UV sample imaging system (Toyobo Co.). NADPH fluorescence persists in the enzyme liquid when the reaction does not progress, but when the reaction progresses the fluorescence disappears as the NADPH decreases in the cell-free extract. Those enzymes that produce a more rapid decrease in fluorescence in comparison with the wild-type enzyme used as a control are selected as enzymes having improved stability with respect to chlorides. The plasmids can be extracted from the culture liquids of the selected enzymes, and the nucleotide sequences of the modified carbonyl reductase genes can be determined with a BigDye Terminator Cycle Sequencing Kit (Applied Biosystems Japan) and an Applied Biosystems 3130xl Genetic Analyzer (Applied Biosystems Japan), and the mutation sites specified.

[Method of screening by plate evaluation of enzymes having improved resistance to reaction inhibition by chlorides]

A suitable medium (such as the medium used above) is inoculated with recombinant bacteria of the mutant enzyme library and a recombinant bacteria that produces the wild-type enzyme (for example, the *E. coli* HB101 (pNBS) shown in Reference Example 3), and shaking cultured for 24 hours at 37°C. The resulting culture liquid is subjected to cell disruption treatment, and a cell-free extract is obtained. Each cell-free extract is dispensed onto a 96-well plate (AGC Technoglass), and a phosphate buffer solution (pH 5 to 7) containing a suitable concentration of a chloride (preferably sodium chloride with a final concentration of 0.64 M to 1.28 M), NADPH (preferably 1.5 mM) and a carbonyl compound (preferably 20 mM 3-quinuclidinone hydrochloride) is added and reacted at 10 to 40°C. NADPH fluorescence is observed over time with a FAS-III UV sample imaging system (Toyobo Co.). NADPH fluorescence persists in the enzyme liquid when the reaction does not progress, but when the reaction progresses the fluorescence disappears as the NADPH decreases in the cell-free extract. Those enzymes that produce a more rapid decrease in fluorescence in comparison with the wild-type enzyme used as a control are selected as enzymes having improved resistance to reaction inhibition by chlorides. The plasmids can be extracted from the culture liquids of the selected enzymes, and the nucleotide sequences of the modified carbonyl reductase genes can be determined by the methods described above, and the mutation sites specified.

By site-directed mutagenesis using mutations of the multiple modified carbonyl reductase mutations thus obtained, either by identical methods or by a combination of methods, it is possible to prepare a modified carbonyl reductase having either one enhanced property or both enhanced properties. Such enzymes are included in the enzyme of the present invention.

The polynucleotide of the present invention is not particularly limited as long as it encodes the polypeptide of the present invention, but may for example be a polynucleotide consisting of the nucleotide sequence represented by SEQ ID NO:2 in the sequence listing, which codes for the wild-type enzyme, or a polypeptide obtained by modifying this sequence. The known methods described in Current Protocols in Molecular Biology (Frederick M. Ausubel, Greene Publishing Associates and Wiley-Interscience (1989)) and the like can be used for modifying the wild-type enzyme gene. That is, 1 or more (for example 40, or preferably 20, or more preferably 10, or still more preferably 5, 4, 3 or 2) nucleotides can be substituted, added, inserted or deleted in the wild-type enzyme gene to prepare a polynucleotide that modifies the amino acid sequence of the wild-type enzyme. For example, it is possible to use a mutagenesis method using PCR, such as error prone PCR (Leung et al., Technique 1, 11-15 (1989)), or a commercial kit such as the Diversify PCR Random Mutagenesis Kit (Clontech), Transformer Mutagenesis Kit (Clontech), EXOIII/Mung Bean Deletion Kit (Stratagene), QuickChange Site Directed Mutagenesis Kit (Stratagene) or the like.

When a polynucleotide is prepared by site directed mutagenesis, examples of site directed mutagenesis methods include the methods of Olfert Landt et al (Gene, 96, 125-128 (1990)), Smith et al (Genetic Engineering, 3, 1, Setlow, J. Plenum Press), Vlasuk et al (Experimental Manipulation of Gene Expression, Inouye, M. Academic Press) and Hos. N. Hun et al (Gene 77, 51 (1989)), or a QuikChange II Kit (Stratagene) commercial kit or the like can be used. When mutations are introduced at 2 locations, the target polynucleotide can be obtained by repeating the methods twice in accordance with the aforementioned methods. The target nucleotide can be obtained by these methods even if multiple other amino acids have been substituted at multiple other positions.

A polynucleotide encoding a polypeptide of the present invention is preferably a polynucleotide that encodes a polypeptide having the activity of producing (R)-3-quinuclidinol by reduction of 3-quinuclidinone or (S)-3-chloro-1-(2-thienyl)propan-1-ol by reduction of 3-chloro-1-(2-thienyl)propan-1-one for example, and having higher reactivity to carbonyl compounds in the presence of halogen atoms than a carbonyl reductase consisting of the amino acid sequence represented by SEQ ID NO:1 in the sequence listing, and that hybridizes under stringent conditions with a polynucleotide comprising a nucleotide sequence complementary to a polynucleotide consisting of the nucleotide sequence represented by SEQ ID NO:2 in the sequence listing.

"A polynucleotide that hybridizes under stringent conditions with a polynucleotide comprising a nucleotide sequence complementary to a polynucleotide consisting of the nucleotide sequence represented by SEQ ID NO:2 in the sequence listing" here means a polynucleotide obtained by colony hybridization, plaque hybridization, Southern hybridization or the like under stringent conditions using as a probe a polynucleotide consisting of a nucleotide sequence complementary to the nucleotide sequence represented by SEQ ID NO:2 in the sequence listing.

Hybridization can be performed by the methods described in Molecular Cloning 2nd Edition (Joseph Sambrook, Cold Spring Harbor Laboratory Press (1989)) and the like. A "polynucleotide that hybridizes under stringent conditions" here may be for example DNA that can be obtained using a filter with polynucleotides from a colony or plaque fixed thereon, by first performing hybridization at 65°C in the presence of 0.7 to 1.0 M sodium chloride, and then washing the filter under conditions of 65°C using a 2x concentration of SSC solution (a 1× concentration of SSC solution is composed of 150 mM sodium chloride and 15 mM sodium citrate). Preferably it is a polynucleotide that can be obtained by washing at 65°C with a 0.5× concentration of SSC solution, or more preferably at 65°C with an 0.2× concentration of SSC solution, or still more preferably at 65°C with an 0.1× concentration of SSC solution.

Although hybridization conditions are described above, the conditions are not limited to these. Multiple other factors such as temperature, salt concentration and the like may affect the stringency of hybridization, and a person skilled in the art can achieve the optimal degree of stringency by appropriate selection of such factors.

An example of a polynucleotide capable of hybridizing under these conditions is DNA having at least 70% or preferably at least 80% or more preferably at least 90% or still more preferably at least 95% or most preferably at least 98% sequence identity with the polynucleotide represented by SEQ ID NO:2, and this polynucleotide is included as long as the polypeptide encoded thereby has the properties of a polypeptide of the present invention as defined in claims 1-15.

A polypeptide expression vector can be prepared by inserting a polynucleotide encoding a polypeptide of the present invention into an expression vector.

The expression vector used above is not particularly limited as long as it is capable of expressing a polypeptide encoded by the polynucleotide in a suitable host organism. Examples include plasmid vectors, phage vectors, cosmid vectors and the like, and shuttle vectors capable of gene transfer between different host strains are also possible.

When such a vector is an *E. coli* for example, it normally includes the lacUV5 promoter, trp promoter, trc promoter, tac promoter, lpp promoter, tufB promoter, recA promoter, pL promoter and other control factors, and can be used favorably as an expression vector containing expression units linked operably to the DNA of the present invention. Examples include pUCN18 (see Reference Example 2), pSTV28 (Takara Bio), pUCNT (WO 94/03613) and the like.

The term "control factor" as used in this Description signifies a nucleotide sequence having a functional promoter and any associated transcription elements (such as an enhancer, CCAAT box, TATA box, SPI site or the like).

The term "operably linked" in this Description means that the gene and its promoters, enhancers and various other elements for regulating gene expression are linked in a way that allows them to operate in host cells. It is well

known to those skilled in the art that the type and species of control factors may vary depending on the host.

Vectors, promoters and the like that can be used in various organisms are described in detail in Biseibutsugaku Kiso Koza (No. 8, Tadahiko Ando, Kyoritsu Publishing (1987)).

The vector may also include a polynucleotide encoding a polypeptide having reductive coenzyme regeneration ability. Examples of polypeptides having reductive coenzyme regeneration ability include glucose dehydrogenase for example.

A transformant can be obtained by transforming host cells with the vector.

Another example of a transformant is one obtained by introducing a polynucleotide encoding the polypeptide of the present invention into a chromosome.

The host cells that are transformed with the vector are not particularly limited as long as they are cells that can be transformed with polypeptide expression vectors comprising polynucleotides encoding each polypeptide, and as long as they can express the polypeptides coded for by the introduced polynucleotides. Organisms that can be used as host cells include for example *Escherichia, Bacillus, Pseudomonas, Serratia, Brevibacterium, Corynebacterium, Streptococcus, Lactobacillus* and other bacteria for which host vector systems have been developed, *Rhodococcus, Streptomyces* and other actinomycetes for which host vector systems have been developed, *Saccharomyces, Kluyveromyces, Schizosaccharomyces, Zygosaccharomyces, Yarrowia, Trichosporon, Rhodosporidium, Pichia, Candida* and other yeasts for which host vector systems have been developed, and *Neurospora, Aspergillus, Cephalosporium, Trichoderma* and other molds for which host vector systems have been developed and the like. Apart from microorganisms, a variety of host vector systems have been developed in plants and animals, and in particular systems for expressing large quantities of foreign proteins in insects using silkworms (Nature, 315, 592-594 (1985)) and in rapeseed, corn, potatoes and other plants have been developed and can be used favorably. Of these, a bacteria is preferred for efficiency of introduction and expression, and *E. coli* is especially desirable.

The vector of the present invention can be introduced into a host microorganism by known methods. For example, using *E. coli* as the host microorganism, plasmids of the present invention (pNBSm 01 to 58 in Examples 2 to 9) comprising polynucleotides encoding modified carbonyl reductases introduced into the pUCN18 expression vector as a polypeptide expression vector can be introduced into host cells using commercial *E. coli* HB101 competent cells (Takara Bio) or the like in accordance with the attached protocols to obtain transformants (for example, *E. coli* HB101 (pNBSm58) in Example 9).

Moreover, it is also possible to breed a transformant comprising the polypeptide of the present invention and a polypeptide having reductive coenzyme regeneration ability, expressed together in the same bacterial cells. That is, a polynucleotide encoding the polypeptide of the present invention and a polynucleotide encoding a polypeptide having reductive coenzyme regeneration ability can be incorporated into the same vector and introduced into host cells, or else these two kinds of DNA can be incorporated into two different vectors belonging to different incompatibility groups, and introduced into the same host cells to obtain a transformant.

Examples of transformants obtained in this way include the transformant *E. coli* HB101 (pNBSm01, pSTVG) (Example 24), which comprises a recombinant vector (in this case, pNBSm01 in Example 2) obtained by introducing a nucleotide encoding a modified carbonyl reductase into the pUCN18 expression vector and a vector comprising a polynucleotide encoding glucose dehydrogenase (a polypeptide having reductive coenzyme regeneration ability), introduced into *E. coli* HB101 competent cells (Takara Bio) (see Example 24).

An alcohol compound can be manufactured by applying the polypeptide or its transformant and/or a treated product of the transformant to a carbonyl compound. The carbonyl compound used as the substrate is not particularly limited, but when the compound having carbonyl groups is an asymmetric ketone, the reaction is extremely beneficial because the product is a useful optically active alcohol.

An example of such a compound having carbonyl groups is the asymmetric ketone represented by Formula (1) below:

[Chem. 3]

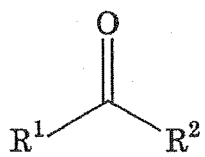

(1)

(wherein $R^1$ and $R^2$ are hydrogen atoms, halogen atoms, optionally substituted alkyl groups, optionally substituted aralkyl groups, optionally substituted aryl groups, optionally substituted alkoxy groups, hydroxyl groups, amino groups or nitro groups, or else $R^1$ and $R^2$ bind with each other to form a ring; but $R^1$ and $R^2$ have different structures), and the product in this case is an optically active alcohol represented by Formula (2) below:

[Chem. 4]

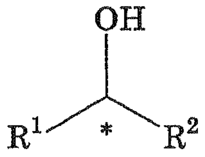

(2)

(wherein $R^1$ and $R^2$ are as described above, and * represents an asymmetric carbon).

$R^1$ and $R^2$ above are preferably $C_{1-14}$ alkyl groups, $C_{6-14}$ aryl groups, $C_{4-14}$ heteroaryl groups, $C_{1-5}$ alkoxy groups, $C_{2-5}$ alkoxycarboxyl groups, $C_{1-5}$ linear or branched alkyl groups, $C_{2-5}$ alkenyl groups, $C_{5-10}$ cycloalkyl groups, $C_{4-9}$ heterocycloalkyl groups, carboxyl groups, hydrogen atoms, halogen atoms, hydroxyl groups, amino groups or nitro groups.

The substituents mentioned above may be halogen atoms or hydroxyl, carboxyl, amino, cyano, nitro, alkyl, aryl, aralkyl or alkoxy groups or the like. The aforementioned halogen atoms are fluorine atoms, chlorine atoms, bromine atoms, iodine atoms or the like.

3-quinuclidinone or its hydrochloride or 3-chloro-1-(2-thienyl)propan-1-one or 3-chloro-1-(2-phenyl)propan-1-one is especially desirable as the asymmetrical ketone which is the substrate.

When the polypeptide of the present invention or a transformant and/or a treated product of a transformant expressing the polypeptide of the present invention is used to manufacture an alcohol by reduction of the aforementioned compound having carbonyl groups, this can be accomplished as follows. However, the method is not limited to the following.

A carbonyl compound as the substrate (for example, 3-quinuclidinone or its hydrochloride or 3-chloro-1-(2-thienyl)propan-1-one) is added to a suitable solvent (for example, a 100 mM phosphate buffer solution (pH 6.5) or the like), and a coenzyme such as NADPH or oxidized nicotinamide adenine dinucleotide phosphate (hereunder, $NADP^+$) is added together with a culture and/or a treated product of the transformant, and reacted with agitation and pH adjustment.

A treated product here means a material retaining the catalytic enzyme activity of the polypeptide, such as for example a crude liquid extract, cultured cells, freeze-dried organism, acetone-dried organism, crushed cell bodies or an immobilized form of these or the like.

This reaction is performed at a temperature of 5 to 80°C or preferably 10 to 60°C or more preferably 20 to 40°C, with the pH of the reaction solution maintained at 3 to 10 or preferably 4 to 9 or more preferably 5 to 8 during the reaction. The reaction can be performed either batch-wise or continuously. In the case of a batch system, the reactive substrate may be added at a concentration of 0.01 to 100% (w/v) or preferably 0.1 to 70% or more preferably 0.5 to 50% of the reaction mixture. Additional substrate can also be added during the reaction.

An aqueous solvent can be used for the reaction, or a mixture of an aqueous solvent and an organic solvent can be used. Examples of organic solvents include toluene, ethyl acetate, n-butyl acetate, hexane, isopropanol, diisopropyl ether, methanol, acetone, dimethyl sulfoxide and the like for example.

A treated product of the transformant here means for example a cell-free extract, cultured cells, freeze-dried cells, acetone dried cells or a ground form or mixture of these. Either the peptide itself or the original cells can also be fixed by known methods for purposes of use.

The amount of the coenzyme that is used in the present reaction can be greatly reduced by using a transformant such as *E. coli* HB101 (pNBSm01, pSTVG) (Example 24) that produces both the polypeptide of the present invention and a polypeptide having reductive coenzyme regeneration ability. The polypeptide having reductive coenzyme regeneration ability is explained in detail below.

When a transformant having the ability to produce the polypeptide of the present invention is used to synthesize an alcohol compound by reduction of a carbonyl compound, NADPH or NADH is required as a coenzyme. As discussed above, this can be accomplished by adding only the necessary amount of NADPH or NADH to the reaction system. However, it is also possible to greatly reduce the amount of the expensive coenzyme used in the reaction by reacting the substrate together with an enzyme having the ability to convert the oxidized coenzyme ($NADP^+$ or $NAD^+$) into reduced NADPH or NADH, (hereunder, reductive coenzyme regeneration ability), or in other words by combining the polypeptide of the present invention with a coenzyme regeneration system in the reaction. A hydrogenase, formate dehydrogenase, carbonyl reductase, glycose-6-phosphate dehydrogenase, glucose dehydrogenase or the like can be used as an enzyme having reductive coenzyme regeneration ability. Preferably, a glucose dehydrogenase is used.

Such a reaction can be performed by adding the coenzyme regeneration system to the asymmetric reduction reaction, but if the catalyst is a transformant that has been transformed with both a polynucleotide encoding the enzyme of the present invention and a polynucleotide encoding a polypeptide having reductive coenzyme regeneration ability, the reaction can be performed efficiently even if the enzyme having reductive coenzyme regeneration ability is not prepared separately and added to the reaction system. Such a transformant can be obtained by the methods described

under "Host vector system and transformant" above. An example is the aforementioned *E. coli* HB101 (pNBSm01, pSTVG) (Example 24).

The method of collecting the alcohol from the reaction solution after the reaction is not particularly limited, but a high-purity alcohol compound can be easily obtained by extraction with a solvent such as ethyl acetate, toluene, t-butyl methyl ether, hexane or methylene chloride either directly from the reaction liquid or after separation of the cell bodies and the like, followed by dehydration and then purification by distillation, recrystallization, silica gel column chromatography or the like.

A bulk pharmaceutical can be manufactured via a step in which the polypeptide of the present invention or a transformant and/or its treated product is applied to a carbonyl compound to obtain an optically active alcohol. Preferably, (1S,3'R)-quinuclidine-3'-yl 1-phenyl-1,2,3,4-tetrahydroisoquinoline-2-carboxylic acid (solifenacin) can be easily manufactured by known methods (such as the methods described in J. Med. Chem. 48, 6597-6606 (2005)) using (R)-3-quinuclidinol as a raw material. Another desirable example of a bulk pharmaceutical is (S)-N-methyl-3-(1-naphthalenyloxy)-3-(2-thiophene)-propanamine (duloxetine), which can be easily manufactured by known methods (such as the methods described in J. Label. Compd. Radiopharm. 34, 213-223 (1995)) using (S)-3-chloro-1-(2-thienyl)propan-1-ol as a raw material.

## EXAMPLES

The following examples illustrate the present invention in detail, but the present invention is not limited to these. Detailed procedures related to recombinant DNA technology used in the following examples are described in the following monographs:

Molecular Cloning 2nd Edition (Joseph Sambrook, Cold Spring Harbor Laboratory Press (1989)); and
Current Protocols in Molecular Biology (Frederick M. Ausubel, Greene Publishing Associates and Wiley-Interscience (1989)).

(Reference Example 1) Isolation of DNA encoding polypeptide (wild-type enzyme) having 3-quinuclidinone reducing activity from *Burkholderia sp.* YT strain

DNA encoding a polypeptide (hereunder, RBS) capable of reducing 3-quinuclidinone and 3-chloro-1-(2-thienyl)propan-1-one was obtained by PCR from the *Burkholderia sp.* YT strain (NITE P-613).

[Preparation of *Burkholderia sp.* YT strain chromosomal DNA]

A 500-ml Sakaguchi flask was charged with 50 ml of a liquid medium (pH 7) containing (per L) 16 g of bacto tryptone, 10 g of yeast extract, 5 g of sodium chloride, and 0.1 g of Adekanol LG-109 (NOF Corporation), and the flask was steam sterilized at 120°C for 20 minutes. The medium was inoculated with 5 ml of a culture fluid of the *Burkholderia sp.* YT strain, which had been obtained by culturing on the same medium in advance. Then, culturing was carried out by shaking culture at 30°C for 18 hours. Chromosomal DNA was extracted from the resulting culture liquid in accordance with the methods of Murray et al. (Nucl. Acids Res., 8, 4321, (1980)).

[PCR Reaction]

Chromosomal DNA of the *Burkholderia sp.* YT strain as a template was subjected to PCR with primer 3: 5'-ATATATACATATGACCCACCCGCAACCCCG-3' (SEQ ID NO:5), and primer 4: 5'-TATAGGTACCTTATCAAAGCTTGTCGAACGCGAG-3' (SEQ ID NO:6). As a result, a double stranded DNA (RBS gene) was obtained comprising an NdeI recognition site added to the initiation codon part of a gene having the nucleotide sequence of SEQ ID NO:2 in the sequence listing, and having a new termination codon (TAA) and a KpnI recognition site added immediately following the termination codon. PCR was performed using PrimeSTAR HS DNA polymerase (Takara Bio) under the reaction conditions recommended in the instruction manual.

(Reference Example 2) Construction of recombinant vector pNBS

The RBS gene obtained in Reference Example 1 was digested with NdeI and KpnI, and the resulting gene was inserted into a plasmid pUCN18 (plasmid derived from pUC18 (Takara Bio) by using PCR to destroy the NdeI site of pUC18 (Takara Bio) by changing the No. 185 T to A, and to introduce a new NdeI site by changing the Nos. 471 and 472 GC to TG) at a site between the NdeI recognition site and KpnI recognition site downstream from the lac promoter, to thereby construct a recombinant vector pNBS.

(Reference Example 3) Preparation of recombinant organisms expressing polypeptides

The recombinant vector pNBS constructed in Reference Example 2 was used to transform *E. coli* HB101 competent cells (Takara Bio) and obtain the living modified organism *E. coli* HB101 (pNBS). pUCN18 was also used to transform *E. coli* HB101 competent cells (Takara Bio) and obtain the living modified organism *E. coli* HB101 (pUCN18).

(Reference Example 4) Expression of DNA in living modified organisms

The two recombinant organisms (*E. coli* HB101 (pUCN18) and *E. coli* HB101 (pNBS)) obtained in Reference Example 3 were each inoculated into 5 ml of 2 × YT medium (tryptone 1.6%, yeast extract 1.0%, sodium chloride 0.5% (pH 7.0)) containing 200 μg/ml of ampicillin, and shaking cultured for 24 hours at 37°C. Cells in each culture liquid obtained by this culture were collected by centrifugation, and then suspended in 5 ml of a 100 mM phosphate buffer (pH 6.5). The cells were disrupted with a UH-50 ultrasonic homogenizer (SMT Co.), and the cell debris was removed by centrifugation to obtain a cell-free extract. The 3-quinuclidinone reduction activity of the resulting cell-free extracts was measured.

3-quinuclidinone reduction activity was determined by adding 3-quinuclidinone (5 mM), the coenzyme NADPH (0.25 mM) and the cell-free extract to a 100 mM phosphate buffer (pH 6.5), allowing the reaction to proceed at 30°C for 1 minute, and then calculating the rate of decrease in the absorbance at a wavelength of 340 nm. The enzyme activity of oxidizing 1 μmol of NADPH to NADP in 1 minute under these reaction conditions was defined as 1 U.

The 3-quinuclidinone reduction activities of the respective living modified organisms are shown below. The 3-quinuclidinone reducing activity of *E. coli* HB101 (pUCN18) was 0.1 U/mg or less. However, the 3-quinuclidinone reducing activity of *E. coli* HB101 (pNBS) expressing RBS was 100 U/mg.

Similarly, 3-chloro-1-(2-thienyl)propan-1-one reduction activity was calculated by adding 3-chloro-1-(2-thienyl)propan-1-one (10 mM), the coenzyme NADPH (0.25 mM) and the cell-free extract to a 100 mM phosphate buffer (pH 6.5), allowing the reaction to proceed at 30°C for 1 minute, and then determining the rate of decrease in the absorbance at a wavelength of 340 nm. The enzyme activity of oxidizing 1 μmol of NADPH to NADP in 1 minute under these reaction conditions was defined as 1 U.

The 3-chloro-1-(2-thienyl)propan-1-one reduction activities of the respective living modified organisms are shown below. The 3-chloro-1-(2-thienyl)propan-1-one reducing activity of *E. coli* HB101 (pUCN18) was 0.1 U/mg or less. However, the 3-chloro-1-(2-thienyl)propan-1-one reducing activity of *E. coli* HB101 (pNBS) expressing RBS was 25 U/mg.

Thus, the living modified organism obtained in Reference Example 3 has reduction activity with respect to 3-quinuclidinone and 3-chloro-1-(2-thienyl)propan-1-one, confirming RBS expression.

(Reference Example 5) Stability of wild-type enzyme RBS with respect to various compounds

A cell-free extract of the wild-type enzyme was obtained by the same methods as in Reference Example 4. The 3-quinuclidinone reduction activity of this cell-free extract was measured, and given as activity at 0 hours. The compounds shown in Table 1 were added to this cell-free extract, the pH was adjusted to 6.5 with sulfuric acid or sodium hydroxide, and the mixture was incubated for 24 hours at 30°C. A cell-free extract with nothing added thereto was also incubated in the same way as a control. After 24 hours, each cell-free extract was diluted, and 3-quinuclidinone activity was measured as in Reference Example 4. Remaining activity was calculated by the following formula, and this value was used as an indicator of stability with respect to the various compounds.

Remaining activity (%) = [enzyme activity at 24 hours] / [enzyme activity at 0 hours] × 100

The results are shown in Table 1.

[Table 1]

| Additives | Concentration | Remaining activity (%) |
|---|---|---|
| No addition | | 84 |
| Sodium chloride | 0.31 M | 46 |
| | 0.62 M | 24 |

(continued)

| Additives | Concentration | Remaining activity (%) |
|---|---|---|
| Potassium chloride | 0.31 M<br>0.62 M | 46<br>26 |
| Sodium sulfate | 0.31 M<br>0.62 M | 76<br>79 |
| 3-Quinuclidinone hydrochloride | 0.31 M<br>0.62 M | 73<br>44 |
| 3-Quinuclidinol | 0.31 M<br>0.62 M | 84<br>92 |
| 3-Chloro-1-(2-thienyl)propan-1-one | 0.1%<br>1.0% | 0<br>0 |
| Acetylthiophene | 1.0% | 72 |
| Acetophenone | 1.0% | 81 |
| Phenacyl chloride | 1.0% | 0 |
| m-chlorophenacyl chloride | 1.0% | 0 |
| 3-Chloro-1-(2-thienyl)propan-1-ol | 1.0% | 0 |
| Chloroacetone | 0.1% | 0 |
| Acetone | 1.0% | 80 |

The wild-type enzyme was less stable in the presence of the inorganic chlorides sodium chloride and potassium chloride than in the presence of sodium sulfate. The wild-type enzyme was also less stable with respect to the organic chlorides phenacyl chloride and m-chlorophenacyl chloride than with respect to acetophenone. Thus, the wild-type enzyme had low stability with respect to chlorides.

(Reference Example 6) Reaction inhibition of wild-type enzyme RBS by various compounds

A cell-free extract of the wild-type enzyme was obtained as in Reference Example 4. The 3-quinuclidinone reduction activity of this cell-free extract was measured, and given as activity without inhibitor. The cell-free extract was added to 100 mM phosphate buffer (pH 6.5) containing the compounds shown in Table 2, 3-quinuclidinone (5 mM) and the coenzyme NADPH (0.25 mM), and reacted for 1 minute at 30°C, and reduction activity of 3-quinuclidinone was calculated from the rate of decrease in absorbancy at 340 nm.

Relative activity was calculated by the following formula, and used as an indicator of reaction inhibition by the various compounds.

$$\text{Relative activity (\%)} = [\text{enzyme activity in presence of compound}] / [\text{enzyme activity without inhibitor}] \times 100$$

The results are shown in Table 2.

[Table 2]

| Additives | Concentration | Relative activity (%) |
|---|---|---|
| No inhibitor | | 100 |
| Sodium chloride | 0.31 M<br>0.62 M | 15<br>3 |
| Potassium chloride | 0.31 M<br>0.62 M | 14<br>5 |

(continued)

| Additives | Concentration | Relative activity (%) |
|---|---|---|
| Sodium sulfate | 0.31 M | 31 |
| | 0.62 M | 17 |

Inhibition of the wild-type enzyme by the inorganic chlorides sodium chloride and potassium chloride was greater than inhibition by sodium sulfate. Thus, the wild-type enzyme was strongly inhibited by chlorides.

(Example 1) Preparation of mutant enzyme library

The pNBS plasmid containing the RBS gene prepared in Reference Example 2 as a template was subjected to error prone PCR (Leung et al., Technique 1, 11-15 (1989)) with primer 1: 5'-GGATAACAATTTCATAAGGAGGTTACATATG-3' (SEQ ID NO:3) and primer 2: 5'-CTAGAGGATCCCCGGGTACCTTA-3' (SEQ ID NO:4) to produce DNA amplified fragments comprising random mutations introduced into the full-length RBS gene. These amplified fragments were digested with the restriction enzymes NdeI and KpnI, and inserted into the hyperexpression vector pUCN18, which had been treated with the same enzyme, to prepare multiple mutant enzyme expresion plasmids. *E. coli* HB101 cells were transformed with these plasmids, and applied to LB plate medium containing 100 μg/mL of ampicillin. Viable colonies were recombinant *E. coli* having mutated RBS genes, and these recombinant *E. coli* constituted mutant enzyme library 1.

(Example 2) Selection 1 of modified carbonyl reductases

Modified carbonyl reductases having improved stability with respect to one inorganic chloride, sodium chloride, were selected from a mutant enzyme library. The recombinant *E. coli* of mutant enzyme library 1 prepared in Example 1 and the *E. coli* HB101 (pNBS) prepared by the same methods in Reference Example 3 as a control were each cultured by the same methods as in Reference Example 4. 240 μL of phosphate buffer (pH 7.0) containing 10 mM EDTA·2Na and 1% Triton X-100 was added to 60 μl of each resulting culture liquid, and incubated for 1 hour at 37°C. Each treated liquid was centrifuged, and the supernatant was taken as a cell-free extract. Phosphate buffer (pH 6.5) containing 1.24 M sodium chloride was added to 100 μL of each cell-free extract, and incubated for 18 hours at 30°C (sodium chloride treatment). 50 μL of the sodium chloride treated cell-free extract was dispensed onto a 96-well plate (AGC Technoglass), and 50 μl of a phosphate buffer (pH 6.5) containing 6 mM NADPH and 100 μl of phosphate buffer (pH 6.5) containing 20 mM 3-quinuclidinone hydrochloride were added and reacted at 30°C. NADPH fluorescence was observed over time with a FAS-III UV sample imaging system (Toyobo Co.). NADPH fluorescence persists in the enzyme liquid when the reaction does not progress, but when the reaction progressed the fluorescence disappeared as the NADPH decreased in the cell-free extract. Enzymes exhibiting a rapid disappearance of fluorescence in comparison with the cell-free extract of *E. coli* HB101 (pNBS) used as a control (wild-type enzyme) were selected as enzymes having high reactivity in the presence of chlorine atoms, or in other words as modified carbonyl reductases having improved stability with respect to sodium chloride. The plasmids were extracted from the culture liquids of the selected enzymes, the nucleotide sequences of the mutant RBS genes were determined with a BigDye Terminator Cycle Sequencing Kit (Applied Biosystems Japan) and an Applied Biosystems 3130xl Genetic Analyzer (Applied Biosystems Japan), and the mutation sites were specified. Modified carbonyl reductases having improved stability with respect to sodium chloride are shown in Table 3.

[Table 3]

| Plasmid | Mutation site |
|---|---|
| pNBSm01 | T2I-L46M |
| pNBSm02 | Q5R-A23T-A100T-T201K |
| pNBSm03 | F26Y |
| pNBSm04 | R28H-S61G-P234L |
| pNBSm05 | F68S |
| pNBSm06 | D80Y-S130N |
| pNBSm07 | A81T |
| pNBSm08 | V83A |
| pNBSm09 | G88S |
| pNBSm10 | D95G |

(continued)

| Plasmid | Mutation site |
|---|---|
| pNBSm11 | E96K |
| pNBSm12 | A105T-A226V |
| pNBSm13 | A119E-S196L |
| pNBSm14 | D123G |
| pNBSm15 | G152S |
| pNBSm16 | D200V |
| pNBSm17 | F230I |

17 different enzymes having improved stability with respect to sodium chloride were obtained as shown in Table 3.

(Example 3) Selection 2 of modified carbonyl reductases

Modified carbonyl reductases having improved stability with respect to one organic chloride, chloroacetone, were selected from a mutant enzyme library. The recombinant *E. coli* of mutant enzyme library 1 prepared in Example 1 and the *E. coli* HB101 (pNBS) prepared by the same methods in Reference Example 3 as a control were each cultured by the same methods as in Reference Example 4. 240 μL of phosphate buffer (pH 7.0) containing 10 mM EDTA·2Na and 1% Triton X-100 was added to 60 μl of each resulting culture liquid, and incubated for 1 hour at 37°C. Each treated liquid was centrifuged, and the supernatant was taken as a cell-free extract. Phosphate buffer (pH 6.5) containing 0.02% chloroacetone was added to 100 μL of each cell-free extract, and incubated for 18 hours at 30°C (chloroacetone treatment). 50 μL of the sodium chloride treated cell-free extract was dispensed onto a 96-well plate (AGC Technoglass), and 50 μl of phosphate buffer (pH 6.5) containing 6 mM NADPH and 100 μl of phosphate buffer (pH 6.5) containing 20 mM 3-quinuclidinone hydrochloride were added and reacted at 30°C. NADPH fluorescence was observed over time with a FAS-III UV sample imaging system (Toyobo Co.). Enzymes exhibiting a rapid disappearance of fluorescence in comparison with the cell-free extract of *E. coli* HB101 (pNBS) used as a control (wild-type enzyme) were selected as enzymes having high reactivity in the presence of chlorine atoms, or in other words as modified carbonyl reductases having improved stability with respect to chloroacetone. The nucleotide sequences of the mutant RBS genes were determined by the same methods as in Example 2, and the mutation sites were specified. Enzymes having improved stability with respect to chloroacetone are shown in Table 4.

[Table 4]

| Plasmid | Mutation site |
|---|---|
| pNBSm18 | T8A |
| pNBSm19 | R28H-T62A-V79A-A164T |
| pNBSm20 | R29C |
| pNBSm21 | V33L |
| pNBSm22 | V34M |
| pNBSm23 | R71Q |
| pNBSm24 | R75H-A226T |
| pNBSm25 | R75C-I137T-D203G |
| pNBSm26 | V83A-A176V |
| pNBSm27 | G90S |
| pNBSm28 | E96D-H170R |
| pNBSm29 | S116T-R128H |
| pNBSm30 | A225S-264R |

13 different enzymes having improved stability with respect to chloroacetone were obtained as shown in Table 4.

(Example 4) Selection 3 of modified carbonyl reductases

Modified carbonyl reductases having improved resistance to reaction inhibition by one inorganic chloride, sodium

chloride, were selected from a mutant enzyme library. The recombinant *E. coli* of mutant enzyme library 1 prepared in Example 1 and the *E. coli* HB101 (pNBS) prepared by the same methods in Reference Example 3 as a control were each cultured by the same methods as in Reference Example 4. 240 μL of phosphate buffer (pH 7.0) containing 10 mM EDTA·2Na and 1% Triton X-100 was added to 60 μl of each resulting culture liquid, and incubated for 1 hour at 37°C. Each treated liquid was centrifuged, and the supernatant was taken as a cell-free extract. 50 μl of the cell-free extract was dispensed onto a 96-well plate (AGC Technoglass), and 50 μl of a phosphate buffer (pH 6.5) containing 6 mM NADPH, 1.24 M of sodium chloride, and 100 μl of phosphate buffer (pH 6.5) containing 20 mM 3-quinuclidinone hydrochloride were added and reacted at 30°C. NADPH fluorescence was observed over time with a FAS-III UV sample imaging system (Toyobo Co.). Enzymes exhibiting a rapid disappearance of fluorescence in comparison with the cell-free extract of *E. coli* HB101 (pNBS) (wild-type enzyme) used as a control were selected as enzymes having high reactivity in the presence of chlorine atoms, or in other words as modified carbonyl reductases having improved resistance to reaction inhibition by sodium chloride. The nucleotide sequences of the mutant RBS genes were determined by the same methods as in Example 2, and the mutation sites were specified. Enzymes having improved resistance to reaction inhibition by sodium chloride are shown in Table 5.

[Table 5]

| Plasmid | Mutation site |
|---|---|
| pNBSm31 | T2A-Q40R-A229V |
| pNBSm18 | T8A |
| pNBSm19 | R28H-T62A-V79A-A164T |
| pNBSm20 | R29C |
| pNBSm32 | G30S-L231F |
| pNBSm21 | V33L |
| pNBSm22 | V34M |
| pNBSm33 | F68L-A105V |
| pNBSm34 | T62A-V115I-A141V-P169S-G180C-A242T-K262M |
| pNBSm23 | R71Q |
| pNBSm35 | I72F-G133D |
| pNBSm24 | R75H-A226T |
| pNBSm25 | R75C-I137T-D203G |
| pNBSm26 | V83A-A176V |
| pNBSm27 | G90S |
| pNBSm28 | E96D-H170R |
| pNBSm36 | R103H-A189T-M191I |
| pNBSm37 | A108T-V146A-A164T-T218V |
| pNBSm29 | S116T-R128H |
| pNBSm38 | T201A |
| pNBSm39 | A211V-264W |
| pNBSm30 | A225S-264R |
| pNBSm40 | T240A |

23 different enzymes having improved resistance to reaction inhibition by sodium chloride were obtained as shown in Table 5.

(Example 5) Preparation of mutant enzyme library

A plasmid pNBSm1 comprising the mutant RBS gene of the T2I-L46M mutant enzyme obtained in Example 2 was used as a template to prepare a mutant enzyme library by the same methods as in Example 1, and this was called mutant enzyme library 2.

(Example 6) Selection 4 of modified carbonyl reductase

Modified carbonyl reductases having improved stability with respect to one organic chloride, chloroacetone, were selected from the mutant enzyme library 2. The recombinant *E. coli* of the mutant enzyme library 2 prepared in

Example 5 and *E. coli* HB101 (pNBSm1) producing the T2I-L46M mutant enzyme obtained in Example 2 as a control were each cultured by the same methods as in Reference Example 4. 240 μL of phosphate buffer (pH 7.0) containing 10 mM EDTA·2Na and 1% Triton X-100 was added to 60 μl of each resulting culture liquid, and incubated for 1 hour at 37°C. Each treated liquid was centrifuged, and the supernatant was taken as a cell-free extract. Phosphate buffer (pH 6.5)containing 0.02% chloroacetone was added to 100 μL of each cell-free extract, and incubated for 18 hours at 30°C (chloroacetone treatment). 50 μL of the chloroacetone treated cell-free extract was dispensed onto a 96-well plate (AGC Technoglass), and 50 μl of phosphate buffer (pH 6.5) containing 6 mM NADPH and 100 μl of phosphate buffer (pH 6.5) containing 20 mM 3-quinuclidinone hydrochloride were added and reacted at 30°C. NADPH fluorescence was observed over time with a FAS-III UV sample imaging system (Toyobo Co.). Enzymes exhibiting a rapid disappearance of fluorescence in comparison with the cell-free extract of *E. coli* HB101 (pNBSm1) used as a control (T2I-L46M mutant enzyme) were selected as enzymes having improved stability with respect to chloroacetone. The nucleotide sequences of the mutant RBS genes were determined by the same methods as in Example 2, and the mutation sites were specified. Modified carbonyl reductases having improved stability with respect to chloroacetone are shown in Table 6.

[Table 6]

| Plasmid | Mutation site |
|---|---|
| pNBSm41 | T2I-I11T-L46M |
| pNBSm42 | T2I-I11V-L46M-A64T |
| pNBSm43 | T2I-R32H-L46M |
| pNBSm44 | T2I-A45S-L46M-G90S |
| pNBSm45 | T2I-A45T-L46M |
| pNBSm46 | T2I-L46I |
| pNBSm47 | T2I-L46M-A48T |
| pNBSm48 | T2I-L46M-A65T |
| pNBSm49 | T2I-L46M-A66V |
| pNBSm50 | T2I-L46M-R78H |
| pNBSm51 | T2I-L46M-V177A |

11 different modified carbonyl reductases having improved stability with respect to chloroacetone were obtained as shown in Table 6.

(Example 7) Selection 7 of modified carbonyl reductases

Modified carbonyl reductases having improved resistance to reaction inhibition by one inorganic chloride, sodium chloride, were selected from mutant enzyme library 2. The recombinant *E. coli* of mutant enzyme library 2 prepared in Example 5 and the *E. coli* HB101 (pNBSm1) producing the T2I-L46M mutant enzyme obtained in Example 2 (control) were each cultured by the same methods as in Reference Example 4. 240 μL of phosphate buffer (pH 7.0) containing 10 mM EDTA·2Na and 1% Triton X-100 was added to 60 μl of each resulting culture liquid, and incubated for 1 hour at 37°C. Each treated liquid was centrifuged, and the supernatant was taken as a cell-free extract. 50 μl of the cell-free extract was dispensed onto a 96-well plate (AGC Technoglass), and 50 μl of a phosphate buffer (pH 6.5) containing 6 mM NADPH, 1.24 M of sodium chloride, and 100 μl of phosphate buffer (pH 6.5) containing 20 mM 3-quinuclidinone hydrochloride were added and reacted at 30°C. NADPH fluorescence was observed over time with a FAS-III UV sample imaging system (Toyobo Co.). Enzymes exhibiting a rapid disappearance of fluorescence in comparison with the cell-free extract of *E. coli* HB101 (pNBSm1) (T2I-L46M mutant enzyme) used as a control were selected as modified carbonyl reductases having improved resistance to reaction inhibition by sodium chloride. The nucleotide sequences of the mutant RBS genes were determined by the same methods as in Example 2, and the mutation sites were specified. The resulting modified carbonyl reductases having improved resistance to reaction inhibition by sodium chloride are shown in Table 7.

[Table 7]

| Plasmid | Mutation site |
|---|---|
| pNBSm52 | T2I-R28-L46M |
| pNBSm53 | T2I-R29L-R32H-L46M |
| pNBSm43 | T2I-R32H-L46M |
| pNBSm54 | T2I-A45P-L46M-G168R |

(continued)

| Plasmid | Mutation site |
|---|---|
| pNBSm44 | T2I-A45S-L46M-G90S |
| pNBSm45 | T2I-A45T-L46M |
| pNBSm55 | T2I-A45V-L46M |
| pNBSm46 | T21-L46I |
| pNBSm47 | T2I-L46M-A48T |
| pNBSm56 | T2I-L46M-A63T |
| pNBSm48 | T2I-L46M-A65T |
| pNBSm49 | T2I-L46M-A66V |
| pNBSm50 | T2I-L46M-R78H |

13 different modified carbonyl reductases having improved resistance to reaction inhibition by sodium chloride were obtained as shown in Table 7.

(Example 8) Preparation of modified carbonyl reductases with multiple mutations

The plasmid pNBSm01 obtained in Example 2 as a template was subjected to PCR with primer 5: 5'-ATATATACATATGATCCACCCGCAACCCCG-3' (SEQ ID NO:7) and primer 6: 5'-CGGCGATCCGCGGCAGGGAGCCGGCCCAGT-3' (SEQ ID NO:8) to obtain double-stranded DNA (N-terminal DNA) encoding an N-terminal polypeptide having T2I, L46M and F68S amino acid substitutions in the amino acid sequence represented by SEQ ID NO:1. Similarly, the plasmid pNBSm01 as a template was subjected to PCR with primer 7: 5' - ACTGGGCCGGCTCCCTGCCGCGGATCGCCG-3' (SEQ ID NO:9) and primer 8: 5' -TATAGGTACCTTATCAAAGCTTGTCGAACG-3' (SEQ ID NO:10) to obtain double-stranded DNA (C-terminal DNA) encoding a C-terminal polypeptide having an F68S amino acid substitution in the amino acid sequence represented by SEQ ID NO:1. The N-terminal DNA and C-terminal DNA were mixed, and used as a template in PCR using primer 5 and primer 8 to obtain double-stranded DNA encoding a polypeptide having T2I, L46M and F68S amino acid substitutions in the amino acid sequence represented by SEQ ID NO:1. This was introduced into pUCN18 by the same methods as in Reference Example 2 to prepare pNBSm57. *E. coli* HB101 competent cells (Takara Bio) were transformed with this pNBSm57, to obtain the living modified organism *E. coli* HB101 (pNBSm57) producing the modified carbonyl reductase T2I-L46M-F68S.

(Example 9) Preparation of modified carbonyl reductase with multiple mutations

The plasmid pNBSm01 obtained in Example 2 as a template was subjected to PCR with primer 9: 5'-ATATATACATATGACCCACCCGCAACCCCG-3' (SEQ ID NO:11) and primer 6, to obtain double-stranded DNA (N-terminal DNA) encoding an N-terminal peptide having L46M and F68S amino acid substitutions in the amino acid sequence represented by SEQ ID NO:1. Similarly, the plasmid pNBSm01 as a template was subjected to PCR with primer 7 and primer 8 to obtain double-stranded DNA (C-terminal DNA)encoding a C-terminal polypeptide having an F68S amino acid substitution in the amino acid sequence represented by SEQ ID NO:1. The N-terminal DNA and C-terminal DNA were mixed, and used as a template in PCR using primer 9 and primer 8 to obtain double-stranded DNA encoding a polypeptide having L46M and F68S amino acid substitutions in the amino acid sequence represented by SEQ ID NO:1. This was introduced into pUCN18 by the same methods as in Reference Example 2 to prepare pNBSm58. *E. coli* HB101 competent cells (Takara Bio) were transformed with this pNBSm58, to obtain the living modified organism *E. coli* HB101 (pNBSm58) producing the modified carbonyl reductase L46M-F68S.

(Example 10) Preparation of modified carbonyl reductase

The plasmid pNBS obtained in Reference Example 2 as a template was subjected to PCR using primer 5: 5' - ATATATACATATGATCCACCCGCAACCCCG-3' (SEQ ID NO:7) and primer 8: 5' -TATAGGTACCTTATCAAAGCTTGTCGAACG-3' (SEQ ID NO:10) to obtain double-stranded DNA encoding a polypeptide having a T2I amino acid substitution in the amino acid sequence represented by SEQ ID NO:1. pUCN18 was introduced by the same methods as in Reference Example 2 to prepare pNBSm59. *E. coli* HB101 competent cells (Takara Bio) were transformed with this pNBSm59, to obtain the living modified organism *E. coli* HB101 (pNBSm59) producing the modified carbonyl reductase T2I.

(Example 11) Preparation of modified carbonyl reductase

The plasmid pNBSm01 obtained in Reference Example 2 as a template was subjected to PCR using primer 9: 5' - ATATATACATATGACCCACCCGCAACCCCG-3' (SEQ ID NO:11) and primer 8: 5'-TATAGGTACCTTATCAAAGCTTGTCGAACG-3' (SEQ ID NO:10) to obtain double-stranded DNA encoding a polypeptide having a L46M amino acid substitution in the amino acid sequence represented by SEQ ID NO:1. pUCN18 was introduced by the same methods as in Reference Example 2 to prepare pNBSm60. *E. coli* HB101 competent cells (Takara Bio) were transformed with this pNBSm60 to obtain the living modified organism *E. coli* HB101 (pNBSm60) producing the modified carbonyl reductase L46M.

(Example 12) Evaluation 1 of modified carbonyl reductase

The recombinant microorganisms of the modified carbonyl reductases obtained in Example 2 and the *E. coli* HB101 (pNBS) prepared in Reference Example 3 (control) were each cultured by the same methods as in Reference Example 4. Cells were collected by centrifugation from each of the resulting culture liquids, and suspended in 100 mM phosphate buffer (pH 6.5) in the same amount as the culture liquid. These were then disrupted with a UH-50 ultrasound homogenizer (SMT Co.), and the cell debris was removed by centrifugation to obtain a cell-free extract. 500 μl of phosphate buffer (pH 7.0) containing 1.24 M sodium chloride was added to 500 μl of the cell-free extract, and incubated at 30°C (sodium chloride treatment). The sodium chloride treated liquid was sampled at 0 hours and 20 hours and diluted, and reduction activity of 3-quinuclidinone was measured by the methods shown in Reference Example 4. Remaining activity was calculated by the following formula, and used as an indicator of stability with respect to sodium chloride.

$$\text{Remaining activity (\%)} = [\text{enzyme activity at 20 hours}] / [\text{enzyme activity at 0 hours}] \times 100$$

The remaining activities of the wild-type enzyme and modified carbonyl reductases are shown in Table 8.

[Table 8]

| Mutation site | Remaining activity (%) |
|---|---|
| Wild-type enzyme | 24 |
| T2I-L46M | 34 |
| Q5R-A23T-A100T-T201K | 26 |
| F26Y | 25 |
| R28H-S61G-P234L | 30 |
| F68S | 59 |
| D80Y-S130N | 52 |
| A81T | 44 |
| V83A | 61 |
| G88S | 26 |
| D95G | 45 |
| E96K | 27 |
| A105T-A226V | 25 |
| A119E-S196L | 47 |
| D123G | 52 |
| G152S | 44 |
| D200V | 26 |
| F230I | 28 |

In comparison with the wild-type enzyme, the modified carbonyl reductases shown in Table 8 had improved stability with respect to one inorganic chloride, sodium chloride.

(Example 13) Evaluation 2 of modified carbonyl reductases

The recombinant microorganisms of the modified carbonyl reductases obtained in Examples 2 to 9 and the *E. coli* HB101 (pNBS) prepared in Reference Example 3 (control) were cultured by the same methods as in Reference Example 4. Cells were collected by centrifugation from the resulting culture liquids, and suspended in 100 mM phosphate buffer (pH 6.5) in the same amount as the culture liquid. These were then disrupted with a UH-50 ultrasound homogenizer (SMT Co.), and the cell debris was removed by centrifugation to obtain a cell-free extract. 500 μl of phosphate buffer (pH 7.0) containing 0.02% chloroacetone was added to 500 μl of the cell-free extract, and incubated at 30°C (chloroacetone treatment). The chloroacetone treated liquid was sampled at 0 hours and 20 hours and diluted, and reduction activity of 3-quinuclidinone was measured by the methods shown in Reference Example 4. Remaining activity was calculated by the following formula, and used as an indicator of stability with respect to chloroacetone.

Remaining activity (%) = [enzyme activity at 20 hours] / [enzyme activity at 0 hours] × 100

The remaining activities of the wild-type enzyme and modified carbonyl reductases are shown in Table 9.

[Table 9]

| Mutation site | Remaining activity (%) |
|---|---|
| Wild-type enzyme | 4 |
| T2I-L46M | 20 |
| R28H-S61G-P234L | 16 |
| F68S | 16 |
| D80Y-S130N | 8 |
| A81T | 7 |
| V83A | 17 |
| G88S | 34 |
| D95G | 32 |
| A119E-S196L | 25 |
| D123G | 7 |
| G152S | 20 |
| T2A-Q40R-A229V | 5 |
| T8A | 6 |
| R28H-T62A-V79A-A164T | 10 |
| R29C | 5 |
| V33L | 14 |
| V34M | 6 |
| R71Q | 6 |
| R75H-A226T | 5 |
| R75C-I137T-D203G | 7 |
| V83A-A176V | 29 |
| G90S | 26 |
| E96D-H170R | 6 |
| S116T-R128H | 10 |
| T201A | 14 |
| A225S-264R | 29 |
| T2I-R28-L46M | 21 |
| T2I-I11T-L46M | 11 |
| T2I-I11V-L46M-A64T | 5 |
| T21-R29L-R32H-L46M | 28 |
| T2I-R32H-L46M | 30 |
| T2I-A45P-L46M-G168R | 23 |

(continued)

| Mutation site | Remaining activity (%) |
|---|---|
| T2I-A45S-L46M-G90S | 66 |
| T2I-A45T-L46M | 26 |
| T2I-A45V-L46M | 25 |
| T2I-L46I | 22 |
| T2I-L46M-A48T | 8 |
| T2I-L46M-A63T | 26 |
| T2I-L46M-A65T | 36 |
| T2I-L46M-A66V | 48 |
| T2I-L46M-F68S | 49 |
| T2I-L46M-R78H | 23 |
| T2I-L46M-V177A | 35 |
| L46M-F68S | 44 |

In comparison with the wild-type enzyme, the modified carbonyl reductases shown in Table 9 had improved stability with respect to one inorganic chloride, chloroacetone. Moreover, the mutant enzymes T2I-L46M-F68S and L46M-F68S with multiple mutations prepared in Examples 8 and 9 had even greater stability than the modified carbonyl reductases T2I-L46M and F68S.

(Example 14) Evaluation 3 of modified carbonyl reductases

The recombinant microorganisms of the modified carbonyl reductases obtained in Examples 2 to 11 and the *E. coli* HB101 (pNBS) prepared in Reference Example 3 (control) were cultured by the same methods as in Reference Example 4. Cells were collected by centrifugation from the resulting culture liquids, and suspended in 100 mM phosphate buffer (pH 6.5) in the same amount as the culture liquid. These were then disrupted with a UH-50 ultrasound homogenizer (SMT Co.), and the cell debris was removed by centrifugation to obtain a cell-free extract. Reduction activity of 3-quinuclidinone in the presence of sodium chloride was determined by adding the cell-free extract to a 100 mM phosphate buffer (pH 6.5) containing sodium chloride (0.62 M), 3-quinuclidinone (5 mM) and the coenzyme NADPH (0.25 mM), and allowing the reaction to proceed at 30°C for 1 minute, and then calculating the rate of decrease in the absorbance at a wavelength of 340 nm. Reducing activity of 3-quinuclidinone without sodium chloride was also calculated by the methods shown in Reference Example 4. Relative activity was calculated by the following formula, and this value was used as an indicator of reaction inhibition by sodium chloride.

$$\text{Relative activity (\%)} = [\text{enzyme activity with sodium chloride}] / [\text{enzyme activity without sodium chloride}] \times 100$$

The relative activities of the wild-type enzyme and modified carbonyl reductases are shown in Table 10.

[Table 10]

| Mutation site | Relative activity (%) |
|---|---|
| Wild-type enzyme | 3 |
| T21 | 9 |
| T2I-L46M | 34 |
| Q5R-A23T-A100T-T201K | 10 |
| R28H-S61G-P234L | 6 |
| L46M | 38 |
| F68S | 14 |
| D80Y-S130N | 5 |
| A81T | 5 |
| V83A | 4 |
| G88S | 14 |

(continued)

| Mutation site | Relative activity (%) |
|---|---|
| D95G | 6 |
| A119E-S196L | 4 |
| D123G | 5 |
| G152S | 7 |
| T2A-Q40R-A229V | 7 |
| T8A | 4 |
| T62A-V115I-A141V-P169S-G180C-A242T-K262M | 6 |
| R28H-T62A-V79A-A164T | 4 |
| R29C | 5 |
| G30S-L231F | 6 |
| V33L | 5 |
| V34M | 4 |
| F68L-A105V | 6 |
| R71Q | 5 |
| I72F-G133D | 5 |
| R75H-A226T | 36 |
| R75C-I137T-D203G | 5 |
| V83A-A176V | 4 |
| G90S | 12 |
| E96D-H170R | 4 |
| R103H-A189T-M1911 | 5 |
| A108T-V146A-A164T-T218V | 6 |
| S116T-R128H | 5 |
| T201A | 8 |
| A211V-264W | 9 |
| A225S-264R | 7 |
| T240A | 4 |
| T2I-R28-L46M | 34 |
| T2I-I11T-L46M | 34 |
| T2I-I11V-L46M-A64T | 34 |
| T2I-R29L-R32H-L46M | 36 |
| T2I-R32H-L46M | 39 |
| T2I-A45P-L46M-G168R | 39 |
| T2I-A45S-L46M-G90S | 70 |
| T2I-A45T-L46M | 36 |
| T2I-A45V-L46M | 34 |
| T2I-L46I | 27 |
| T2I-L46M-A48T | 29 |
| T2I-L46M-A63T | 36 |
| T2I-L46M-A65T | 36 |
| T2I-L46M-A66V | 44 |
| T2I-L46M-R78H | 33 |
| T2I-L46M-F68S | 76 |
| T2I-L46M-V177A | 26 |
| L46M-F68S | 80 |

In comparison with the wild-type enzyme, the modified carbonyl reductases shown in Table 10 had improved resistance to reaction inhibition by one inorganic chloride, sodium chloride. Moreover, the mutant enzymes T2I-L46M-F68S and L46M-F68S with multiple mutations prepared in Examples 8 and 9 had improved resistance to reaction inhibition in comparison with the modified carbonyl reductases T2I-L46M and F68S.

(Example 15)

The *E. coli* HB101 (pNBS01) producing the wild-type enzyme and the modified carbonyl reductase T2I-L46M and the *E. coli* HB101 (pNBS43) producing T2I-R32H-L46M were cultured by the same methods as in Reference Example 4, and cell-free extracts were obtained. The 3-quinuclidinone reducing activity of these cell-free extracts was measured, and given as activity without inhibitor. The cell free-extracts were added to 100 mM phosphate buffers (pH 6.5) containing the compounds shown in Table 11, 5 mM 3-quinuclidinone and 0.25 mM of coenzyme NADPH, and reacted for 1 minute at 30°C, and reducing activity of 3-quinuclidinone was calculated from the rate of decrease in the absorbance at a wavelength of 340 nm.

Relative activity was calculated from the following formula, and this value was used as an indicator of reaction inhibition by each compound. The results are shown in Table 11.

$$\text{Relative activity (\%)} = [\text{enzyme activity with compound}] / [\text{enzyme activity without inhibitor}] \times 100$$

[Table 11]

| Additives | Concentration | Relative activity (%) | | |
|---|---|---|---|---|
| | | Wild-type enzyme | T2I-L46M | T2I-R32H-L46M |
| No inhibitor | | 100 | 100 | 100 |
| Sodium chloride | 0.62 M | 3 | 34 | 39 |
| Potassium bromide | 0.06 M | 51 | 96 | 94 |
| | 0.31 M | 7 | 12 | 15 |
| Sodium iodide | 0.06 M | 13 | 44 | 29 |
| | 0.31 M | 0 | 1 | 1 |

In comparison with the wild-type enzyme, the modified carbonyl reductases shown in Table 11 exhibited improved resistance to reaction inhibition to compounds containing halogen atoms, such as potassium bromide and sodium iodide.

(Example 16) Manufacture of (R)-3-quinuclidinol using modified carbonyl reductases

The recombinant microorganisms producing the modified carbonyl reductases obtained in Examples 2 to 11 were cultured as in Reference Example 4, and the cells were disrupted with an ultrasound homogenizer to obtain 1 ml cell-free extracts. 51 mg of NADPH and 10 mg of 3-quinuclidinone hydrochloride were added to 1 ml of each cell-free extract, the pH was adjusted to 6.5 with 6 N sodium hydroxide, and the mixture was shaken for 1 hour at 30°C. Part of the reaction liquid was sampled, adjusted to pH 13 or more with 6 N sodium hydroxide, and extracted with n-butanol. The extract was analyzed under the "Analysis conditions (2) for gas chromatography" described below to confirm production of 3-quinuclidinol. All the obtained modified carbonyl reductases steroselectively reduced 3-quinuclidinone to produce (R)-3-quinuclidinol.

(Comparative Example 1) Manufacture of (R)-3-quinuclidinol using living modified organism *E. coli* HB101 (pNBS)

*E. coli* HB101 (pNBS) producing the wild-type enzyme was cultured as in Reference Example 4, the cells were disrupted with an ultrasound homogenizer, and 100 ml of cell-free extract was obtained. 1000 U of glucose dehydrogenase (GLUCDH "Amano" II, Amano Enzyme Inc.), 24 g of glucose, 3 mg of $NADP^+$ and 19 g of 3-quinuclidinone hydrochloride containing hydrochloride acid as a chloride were added, and the pH was adjusted to 6.5 by dripping 5 N sodium hydroxide aqueous solution as the mixture was shaken for 20 hours at 30°C. Part of the reaction liquid was sampled, adjusted to pH 13 or more with 6 N sodium hydroxide, and then extracted with n-butanol. The extract was analyzed under the "Analysis conditions (1) for gas chromatography" described below to measure the conversion rate to (R)-3-quinuclidinol. The extract was analyzed under the "Analysis conditions (2) for gas chromatography" described below to measure the optical purity of (R)-3-quinuclidinol. As a result, the conversion rate of (R)-3-quinuclidinol was 45%, and optical purity was 99% e.e. or more. In the presence of a chloride, the wild-type enzyme suffered reaction inhibition and enzyme

inactivation, resulting in low conversion efficiency.

[Analysis conditions (1) for gas chromatography]

| | |
|---|---|
| Column: | Rtx-5 amine capillary column (30 m, 0.25 mm i.d., Restek) |
| Detector: | Hydrogen flame ionization detector |
| Injection temperature: | 220°C |
| Column temperature: | 150°C |
| Detector temperature: | 220°C |
| Carrier gas: | Helium, flow volume 130 KPa |

[Analysis conditions (2) for gas chromatography]

| | |
|---|---|
| Column: | Gamma DEX (30 m, 0.25 mm i.d., Supelco) |
| Detector: | Hydrogen flame ionization detector |
| Injection temperature: | 220°C |
| Column temperature: | 150°C |
| Detector temperature: | 220°C |
| Carrier gas: | Helium, flow volume 70 KPa |

(Comparative Example 2) Manufacture of (R)-3-quinuclidinol using living modified organism *E. coli* HB101 (pNBS)

(R)-3-quinuclidinol was manufactured using the wild-type enzyme without chlorides. 3-quinuclidinone hydrochloride was dissolved in an equal amount of water, adjusted to pH 12 with sodium hydroxide, and extracted 3 times with double the amount of n-butanol. The extract was concentrated under reduced pressure to obtain crystals of free form 3-quinuclidinone. *E. coli* HB101 (pNBS) producing the wild-type enzyme was cultured as in Reference Example 4, the cells were disrupted with an ultrasound homogenizer, and 100 ml of cell-free extract was obtained. 1000 U of glucose dehydrogenase (GLUCDH "Amano" II, Amano Enzyme Inc.), 24 g of glucose and 3 mg of $NADP^+$ were added to this 100 ml of cell-free extract, and the pH was adjusted to 6.5 by means of sulfate, with 15 g of 3-quinuclidinone being added. As the pH was adjusted to 6.5 by dropping of a 5 N aqueous solution of sodium hydroxide, the mixture was shaken for 20 hours at 30°C. Part of the reaction liquid was sampled, adjusted to pH 13 or more with 6 N sodium hydroxide, and then extracted with n-butanol. The extract was analyzed under the "Analysis conditions (1) for gas chromatography" described above to measure the conversion rate to (R)-3-quinuclidinol. The extract was also analyzed under the "Analysis conditions (2) for gas chromatography" described below to measure the optical purity of (R)-3-quinuclidinol. As a result, the conversion rate of (R)-3-quinuclidinol was 100%, and the optical purity was 99% e.e. or more. In comparison with Comparative Example 1, the wild-type enzyme exhibited a higher conversion rate without chlorides present. However, this was not an efficient manufacturing method because it required a step of preparing free form 3-quinuclidinone.

(Example 17) Manufacture of (R)-3-quinuclidinol using living modified organism *E. coli* HB101 (pNBSm01)

A cell free extract was obtained by the same methods as in Reference Example 4 from *E. coli* HB101 (pNBSm01), which produces the modified carbonyl reductase T2I-L46M obtained in Example 2, and a quinuclidinone reduction reaction was performed by the same methods as in Comparative Example 1. As a result, the conversion rate to (R)-3-quinuclidinol was 100%, and the optical purity was 99% e.e. or more.

The conversion rate in the reaction using the modified carbonyl reductase T2I-L46M was greater than when using the wild-type enzyme of Comparative Example 1, indicating improved reactivity in the presence of one chloride, 3-quinuclidinone hydrochloride.

(Example 18) Manufacture of (R)-3-quinuclidinol using living modified organism *E. coli* HB101 (pNBSm05)

A quinuclidinone reduction reaction was performed by the same methods as in Example 17 using *E. coli* HB101

(pNBSm05), which produces the modified carbonyl reductase F68S obtained in Example 2. As a result, the conversion rate was 100%, and the optical purity was 99% e.e. or more.

The conversion rate in the reaction using the modified carbonyl reductase F68S was higher than using the wild-type enzyme of Comparative Example 1, indicating improved reactivity in the presence of one chloride, 3-quinuclidinone hydrochloride.

(Example 19) Manufacture of (R)-3-quinuclidinol using living modified organism *E. coli* HB101 (pNBSm10)

A quinuclidinone reduction reaction was performed by the same methods as in Example 17 using *E. coli* HB101 (pNBSm10), which produces the modified carbonyl reductase D95G obtained in Example 2. As a result, the conversion rate was 100%, and the optical purity was 99% e.e. or more.

The conversion rate in the reaction using the modified carbonyl reductase D95G was higher than using the wild-type enzyme of Comparative Example 1, indicating improved reactivity in the presence of one chloride, 3-quinuclidinone hydrochloride.

(Example 20) Manufacture of (R)-3-quinuclidinone using living modified organism *E. coli* HB101 (pNBS01)

*E. coli* HB101 (pNBSm01) producing the modified carbonyl reductase T2I-L46M obtained in Example 2 was cultured as in Reference Example 4, after which the cells were disrupted with an ultrasound homogenizer to give 100 ml of a cell-free extract. 1000 U of glucose dehydrogenase (GLUCDH "Amano" II, Amano Enzyme Inc.), 32 g of glucose, 3 mg of $NADP^+$ and 26 g of 3-quinuclidinone hydrochloride were added to this 100 ml of cell-free extract, and stirred for 30 hours at 30°C as the pH was adjusted to 6.5 by dropping of a 5 N aqueous solution of sodium hydroxide. Part of the reaction liquid was sampled, adjusted with 6 N sodium hydroxide to a pH of at least 13, and then extracted with n-butanol. The extract was analyzed by the same methods as in Comparative Example 1. The measured conversion rate to (R)-3-quinuclidinol was 95%, and the optical purity was 99% e.e. or more.

The modified carbonyl reductase T2I-L46 having improved reactivity in the presence of chlorides provided a reaction at a higher concentration than the wild-type enzyme of Comparative Example 1.

(Example 21) Manufacture of (R)-3-quinuclidinol using living modified organism *E. coli* HB101 (pNBSm43)

A quinuclidinone reduction reaction was performed by the same methods as in Example 20 using *E. coli* HB101 (pNBSm43), which produces the modified carbonyl reductase T2I-R32H-L46M obtained in Example 6. As a result, the conversion rate to (R)-3-quinuclidinol was 100%, and the optical purity was 99% e.e. or more.

(Example 22) Manufacture of (R)-3-quinuclidinol using living modified organism *E. coli* HB101 (pNBSm44)

A quinuclidinone reduction reaction was performed by the same methods as in Example 20 using *E. coli* HB101 (pNBSm44), which produces the modified carbonyl reductase T2I-A45S-L46M-G90S obtained in Example 6. As a result, the conversion rate to (R)-3-quinuclidinol was 100%, and the optical purity was 99% e.e. or more.

(Example 23) Manufacture of (R)-3-quinuclidinol using living modified organism *E. coli* HB101 (pNBSm45)

A quinuclidinone reduction reaction was performed by the same methods as in Example 20 using *E. coli* HB101 (pNBSm45), which produces the modified carbonyl reductase T2I-A45S-L46M obtained in Example 6. As a result, the conversion rate to (R)-3-quinuclidinol was 100%, and the optical purity was 99% e.e. or more.

(Reference Example 7) Construction of recombinant vector pSTVG

A plasmid pGDK1 (which can be obtained and prepared by the method described in Eur. J. Biochem., 186, 389 (1989)) as a template was subjected to PCR with primer 10: 5'-GCCGAATTCTAAGGAGGTTAACAATGTATAAAGATTTAGAAGG-3' (SEQ ID NO:12 in the sequence listing), and primer 11: 5'-CTGCAGGTCGACTTATCCGCGTCCTGCTTGG-3' (SEQ ID NO:13 in the sequence listing). As a result, a double stranded DNA (GDH gene) was obtained comprising the ribosome-binding site of *E. coli* added at a site 5 bases upstream from the initiation codon of a glucose dehydrogenase (hereunder, GDH) gene derived from the *Bacillus megaterium* IAM 1030 strain; a EcoRI recognition site added at a site just before the ribosome-binding site; and SalI and PstI recognition sites added immediately following the termination codon. The resulting GDH gene was digested with EcoRI and PstI, and inserted into the EcoRI-PstiI site of the plasmid pSTV28 (Takara Bio) to construct the recombinant vector pSTVG.

(Reference Example 8) Preparation of living modified organism expressing polypeptide

*E. coli* HB101 competent cells (Takara Bio) were transformed with two recombinant vectors, the recombinant vector pNBS constructed in Reference Example 2 and the pSTVG constructed in Reference Example 7, to obtain the living modified organism *E. coli* HB101 (pNBS, pSTVG). *E. coli* HB101 competent cells (Takara Bio) were also transformed with pUCN18 and pSTV28 to obtain the living modified organism *E. coli* HB101 (pUCN18, pSTV28).

(Reference Example 9) Expression of DNA in living modified organisms

The two living modified organisms obtained in Reference Example 8 (*E. coli* HB101 (pNBS, pSTVG) and *E. coli* HB101 (pUCN18, pSTV28)) were each inoculated on 5 ml of 2 x YT medium (tryptone 1.6%, yeast extract 1.0%, sodium chloride 0.5%, pH 7.0) containing 200 μg/ml of ampicillin and 100 μg/ml of chloramphenicol, and shaking cultured for 24 hours at 37°C.

Cells were collected by centrifugation from each of the culture liquids obtained by the aforementioned culture, and suspended in 5 ml of 100 mM phosphate buffer (pH 6.5). These were then disrupted with a UH-50 ultrasound homogenizer (SMT Co.), and the cell debris was removed by centrifugation to obtain a cell-free extract. The 3-quinuclidinone reduction activity of these cell-free extracts was measured by the same methods as in Reference Example 6. Moreover, GDH activity was also measured.

GDH activity was determined by adding glucose (0.1 M) the coenzyme NADP (2 mM) and the cell free extract to 1 M tris-hydrochloride buffer (pH 8.0), allowing the reaction to proceed for 1 minute at 25°C, and then determining the rate of increase in the absorbance at a wavelength of 340 nm. The enzyme activity of reducing 1 μmol of NADP to NADPH in 1 minute under these reaction conditions was defined as 1 U.

The 3-quinuclidinone reduction activities and GDH activities of the respective living modified organisms are shown below.

The 3-quinuclidinone reduction activity and GDH activity of *E. coli* HB101 (pUCN18, pSTV28) were both 0.1 U/mg or less.

*E. coli* HB101 (pNBS, pSTVG) coexpressing RBS and GDH had 3-quinuclidinone reduction activity of 100 U/mg and GDH activity of 40 U/mg.

(Example 24) Preparation of living modified organism *E. coli* HB101 (pNBSm01, pSTVG)

HB101 competent cells (Takara Bio) were transformed with two recombinant vectors, the recombinant vector pNBSm01 containing a gene encoding the modified carbonyl reductase T2I-L46M obtained in Example 2 and the pSTVG vector constructed in Reference Example 5, to obtain a living modified organism *E. coli* HB101 (pNBSm01, pSTVG) .

(Example 25) Manufacture of (R)-3-quinuclidinol using living modified organism *E. coli* HB101 (pNBSm01, pSTVG)

The *E. coli* HB101 (pNBSm01, pSTVG) obtained in Example 24 was cultured as in Reference Example 4, and the cells were disrupted with an ultrasound homogenizer to give 100 ml of a cell-free extract. 24 g of glucose, 3 mg of $NADP^+$ and 19 g of 3-quinuclidinone hydrochloride were added to this 100 ml of cell-free extract, and stirred for 20 hours at 30°C as the pH was adjusted to 6.5 by dropping of a 5 N aqueous solution of sodium hydroxide. Part of the reaction liquid was sampled, adjusted with 6 N sodium hydroxide to a pH of at least 13, and then extracted with n-butanol. The extract was analyzed by the same methods as in Comparative Example 1. The measured conversion rate to (R)-3-quinuclidinol was 100%, and the optical purity was 99% e.e. or more.

(Comparative Example 3) Manufacture of (S)-3-chloro-1-(2-thienyl)propan-1-ol using living modified organism *E. coli* HB101 (pNBS)

*E. coli* HB101 (pNBS) producing the wild-type enzyme was cultured by the same methods as in Reference Example 4, after which the cells were disrupted with an ultrasound homogenizer to give 100 ml of a cell-free extract. 1000 U of glucose dehydrogenase (GLUCDH "Amano" II, Amano Enzyme Inc.), 30 g of glucose, 10 g of toluene, 10 mg of $NADP^+$ and 15 g of 3-chloro-1-(2-thienyl)propan-1-one were added to 100 ml of the cell-free extract, and the pH was adjusted to 6.25 by dropping 5 N sodium hydroxide aqueous solution as the mixture was stirred at 30°C. 24 hours after the start of the reaction part of the reaction liquid was sampled, and extracted with ethyl acetate. The extract was analyzed under the following high-performance liquid chromatography conditions, and the (S)-3-chloro-1-(2-thienyl)propan-1-ol conversion rate and optical purity were measured. The measured conversion rate was 78%, and the optical purity was 99% e.e.

[High-performance liquid chromatography analysis conditions]

| | |
|---|---|
| Column: | Chiralcel OD-H (4.6 μm i.d. x 250 mm; Daicel Chemical Industries) |
| Column temperature: | 30°C |
| Detection wavelength: | 240 nm |
| Mobile phase: | n-hexane/isopropanol = 97.5/2.5 |
| Retention times: | (S)-3-chloro-1-(2-thienyl)propan-1-ol about 19.9 minutes, (R)-3-chloro-1-(2-thienyl)propan-1-ol about 21.5 minutes, 3-chloro-1-(2-thienyl)propan-1-one about 12.2 minutes |

(Example 26) Manufacture of (S)-3-chloro-1-(2-thienyl)propan-1-ol using living modified organism *E. coli* HB101 (pNBSm01)

A 3-chloro-1-(2-thienyl)propan-1-one reduction reaction was performed by the same methods as in Comparative Example 3 using *E. coli* HB101 (pNBSm01), which produces the modified carbonyl reductase T2I-L46M obtained in Example 2. After completion of the reaction, this was analyzed by the same methods as in Comparative Example 2. As a result, the (S)-3-chloro-1-(2-thienyl)propan-1-ol conversion rate was 95%, and the optical purity was 99% e.e.

The conversion rate in the reaction using the modified carbonyl reductase T2I-L46M was higher than using the wild-type enzyme of Comparative Example 3, indicating improved reactivity in the presence of one organic chloride, 3-chloro-1-(2-thienyl)propan-1-one.

(Example 27) Manufacture of (S)-3-chloro-1-(2-thienyl)propan-1-ol using living modified organism *E. coli* HB101 (pNBSm05)

A 3-chloro-1-(2-thienyl)propan-1-one reduction reaction was performed by the same methods as in Example 26 using *E. coli* Hub101 (pNBSm05), which produces the modified carbonyl reductase F68S obtained in Example 2. As a result, the conversion rate to (S)-3-chloro-1-(2-thienyl)propan-1-ol was 92%, and the optical purity was 99% e.e.

The conversion rate in the reaction using the modified carbonyl reductase F68S was higher than using the wild-type enzyme of Comparative Example 3, indicating improved reactivity in the presence of one organic chloride, 3-chloro-1-(2-thienyl)propan-1-one.

(Example 28) Manufacture of (S)-3-chloro-1-(2-thienyl)propan-1-ol using living modified organism *E. coli* HB101 (pNBSm10)

A 3-chloro-1-(2-thienyl)propan-1-one reduction reaction was performed by the same methods as in Example 26 using *E. coli* HB101 (pNBSm10), which produces the modified carbonyl reductase D95G obtained in Example 2. As a result, the conversion rate to (S)-3-chloro-1-(2-thienyl)propan-1-ol was 96%, and the optical purity was 99% e.e. or more.

The conversion rate in the reaction using the modified carbonyl reductase D95G was higher than using the wild-type enzyme of Comparative Example 3, indicating improved reactivity in the presence of one organic chloride, 3-chloro-1-(2-thienyl)propan-1-one.

(Example 29) Manufacture of (S)-3-chloro-1-(2-thienyl)propan-1-ol using living modified organism *E. coli* HB101 (pNBSm43)

A 3-chloro-1-(2-thienyl)propan-1-one reduction reaction was performed by the same methods as in Example 26 using *E. coli* HB101 (pNBSm43), which produces the modified carbonyl reductase T2I-R32H-L46M obtained in Example 6. As a result, the conversion rate to (S)-3-chloro-1-(2-thienyl)propan-1-ol was 98%, and the optical purity was 99% e.e. or more,

The conversion rate in the reaction using the modified carbonyl reductase T2I-R32-L46M was higher than using the wild-type enzyme of Comparative Example 3, indicating improved reactivity in the presence of one organic chloride, 3-chloro-1-(2-thienyl)propan-1-one.

(Example 30) Manufacture of (S)-3-chloro-1-(2-thienyl)propan-1-ol using living modified organism *E. coli* HB101 (pNBSm01, pSTVG)

The *E. coli* HB101 (pNBSm01, pSTVG) obtained in Example 24 was cultured as in Reference Example 4 to

obtain 100 ml of culture liquid. 30 g of glucose, 10 g of toluene, 10 mg of $NADP^+$ and 15 g of 3-chloro-1-(2-thienyl)propan-1-one were added to this 100 ml of culture liquid, which was stirred at 30°C as the pH was adjusted to 6.25 by dropping a 5 N aqueous solution of sodium hydroxide. 24 hours after the start of the reaction part of the reaction liquid was sampled, and extracted with ethyl acetate. The liquid extract was analyzed by the same methods as in Comparative Example 2. As a result, the conversion rate to (S)-3-chloro-1-(2-thienyl)propan-1-ol was 96%, and the optical purity was 99% e.e. or more.

(Example 31) Manufacture of (1S,3'R)-quinuclidine-3'-yl 1-phenyl-1,2,3,4-tetrahydroisoquinoline-2-carboxylate hydrochloride

(Step 1)

The *E. coli* HB101 (pNBSm01, pSTVG) obtained in Example 24 was cultured as in Reference Example 4, and the cells were disrupted with an ultrasound homogenizer to give 200 ml of a cell-free extract. 48 g of glucose, 6 mg of $NADP^+$ and 38 g of 3-quinuclidinone hydrochloride were added to this 200 ml of cell-free extract, which was stirred for 20 hours at 30°C as the pH was adjusted to 6.5 by dropping a 5 N aqueous solution of sodium hydroxide. After 20 hours, the reaction solution was analyzed by the same methods as in Comparative Example 1. As a result, the optical purity of (R)-3-quinuclidinol was 99% e.e. or more. The reaction solution was adjusted to pH 13 or more with 6 N sodium hydroxide, and then extracted with n-butanol, and the extract was dried under reduced pressure to give crystals. These crystals were recrystallized to obtain 21 g of (R)-3-quinuclidinol crystals.

(Step 2)

10.6 g of (S)-1-phenyl-1,2,3,4-tetrahydroisoquinoline and 10.6 g of potassium carbonate were added to 200 ml of dichloromethane, and cooled in an ice bath as a mixed solution of 20 ml dichloromethane and 5.8 ml ethyl chloroformate was added, and the mixture was stirred overnight at room temperature. After the overnight stirring, water was added to the reaction solution and stirred for 30 minutes at room temperature. The organic layer was separated, washed with water and saturated saline, dehydrated with magnesium sulfate, and vacuum concentrated. The liquid concentrated was purified by silica gel chromatography to give (S)-1-phenyl-1,2,3,4-tetrahydroisoquinoline-2-carboxylic acid.

(Step 3)

12 g of the purified (S)-1-phenyl-1,2,3,4-tetrahydroisoquinoline-2-carboxylic acid and 16.3 g of (R) - 3-quinuclidinol were added to 120 ml of toluene, and 1.7 g of sodium hydride was added to the mixture, which was then heated for 3 hours at 140°C. After the reaction the mixture was cooled to room temperature, and extracted with ethyl acetate after addition of saturated saline. The extract was water washed, and extracted with 20% hydrochloric acid. The water layer was adjusted to pH 10 with 1 M sodium hydroxide, and extracted with ethyl acetate. The organic layer was washed with saturated saline, and dehydrated with magnesium sulfate. The liquid extracted was vacuum concentrated, and crystallized in diethyl ether and acetonitrile with added hydrochloric acid to give 10.1 g of (1S,3'R)-quinuclidine-3'-yl 1-phenyl-1,2,3,4-tetrahydroisoquinoline-2-carboxylate hydrochloride.

The bulk pharmaceutical (1S,3'R)-quinuclidine-3'-yl 1-phenyl-1,2,3,4-tetrahydroisoquinoline-2-carboxylate hydrochloride (solifenacin) was easily manufactured using as a raw material (R)-3-quinuclidinol manufactured with a modified carbonyl reductase.

(Example 32) Manufacture of (S)-N-methyl-3-(1-naphthalenyloxy)-3-(2-thiophene)-propanamine hydrochloride

(Step 1)

The *E. coli* HB101 (pNBSm01, pSTVG) obtained in Example 24 was cultured as in Reference Example 4 to give 200 ml of a culture liquid. 60 g of glucose, 20 g of toluene, 20 mg of $NADP^+$ and 30 g of 3-chloro-1-(2-thienyl)propan-1-one were added to 100 ml of this culture liquid, which was then stirred for 24 hours at 30°C as the pH was adjusted to 6.25 by dropping a 5 N aqueous solution of sodium hydroxide. After the reaction this was extracted with toluene, and the organic layer was vacuum concentrated to give 26 g of (S)-3-chloro-1-(2-thienyl)propan-1-ol. When the liquid concentrated was analyzed by the same methods as in Comparative Example 2, the optical purity of the (S)-3-chloro-1-(2-thienyl)propan-1-ol was 99% e.e. or more.

(Step 2)

21.6 g of (S)-3-chloro-1-(2-thienyl)propan-1-ol was dropped at room temperature into 93 g of 40% methylamine/methanol solution, and reacted for 30 minutes. The methanol and methylamine were distilled off under reduced pressure, water was added, and the mixture was extracted with methyl-t-butyl ether. The organic layer was washed with saline, and vacuum concentrated. N-heptane was added to the concentrate, and the precipitated solids were filtered out to give 17.4 g of light yellow crystals of (S)-3-N-methylamino-1-(2-thienyl)propan-1-ol.

(Step 3)

5 g of pentane-washed sodium hydride was suspended in dimethylacetamide, 17 g of (S)-3-N-methylamino-1-(2-thienyl)propan-1-ol was added, and the mixture was agitated with heating at 70°C. Once the solution had turned transparent, 26 g of 1-fluoronaphthalene was added, and agitated for 2 hours at 90°C. After the reaction water was added, the mixture was extracted with diethyl ether. The organic layer was washed with saturated saline, and vacuum concentrated. The liquid concentrate was purified by column chromatography to give 17 g of (S)-N-methyl-3-(1-naphthalenyloxy)-3-(2-thiophene)-propanamine. Next, the (S)-N-methyl-3-(1-naphthalenyloxy)-3-(2-thiophene)-propanamine was dissolved in ethyl acetate, and crystalized by dropping a 0.44 M hydrochloric acid/ethyl acetate solution. After 1 hour of agitation at room temperature this was cooled and mixed with diethyl ether, and the crystals were filtered out to obtain 15 g of (S)-N-methyl-3-(1-naphthalenyloxy)-3-(2-thiophene)-propanamine hydrochloride.

The bulk pharmaceutical (S)-N-methyl-3-(1-naphthalenyloxy)-3-(2-thiophene)-propanamine hydrochloride was easily manufactured using as a raw material (S)-3-chloro-1-(2-thienyl)propan-1-ol manufactured with a modified carbonyl reductase.

(Comparative Example 4) Calculating the Km value of wild-type enzyme for coenzyme

The *E. coli* HB101 (pNBS) (control) prepared in Reference Example 3 was cultured by the same methods as in Reference Example 4. Cells were collected by centrifugation from the resulting culture liquid, and suspended in 100 mM phosphate buffer (pH 6.5) in the same amount as the culture liquid. The cells were then disrupted with a UH-50 ultrasound homogenizer (SMT Co.), and the cell debris was removed by centrifugation to give a cell-free extract. Reduction activity of 3-quinuclidinone was determined by adding the cell-free extract to a 10 mM phosphate buffer solution (pH 6.5) containing 5 mM 3-quinuclidinone and 0.025 mM to 0.25 mM of the coenzyme NADPH, allowing the reaction to proceed for 1 minute at 30°C, and then calculating the rate of decrease in the absorbance at a wavelength of 340 nm. Similarly, reduction activity of 3-quinuclidinone in the presence of 0.62 M sodium chloride was measured at different enzyme concentrations. A Lineweaver-Burk plot of the measurement results is shown in FIG. 2.

The Km value for NADPH was 18.9 μM without sodium chloride and 191.3 μM with sodium chloride. The relationship between the Lineweaver-Burk plots with and without sodium chloride shows that sodium chloride is a competitive inhibitor of NADPH.

(Example 33) Km of mutant enzymes for coenzyme

The Km values for NADPH were calculated without sodium chloride by the same methods as in Comparative Example 4 with *E. coli* HB101 (pNBSm01), *E.* coli HB101 (pNBSm05), *E.* coli HB101 (pNBSm10) and *E.* coli HB101 (pNBSm60), which produce, respectively, the modified carbonyl reductases T2I-L46M, F68S, D95G and L46M obtained in Example 2 and Example 11.

As a result, the Km value for NADPH without sodium chloride was 6.4 μM for T2I-L46M, 13.8 μM for F68S, 15.5 μM for D95G and 6.3 μM for L46M. Given 100% as the Km value of the wild-type enzyme of Comparative Example 4, these modified carbonyl reductases had lower Km values for NADPH of 33.9%, 73.0%, 82.0% and 33.3%, respectively, indicating improved affinity for the coenzyme NADPH. Also, as shown in Example 14, these modified carbonyl reductases had better resistance to inhibition to sodium chloride than the wild-type enzyme. As shown in Reference Example 10, sodium chloride is a competitive inhibitor of NADPH when using the wild-type enzyme, and the improvement in affinity for the coenzyme provides improved resistance to inhibition by sodium chloride.

(SEQUENCE LISTING FREE TEXT)

Explanation of described sequences.

SEQ ID NO:1 Polypeptide sequence derived from *Burkholderia sp*.

(continued)

SEQ ID NO 2: Polypeptide sequence derived from *Burkholderia sp.*
SEQ ID NO 3: Primer 1
SEQ ID NO 4: Primer 2
SEQ ID NO 5: Primer 3
SEQ ID NO 6: Primer 4
SEQ ID NO 7: Primer 5
SEQ ID NO 8: Primer 6
SEQ ID NO 9: Primer 7
SEQ ID NO 10: Primer 8
SEQ ID NO 11: Primer 9
SEQ ID NO 12: Primer 10
SEQ ID NO 13: Primer 11

## Claims

**1.** A polypeptide having the following properties (a) to (c) :

(a) at least 97% sequence identity with an amino acid sequence represented by SEQ ID NO:1 in the sequence listing;
(b) production of (R)-3-quinuclidinol by reduction of 3-quinuclidinone or production of (S)-3-chloro-1-(2-thienyl)propan-1-ol by reduction of 3-chloro-1-(2-thienyl)propan-1-one; and
(c) higher reactivity to carbonyl compounds in the presence of a halogen atom in comparison with a carbonyl reductase consisting of the amino acid sequence represented by SEQ ID NO:1 in the sequence listing.

**2.** The polypeptide according to Claim 1, wherein the halogen atom is a chlorine atom.

**3.** The polypeptide according to Claim 1 or 2, wherein one or multiple amino acids selected from the group consisting of amino acids Nos. 2, 5, 8, 11, 23, 26, 28, 29, 30, 32, 33, 34, 40, 45, 46, 48, 61, 62, 64, 65, 66, 68, 71, 72, 75, 78, 79, 80, 81, 83, 88, 90, 95, 96, 100, 103, 105, 108, 115, 116, 119, 123, 128, 130, 133, 137, 141, 146, 152, 164, 168, 169, 170, 176, 177, 180, 189, 191, 196, 200, 201, 203, 211, 218, 225, 226, 229, 230, 231, 234, 240, 242 and 262 is substituted, and/or one or multiple amino acids are added to either the N or C terminus in the amino acid sequence represented by SEQ ID NO:1 in the sequence listing.

**4.** The polypeptide according to Claim 3, wherein one or multiple amino acid substitutions and/or amino acid additions selected from the group consisting of:

substitution of isoleucine or alanine for No. 2, arginine for No. 5, alanine for No. 8, valine or threonine for No.

11, threonine for No. 23, tyrosine for No. 26, histidine or leucine for No. 28, leucine for No. 29, serine for No. 30, histidine for No. 32, leucine for No. 33, methionine for No. 34, arginine for No. 40, serine, threonine, valine or proline for No. 45, methionine or isoleucine for No. 46, threonine for No. 48, glycine for No. 61, alanine or threonine for No. 62, threonine for No. 64, threonine for No. 65, valine for No. 66, serine or leucine for No. 68, glutamine for No. 71, phenylalanine for No. 72, cysteine or histidine for No. 75, histidine for No. 78, alanine for No. 79, tyrosine for No. 80, threonine for No. 81, alanine for No. 83, serine for No. 88, serine for No. 90, glycine for No. 95, aspartic acid or lysine for No. 96, threonine for No. 100, histidine for No. 103, threonine or valine for No. 105, threonine for No. 108, isoleucine for No. 115, threonine for No. 116, glutamic acid for No. 119, glycine for No. 123, histidine for No. 128, asparagine for No. 130, aspartic acid for No. 133, threonine for No. 137, valine for No. 141, alanine for No. 146, serine for No. 152, threonine for No. 164, arginine for No. 168, serine for No. 169, arginine for No. 170, valine for No. 176, alanine for No. 177, cysteine for No. 180, threonine for No. 189, isoleucine for No. 191, leucine for No. 196, valine for No. 200, alanine for No. 201, glycine for No. 203, valine for No. 211, valine for No. 218, serine for No. 225, threonine or valine for No. 226, valine for No. 229, isoleucine for No. 230, phenylalanine for No. 231, leucine for No. 234, alanine for No. 240, threonine for No. 242 and methionine for No. 262 and addition of the tryptophan and arginine to the C terminus is introduced, of the amino acid sequence represented by SEQ ID NO:1 in the sequence listing.

**5.** The polypeptide according to Claim 4, comprising one or multiple amino acid substitutions selected from the group consisting of: substitution of isoleucine for amino acid No. 2, tyrosine for No. 26, histidine for No. 28, methionine for No. 46, glycine for No. 61, serine for No. 68, tyrosine for No. 80, threonine for No. 81, alanine for No. 83, glycine for No. 95, lysine for No. 96, glutamic acid for No. 119, glycine for No. 123, asparagine for No. 130, serine for No. 152, leucine for No. 196, valine for No. 200, isoleucine for No. 230 and leucine for No. 234 introduced, of the amino acid sequence represented by SEQ ID NO:1 in the sequence listings, and having improved stability in the presence of chloride atoms in comparison with a carbonyl reductase consisting of the amino acid sequence represented by SEQ ID NO:1 in the sequence listing.

**6.** The polypeptide according to Claim 5, comprising an amino acid substitution or substitutions selected from the following (1) to (17):

(1) isoleucine for No. 2 and methionine for No. 46,
(2) arginine for No. 5, threonine for No. 23, threonine for No. 100 and lysine for No. 201,
(3) tyrosine for No. 26,
(4) histidine for No. 28, glycine for No. 61 and leucine for No. 234,
(5) serine for No. 68,
(6) tyrosine for No. 80 and asparagine for No. 130,
(7) threonine for No. 81,
(8) alanine for No. 83,
(9) serine for No. 88,
(10) glycine for No. 95,
(11) lysine for No. 96,
(12) threonine for No. 105 and valine for No. 226,
(13) glutamic acid for No. 119 and leucine for No. 196,
(14) glycine for No. 123,
(15) serine for No. 152,
(16) valine for No. 200 and
(17) isoleucine for No. 230

introduced into the amino acid sequence represented by SEQ ID NO:1 in the sequence listings.

**7.** The polypeptide according to Claim 4, comprising one or multiple amino acid substitutions and/or amino acid additions selected from the group consisting of:

substitution of isoleucine or alanine for No. 2, arginine for No. 5, alanine for No. 8, threonine for No. 23, tyrosine for No. 26, histidine or leucine for No. 28, leucine for No. 29, serine for No. 30, histidine for No. 32, leucine for No. 33, methionine for No. 34, arginine for No. 40, serine, threonine, valine or proline for No. 45, methionine or isoleucine for No. 46, threonine for No. 48, glycine for No. 61, alanine or threonine for No. 62, threonine for No. 65, valine for No. 66, serine or leucine for No. 68, glutamine for No. 71, phenylalanine for No. 72, cysteine or histidine for No. 75, histidine for No. 78, alanine for No. 79, tyrosine for No. 80, threonine for No. 81, serine for

No. 88, serine for No. 90, glycine for No. 95, aspartic acid or lysine for No. 96, threonine for No. 100, histidine for No. 103, valine for No. 105, threonine for No. 108, isoleucine for No. 115, threonine for No. 116, glycine for No. 123, histidine for No. 128, asparagine for No. 130, aspartic acid for No. 133, threonine for No. 137, valine for No. 141, alanine for No. 146, serine for No. 152, threonine for No. 164, arginine for No. 168, serine for No. 169, arginine for No. 170, valine for No. 176, alanine for No. 177, cysteine for No. 180, threonine for No. 189, isoleucine for No. 191, valine for No. 200, glycine for No. 203, valine for No. 211, valine for No. 218, serine for No. 225, threonine for No. 226, valine for No. 229, isoleucine for No. 230, phenylalanine for No. 231, leucine for No. 234, alanine for No. 240, threonine for No. 242 and methionine for No. 262 and addition of the tryptophan and arginine to the C terminus, introduced into the amino acid sequence represented by SEQ ID NO:1 in the sequence listing, and having improved resistance to reaction inhibition by chloride ions in comparison with a carbonyl reductase consisting of the amino acid sequence represented by SEQ ID NO:1 in the sequence listing.

**8.** The polypeptide according to Claim 7, comprising an amino acid addition and/or an amino acid substitution or substitutions selected from the following (1) to (55):

(1) alanine for No. 2, arginine for No. 40 and valine for No. 229,
(2) isoleucine for No. 2,
(3) isoleucine for No. 2, valine for No. 11, methionine for No. 46 and threonine for No. 64,
(4) isoleucine for No. 2, threonine for No. 11 and methionine for No. 46,
(5) isoleucine for No. 2, leucine for No. 28 and methionine for No. 46,
(6) isoleucine for No. 2, leucine for No. 29, histidine for No. 32 and methionine for No. 46,
(7) isoleucine for No. 2, histidine for No. 32 and methionine for No. 46,
(8) isoleucine for No. 2, threonine for No. 45 and methionine for No. 46,
(9) isoleucine for No. 2, valine for No. 45 and methionine for No. 46,
(10) isoleucine for No. 2, proline for No. 45, methionine for No. 46 and arginine for No. 168,
(11) isoleucine for No. 2, serine for No. 45, methionine for No. 46 and serine for No. 90,
(12) isoleucine for No. 2 and isoleucine for No. 46,
(13) isoleucine for No. 2 and methionine for No. 46,
(14) isoleucine for No. 2, methionine for No. 46 and threonine for No. 48,
(15) isoleucine for No. 2, methionine for No. 46 and threonine for No. 62,
(16) isoleucine for No. 2, methionine for No. 46 and threonine for No. 65,
(17) isoleucine for No. 2, methionine for No. 46 and valine for No. 66,
(18) isoleucine for No. 2, methionine for No. 46 and serine for No. 68,
(19) isoleucine for No. 2, methionine for No. 46 and histidine for No. 78,
(20) isoleucine for No. 2, methionine for No. 46 and alanine for No. 177,
(21) arginine for No. 5, threonine for No. 23, threonine for No. 100 and lysine for No. 201,
(22) alanine for No. 8,
(23) histidine for No. 28, glycine for No. 61 and leucine for No. 234,
(24) histidine for No. 28, alanine for No. 62, alanine for No. 79 and threonine for No. 164,
(25) cysteine for No. 29,
(26) serine for No. 30 and phenylalanine for No. 231,
(27) leucine for No. 33,
(28) methionine for No. 34,
(29) methionine for No. 46,
(30) methionine for No. 46 and serine for No. 68,
(31) alanine for No. 62, isoleucine for No. 115, valine for No. 141, serine for No. 169, cysteine for No. 180, threonine for No. 242 and methionine for No. 262,
(32) serine for No. 68,
(33) leucine for No. 68 and valine for No. 105,
(34) glutamine for No. 71,
(35) phenylalanine for No. 72 and aspartic acid for No. 133,
(36) cysteine for No. 75, threonine for No. 137 and glycine for No. 203,
(37) histidine for No. 75 and threonine for No. 226,
(38) tyrosine for No. 80 and asparagine for No. 130,
(39) threonine for No. 81,
(40) alanine for No. 83,
(41) alanine for No. 83 and threonine for No. 176,
(42) serine for No. 88,

(43) serine for No. 90,
(44) glycine for No. 95,
(45) aspartic acid for No. 96 and arginine for No. 170,
(46) histidine for No. 103, threonine for No. 189 and isoleucine for No. 191,
(47) threonine for No. 108, alanine for No. 146, threonine for No. 164 and valine for No. 218,
(48) threonine for No. 116 and histidine for No. 128,
(49) glutamic acid for No. 119 and leucine for No. 196,
(50) glycine for No. 123,
(51) serine for No. 152,
(52) alanine for No. 201,
(53) valine for No. 211 and tryptophan for No. 264,
(54) serine for No. 225 and arginine for No. 264 and
(55) alanine for No. 240,

introduced into the amino acid sequence represented by SEQ ID NO:1 in the sequence listing.

**9.** The polypeptide according to Claim 4, comprising one or multiple amino acid substitutions and/or amino acid additions selected from the following group: substitution of isoleucine for No. 2, alanine for No. 8, valine or threonine for No. 11, tyrosine for No. 26, histidine for No. 28, histidine for No. 32, leucine for No. 33, methionine for No. 34, serine or threonine for No. 45, methionine or isoleucine for No. 46, threonine for No. 48, glycine for No. 61, threonine for No. 62, threonine for No. 64, threonine for No. 65, valine for No. 66, serine for No. 68, glutamine for No. 71, cysteine or histidine for No. 75, histidine for No. 78, alanine for No. 79, tyrosine for No. 80, threonine for No. 81, alanine for No. 83, serine for No. 88, serine for No. 90, glycine for No. 95, aspartic acid or lysine for No. 96, threonine for No. 116, glutamic acid for No. 119, glycine for No. 123, histidine for No. 128, asparagine for No. 130, threonine for No. 137, serine for No. 152, arginine for No. 170, valine for No. 176, alanine for No. 177, leucine for No. 196, valine for No. 200, glycine for No. 203, serine for No. 225, threonine for No. 226, isoleucine for No. 230 and leucine for No. 234 introduced into the amino acid sequence represented by SEQ ID NO:1 in the sequence listing, and having improved stability in the presence of organic chlorides in comparison with a carbonyl reductase consisting of the amino acid sequence represented by SEQ ID NO:1 in the sequence listing.

**10.** The polypeptide according to Claim 9, comprising an amino acid substitution or substitutions selected from the following (1) to (44):

(1) alanine for No. 2, arginine for No. 40 and valine for No. 229,
(2) isoleucine for No. 2, valine for No. 11, methionine for No. 46 and threonine for No. 64,
(3) isoleucine for No. 2, threonine for No. 11 and methionine for No. 46,
(4) isoleucine for No. 2, leucine for No. 28 and methionine for No. 46,
(5) isoleucine for No. 2, leucine for No. 29, histidine for No. 32 and methionine for No. 46,
(6) isoleucine for No. 2, histidine for No. 32 and methionine for No. 46,
(7) isoleucine for No. 2, threonine for No. 45 and methionine for No. 46,
(8) isoleucine for No. 2, valine for No. 45 and methionine for No. 46,
(9) isoleucine for No. 2, proline for No. 45, methionine for No. 46 and arginine for No. 168,
(10) isoleucine for No. 2, serine for No. 45, methionine for No. 46 and serine for No. 90,
(11) isoleucine for No. 2 and isoleucine for No. 46,
(12) isoleucine for No. 2 and methionine for No. 46,
(13) isoleucine for No. 2, methionine for No. 46 and threonine for No. 48,
(14) isoleucine for No. 2, methionine for No. 46 and threonine for No. 62,
(15) isoleucine for No. 2, methionine for No. 46 and threonine for No. 65,
(16) isoleucine for No. 2, methionine for No. 46 and valine for No. 66,
(17) isoleucine for No. 2, methionine for No. 46 and serine for No. 68,
(18) isoleucine for No. 2, methionine for No. 46 and histidine for No. 78,
(19) isoleucine for No. 2, methionine for No. 46 and alanine for No. 177,
(20) alanine for No. 8,
(21) histidine for No. 28, glycine for No. 61 and leucine for No. 234,
(22) histidine for No. 28, alanine for No. 62, alanine for No. 79 and threonine for No. 164,
(23) cysteine for No. 29,
(24) leucine for No. 33,
(25) methionine for No. 34,

(26) methionine for No. 46 and serine for No. 68,
(27) serine for No. 68,
(28) glutamine for No. 71,
(29) cysteine for No. 75, threonine for No. 137 and glycine for No. 203,
(30) histidine for No. 75 and threonine for No. 226,
(31) tyrosine for No. 80 and asparagine for No. 130,
(32) threonine for No. 81,
(33) alanine for No. 83,
(34) alanine for No. 83 and valine for No. 176,
(35) serine for No. 88,
(36) serine for No. 90,
(37) glycine for No. 95,
(38) aspartic acid for No. 96 and arginine for No. 170,
(39) threonine for No. 116 and histidine for No. 128,
(40) glutamic acid for No. 119 and leucine for No. 196,
(41) glycine for No. 123,
(42) serine for No. 152,
(43) alanine for No. 201 and
(44) serine for No. 225 and arginine for No. 264, introduced into the amino acid sequence represented by SEQ ID NO:1 in the sequence listing.

**11.** The polypeptide according to Claim 9 or 10, wherein the organic chloride is a chloroketone.

**12.** The polypeptide according to Claim 11, wherein the chloroketone is either chloroacetone or 3-chloro-1-(2-thienyl)propan-1-one.

**13.** The polypeptide according to Claim 1 or 2, having, in addition to properties the (a) to (c), property (d) which is greater affinity with a coenzyme than a carbonyl reductase consisting of the amino acid sequence represented by SEQ ID NO:1 in the sequence listing.

**14.** The polypeptide according to Claim 13, wherein one or multiple amino acids selected from the group consisting of the following:

amino acids Nos. 2, 46, 68 and 95,
is substituted in the amino acid sequence represented by SEQ ID NO:1 in the sequence listing.

**15.** The polypeptide according to Claim 14, wherein one or multiple amino acid substitutions selected from the group consisting of the following:

isoleucine for No. 2, methionine for No. 46, serine for No. 68 and glycine for No. 95,
is introduced, of the amino acid sequence represented by SEQ ID NO:1 in the sequence listing.

**16.** A polynucleotide encoding the polypeptide according to any one of Claims 1 to 15.

**17.** A vector comprising the polynucleotide according to Claim 16.

**18.** The vector according to Claim 17, also comprising a polynucleotide encoding a polypeptide having reductive coenzyme regeneration ability.

**19.** A transformant obtained by transforming host cells with the vector according to any of Claims 17 to 18.

**20.** A method for manufacturing an alcohol compound, wherein the polypeptide according to any of Claims 1 to 15 or the transformant according to Claim 19 and/or a treated product thereof is applied to a carbonyl compound.

**21.** The manufacturing method according to Claim 20, wherein the carbonyl compound is an asymmetric ketone, and the product is an optically active alcohol.

**22.** The manufacturing method according to Claim 21, wherein the compound having a carbonyl group is an asymmetric

ketone represented by the following Formula (1):

[Chem. 1]

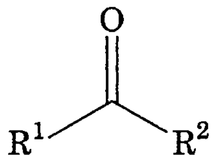
(1)

(where $R^1$ and $R^2$ are hydrogen atoms, halogen atoms, optionally substituted alkyl groups, optionally substituted aralkyl groups, optionally substituted aryl groups, optionally substituted alkoxy groups, amino groups or nitro groups, or else $R^1$ and $R^2$ bind with each other to form a ring; but $R^1$ and $R^2$ have different structures), and the product is an optically active alcohol represented by the following Formula (2):

[Chem. 2]

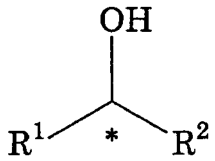
(2)

(where $R^1$ and $R^2$ are as described above, and * represents an asymmetric carbon).

**23.** The manufacturing method according to Claim 21, wherein the compound having a carbonyl group is 3-quinuclidinone or salt thereof , 3-chloro-1-(2-thienyl)propan-1-one, or 3-chloro-1-(2-phenyl)propan-1-one.

**24.** A method for manufacturing a bulk pharmaceutical, the method comprising a step in which the polypeptide according to any of Claims 1 to 15 or the transformant according to Claim 19 and/or the treated product thereof is applied to a carbonyl compound to produce an optically active alcohol.

**Patentansprüche**

**1.** Polypeptid, welches die folgenden Eigenschaften (a) bis (c) aufweist:

(a) wenigstens 97% Sequenzidentität zu einer Aminosäuresequenz, die durch SEQ ID NO: 1 im Sequenzprotokoll repräsentiert wird;
(b) Produktion von (R)-3-Chinuclidinol durch Reduktion von 3-Chinuclidinon oder Produktion von (S)-3-Chlor-1-(2-thienyl)propan-1-ol durch Reduktion von 3-Chlor-1-(2-thienyl)propan-1-on; und
(c) höhere Reaktivität gegenüber Carbonylverbindungen in der Gegenwart eines Halogenatoms im Vergleich zu einer Carbonylreduktase, bestehend aus der Aminosäuresequenz, die durch SEQ ID NO: 1 im Sequenzprotokoll repräsentiert wird.

**2.** Polypeptid gemäß Anspruch 1, wobei das Halogenatom ein Chloratom ist.

**3.** Polypeptid gemäß Anspruch 1 oder 2, wobei eine oder mehrere Aminosäuren, ausgewählt aus der Gruppe, bestehend aus den Aminosäuren Nrn. 2, 5, 8, 11, 23, 26, 28, 29, 30, 32, 33, 34, 40, 45, 46, 48, 61, 62, 64, 65, 66, 68, 71, 72, 75, 78, 79, 80, 81, 83, 88, 90, 95, 96, 100, 103, 105, 108, 115, 116, 119, 123, 128, 130, 133, 137, 141, 146, 152, 164, 168, 169, 170, 176, 177, 180, 189, 191, 196, 200, 201, 203, 211, 218, 225, 226, 229, 230, 231, 234, 240, 242 und 262 substituiert ist/sind und/oder eine oder mehrere Aminosäuren entweder zum N- oder zum C-Terminus in der Aminosäuresequenz, die durch SEQ ID NO: 1 im Sequenzprotokoll repräsentiert wird, hinzugefügt sind.

**4.** Polypeptid gemäß Anspruch 3, wobei eine oder mehrere Aminosäuresubstitutionen und/oder Aminosäureadditionen der Aminosäuresequenz, die durch SEQ ID NO: 1 im Sequenzprotokoll repräsentiert wird, eingebracht ist/sind,

ausgewählt aus der Gruppe, bestehend aus:

Substitution von Isoleucin oder Alanin für Nr. 2, Arginin für Nr. 5, Alanin für Nr. 8, Valin oder Threonin für Nr. 11, Threonin für Nr. 23, Tyrosin für Nr. 26, Histidin oder Leucin für Nr. 28, Leucin für Nr. 29, Serin für Nr. 30, Histidin für Nr. 32, Leucin für Nr. 33, Methionin für Nr. 34, Arginin für Nr. 40, Serin, Threonin, Valin oder Prolin für Nr. 45, Methionin oder Isoleucin für Nr. 46, Threonin für Nr. 48, Glycin für Nr. 61, Alanin oder Threonin für Nr. 62, Threonin für Nr. 64, Threonin für Nr. 65, Valin für Nr. 66, Serin oder Leucin für Nr. 68, Glutamin für Nr. 71, Phenylalanin für Nr. 72, Cystein oder Histidin für Nr. 75, Histidin für Nr. 78, Alanin für Nr. 79, Tyrosin für Nr. 80, Threonin für Nr. 81, Alanin für Nr. 83, Serin für Nr. 88, Serin für Nr. 90, Glycin für Nr. 95, Asparaginsäure oder Lysin für Nr. 96, Threonin für Nr. 100, Histidin für Nr. 103, Threonin oder Valin für Nr. 105, Threonin für Nr. 108, Isoleucin für Nr. 115, Threonin für Nr. 116, Glutaminsäure für Nr. 119, Glycin für Nr. 123, Histidin für Nr. 128, Asparagin für Nr. 130, Asparaginsäure für Nr. 133, Threonin für Nr. 137, Valin für Nr. 141, Alanin für Nr. 146, Serin für Nr. 152, Threonin für Nr. 164, Arginin für Nr. 168, Serin für Nr. 169, Arginin für Nr. 170, Valin für Nr. 176, Alanin für Nr. 177, Cystein für Nr. 180, Threonin für Nr. 189, Isoleucin für Nr. 191, Leucin für Nr. 196, Valin für Nr. 200, Alanin für Nr. 201, Glycin für Nr. 203, Valin für Nr. 211, Valin für Nr. 218, Serin für Nr. 225, Threonin oder Valin für Nr. 226, Valin für Nr. 229, Isoleucin für Nr. 230, Phenylalanin für Nr. 231, Leucin für Nr. 234, Alanin für Nr. 240, Threonin für Nr. 242 und Methionin für Nr. 262 und Addition des Tryptophans und Arginins zum C-Terminus.

**5.** Polypeptid gemäß Anspruch 4, umfassend eine oder mehrere eingebrachte Aminosäuresubstitutionen der Aminosäuresequenz, die durch SEQ ID NO: 1 im Sequenzprotokoll repräsentiert wird, ausgewählt aus der Gruppe, bestehend aus: Substitution von Isoleucin für Aminosäure Nr. 2, Tyrosin für Nr. 26, Histidin für Nr. 28, Methionin für Nr. 46, Glycin für Nr. 61, Serin für Nr. 68, Tyrosin für Nr. 80, Threonin für Nr. 81, Alanin für Nr. 83, Glycin für Nr. 95, Lysin für Nr. 96, Glutaminsäure für Nr. 119, Glycin für Nr. 123, Asparagin für Nr. 130, Serin für Nr. 152, Leucin für Nr. 196, Valin für Nr. 200, Isoleucin für Nr. 230 und Leucin für Nr. 234, und das verbesserte Stabilität in der Gegenwart von Chloridatomen im Vergleich zu einer Carbonylreduktase, bestehend aus der Aminosäuresequenz, die durch SEQ ID NO: 1 im Sequenzprotokoll repräsentiert wird, aufweist.

**6.** Polypeptid gemäß Anspruch 5, umfassend eine Aminosäuresubstitution oder -substitutionen, ausgewählt aus den folgenden (1) bis (17):

(1) Isoleucin für Nr. 2 und Methionin für Nr. 46,
(2) Arginin für Nr. 5, Threonin für Nr. 23, Threonin für Nr. 100 und Lysin für Nr. 201,
(3) Tyrosin für Nr. 26,
(4) Histidin für Nr. 28, Glycin für Nr. 61 und Leucin für Nr. 234,
(5) Serin für Nr. 68,
(6) Tyrosin für Nr. 80 und Asparagin für Nr. 130,
(7) Threonin für Nr. 81,
(8) Alanin für Nr. 83,
(9) Serin für Nr. 88,
(10) Glycin für Nr. 95,
(11) Lysin für Nr. 96,
(12) Threonin für Nr. 105 und Valin für Nr. 226,
(13) Glutaminsäure für Nr. 119 und Leucin für Nr. 196,
(14) Glycin für Nr. 123,
(15) Serin für Nr. 152,
(16) Valin für Nr. 200 und
(17) Isoleucin für Nr. 230,

eingebracht in die Aminosäuresequenz, die durch SEQ ID NO: 1 im Sequenzprotokoll repräsentiert wird.

**7.** Polypeptid gemäß Anspruch 4, umfassend eine oder mehrere Aminosäuresubstitutionen und/oder Aminosäureadditionen, ausgewählt aus der Gruppe, bestehend aus: Substitution von Isoleucin oder Alanin für Nr. 2, Arginin für Nr. 5, Alanin für Nr. 8, Threonin für Nr. 23, Tyrosin für Nr. 26, Histidin oder Leucin für Nr. 28, Leucin für Nr. 29, Serin für Nr. 30, Histidin für Nr. 32, Leucin für Nr. 33, Methionin für Nr. 34, Arginin für Nr. 40, Serin, Threonin, Valin oder Prolin für Nr. 45, Methionin oder Isoleucin für Nr. 46, Threonin für Nr. 48, Glycin für Nr. 61, Alanin oder Threonin für Nr. 62, Threonin für Nr. 65, Valin für Nr. 66, Serin oder Leucin für Nr. 68, Glutamin für Nr. 71, Phenylalanin für Nr. 72, Cystein oder Histidin für Nr. 75, Histidin für Nr. 78, Alanin für Nr. 79, Tyrosin für Nr. 80, Threonin für Nr. 81,

Serin für Nr. 88, Serin für Nr. 90, Glycin für Nr. 95, Asparaginsäure oder Lysin für Nr. 96, Threonin für Nr. 100, Histidin für Nr. 103, Valin für Nr. 105, Threonin für Nr. 108, Isoleucin für Nr. 115, Threonin für Nr. 116, Glycin für Nr. 123, Histidin für Nr. 128, Asparagin für Nr. 130, Asparaginsäure für Nr. 133, Threonin für Nr. 137, Valin für Nr. 141, Alanin für Nr. 146, Serin für Nr. 152, Threonin für Nr. 164, Arginin für Nr. 168, Serin für Nr. 169, Arginin für Nr. 170, Valin für Nr. 176, Alanin für Nr. 177, Cystein für Nr. 180, Threonin für Nr. 189, Isoleucin für Nr. 191, Valin für Nr. 200, Glycin für Nr. 203, Valin für Nr. 211, Valin für Nr. 218, Serin für Nr. 225, Threonin für Nr. 226, Valin für Nr. 229, Isoleucin für Nr. 230, Phenylalanin für Nr. 231, Leucin für Nr. 234, Alanin für Nr. 240, Threonin für Nr. 242 und Methionin für Nr. 262 und Addition des Tryptophans und Arginins zum C-Terminus, eingebracht in die Aminosäuresequenz, die durch SEQ ID NO: 1 im Sequenzprotokoll repräsentiert wird, und das verbesserte Beständigkeit gegen Reaktionshemmung durch Chloridionen im Vergleich zu einer Carbonylreduktase, bestehend aus der Aminosäuresequenz, die durch SEQ ID NO: 1 im Sequenzprotokoll repräsentiert wird, aufweist.

**8.** Polypeptid gemäß Anspruch 7, umfassend eine Aminosäureaddition und/oder eine Aminosäuresubstitution oder -substitutionen, ausgewählt aus den folgenden (1) bis (55):

(1) Alanin für Nr. 2, Arginin für Nr. 40 und Valin für Nr. 229,
(2) Isoleucin für Nr. 2,
(3) Isoleucin für Nr. 2, Valin für Nr. 11, Methionin für Nr. 46 und Threonin für Nr. 64,
(4) Isoleucin für Nr. 2, Threonin für Nr. 11 und Methionin für Nr. 46,
(5) Isoleucin für Nr. 2, Leucin für Nr. 28 und Methionin für Nr. 46,
(6) Isoleucin für Nr. 2, Leucin für Nr. 29, Histidin für Nr. 32 und Methionin für Nr. 46,
(7) Isoleucin für Nr. 2, Histidin für Nr. 32 und Methionin für Nr. 46,
(8) Isoleucin für Nr. 2, Threonin für Nr. 45 und Methionin für Nr. 46,
(9) Isoleucin für Nr. 2, Valin für Nr. 45 und Methionin für Nr. 46,
(10) Isoleucin für Nr. 2, Prolin für Nr. 45, Methionin für Nr. 46 und Arginin für Nr. 168,
(11) Isoleucin für Nr. 2, Serin für Nr. 45, Methionin für Nr. 46 und Serin für Nr. 90,
(12) Isoleucin für Nr. 2 und Isoleucin für Nr. 46,
(13) Isoleucin für Nr. 2 und Methionin für Nr. 46,
(14) Isoleucin für Nr. 2, Methionin für Nr. 46 und Threonin für Nr. 48,
(15) Isoleucin für Nr. 2, Methionin für Nr. 46 und Threonin für Nr. 62,
(16) Isoleucin für Nr. 2, Methionin für Nr. 46 und Threonin für Nr. 65,
(17) Isoleucin für Nr. 2, Methionin für Nr. 46 und Valin für Nr. 66,
(18) Isoleucin für Nr. 2, Methionin für Nr. 46 und Serin für Nr. 68,
(19) Isoleucin für Nr. 2, Methionin für Nr. 46 und Histidin für Nr. 78,
(20) Isoleucin für Nr. 2, Methionin für Nr. 46 und Alanin für Nr. 177,
(21) Arginin für Nr. 5, Threonin für Nr. 23, Threonin für Nr. 100 und Lysin für Nr. 201,
(22) Alanin für Nr. 8,
(23) Histidin für Nr. 28, Glycin für Nr. 61 und Leucin für Nr. 234,
(24) Histidin für Nr. 28, Alanin für Nr. 62, Alanin für Nr. 79 und Threonin für Nr. 164,
(25) Cystein für Nr. 29,
(26) Serin für Nr. 30 und Phenylalanin für Nr. 231,
(27) Leucin für Nr. 33,
(28) Methionin für Nr. 34,
(29) Methionin für Nr. 46,
(30) Methionin für Nr. 46 und Serin für Nr. 68,
(31) Alanin für Nr. 62, Isoleucin für Nr. 115, Valin für Nr. 141, Serin für Nr. 169, Cystein für Nr. 180, Threonin für Nr. 242 und Methionin für Nr. 262,
(32) Serin für Nr. 68,
(33) Leucin für Nr. 68 und Valin für Nr. 105,
(34) Glutamin für Nr. 71,
(35) Phenylalanin für Nr. 72 und Asparaginsäure für Nr. 133,
(36) Cystein für Nr. 75, Threonin für Nr. 137 und Glycin für Nr. 203,
(37) Histidin für Nr. 75 und Threonin für Nr. 226,
(38) Tyrosin für Nr. 80 und Asparagin für Nr. 130,
(39) Threonin für Nr. 81,
(40) Alanin für Nr. 83,
(41) Alanin für Nr. 83 und Threonin für Nr. 176,
(42) Serin für Nr. 88,

(43) Serin für Nr. 90,
(44) Glycin für Nr. 95,
(45) Asparaginsäure für Nr. 96 und Arginin für Nr. 170,
(46) Histidin für Nr. 103, Threonin für Nr. 189 und Isoleucin für Nr. 191,
(47) Threonin für Nr. 108, Alanin für Nr. 146, Threonin für Nr. 164 und Valin für Nr. 218,
(48) Threonin für Nr. 116 und Histidin für Nr. 128,
(49) Glutaminsäure für Nr. 119 und Leucin für Nr. 196,
(50) Glycin für Nr. 123,
(51) Serin für Nr. 152,
(52) Alanin für Nr. 201,
(53) Valin für Nr. 211 und Tryptophan für Nr. 264,
(54) Serin für Nr. 225 und Arginin für Nr. 264 und
(55) Alanin für Nr. 240,

eingebracht in die Aminosäuresequenz, die durch SEQ ID NO: 1 im Sequenzprotokoll repräsentiert wird.

**9.** Polypeptid gemäß Anspruch 4, umfassend eine oder mehrere Aminosäuresubstitutiönen und/oder Aminosäureadditionen, ausgewählt aus der folgenden Gruppe: Substitution von Isoleucin für Nr. 2, Alanin für Nr. 8, Valin oder Threonin für Nr. 11, Tyrosin für Nr. 26, Histidin für Nr. 28, Histidin für Nr. 32, Leucin für Nr. 33, Methionin für Nr. 34, Serin oder Threonin für Nr. 45, Methionin oder Isoleucin für Nr. 46, Threonin für Nr. 48, Glycin für Nr. 61, Threonin für Nr. 62, Threonin für Nr. 64, Threonin für Nr. 65, Valin für Nr. 66, Serin für Nr. 68, Glutamin für Nr. 71, Cystein oder Histidin für Nr. 75, Histidin für Nr. 78, Alanin für Nr. 79, Tyrosin für Nr. 80, Threonin für Nr. 81, Alanin für Nr. 83, Serin für Nr. 88, Serin für Nr. 90, Glycin für Nr. 95, Asparaginsäure oder Lysin für Nr. 96, Threonin für Nr. 116, Glutaminsäure für Nr. 119, Glycin für Nr. 123, Histidin für Nr. 128, Asparagin für Nr. 130, Threonin für Nr. 137, Serin für Nr. 152, Arginin für Nr. 170, Valin für Nr. 176, Alanin für Nr. 177, Leucin für Nr. 196, Valin für Nr. 200, Glycin für Nr. 203, Serin für Nr. 225, Threonin für Nr. 226, Isoleucin für Nr. 230 und Leucin für Nr. 234, eingebracht in die Aminosäuresequenz, die durch SEQ ID NO: 1 im Sequenzprotokoll repräsentiert wird, und das verbesserte Stabilität in der Gegenwart von organischen Chloriden im Vergleich zu einer Carbonylreduktase, bestehend aus der Aminosäuresequenz, die durch SEQ ID NO: 1 im Sequenzprotokoll repräsentiert wird, aufweist.

**10.** Polypeptid gemäß Anspruch 9, umfassend eine Aminosäuresubstitution oder -substitutionen, ausgewählt aus den folgenden (1) bis (44):

(1) Alanin für Nr. 2, Arginin für Nr. 40 und Valin für Nr. 229,
(2) Isoleucin für Nr. 2, Valin für Nr. 11, Methionin für Nr. 46 und Threonin für Nr. 64,
(3) Isoleucin für Nr. 2, Threonin für Nr. 11 und Methionin für Nr. 46,
(4) Isoleucin für Nr. 2, Leucin für Nr. 28 und Methionin für Nr. 46,
(5) Isoleucin für Nr. 2, Leucin für Nr. 29, Histidin für Nr. 32 und Methionin für Nr. 46,
(6) Isoleucin für Nr. 2, Histidin für Nr. 32 und Methionin für Nr. 46,
(7) Isoleucin für Nr. 2, Threonin für Nr. 45 und Methionin für Nr. 46,
(8) Isoleucin für Nr. 2, Valin für Nr. 45 und Methionin für Nr. 46,
(9) Isoleucin für Nr. 2, Prolin für Nr. 45, Methionin für Nr. 46 und Arginin für Nr. 168,
(10) Isoleucin für Nr. 2, Serin für Nr. 45, Methionin für Nr. 46 und Serin für Nr. 90,
(11) Isoleucin für Nr. 2 und Isoleucin für Nr. 46,
(12) Isoleucin für Nr. 2 und Methionin für Nr. 46,
(13) Isoleucin für Nr. 2, Methionin für Nr. 46 und Threonin für Nr. 48,
(14) Isoleucin für Nr. 2, Methionin für Nr. 46 und Threonin für Nr. 62,
(15) Isoleucin für Nr. 2, Methionin für Nr. 46 und Threonin für Nr. 65,
(16) Isoleucin für Nr. 2, Methionin für Nr. 46 und Valin für Nr. 66,
(17) Isoleucin für Nr. 2, Methionin für Nr. 46 und Serin für Nr. 68,
(18) Isoleucin für Nr. 2, Methionin für Nr. 46 und Histidin für Nr. 78,
(19) Isoleucin für Nr. 2, Methionin für Nr. 46 und Alanin für Nr. 177,
(20) Alanin für Nr. 8,
(21) Histidin für Nr. 28, Glycin für Nr. 61 und Leucin für Nr. 234,
(22) Histidin für Nr. 28, Alanin für Nr. 62, Alanin für Nr. 79 und Threonin für Nr. 164,
(23) Cystein für Nr. 29,
(24) Leucin für Nr. 33,
(25) Methionin für Nr. 34,

(26) Methionin für Nr. 46 und Serin für Nr. 68,
(27) Serin für Nr. 68,
(28) Glutamin für Nr. 71,
(29) Cystein für Nr. 75, Threonin für Nr. 137 und Glycin für Nr. 203,
(30) Histidin für Nr. 75 und Threonin für Nr. 226,
(31) Tyrosin für Nr. 80 und Asparagin für Nr. 130,
(32) Threonin für Nr. 81,
(33) Alanin für Nr. 83,
(34) Alanin für Nr. 83 und Valin für Nr. 176,
(35) Serin für Nr. 88,
(36) Serin für Nr. 90,
(37) Glycin für Nr. 95,
(38) Asparaginsäure für Nr. 96 und Arginin für Nr. 170,
(39) Threonin für Nr. 116 und Histidin für Nr. 128,
(40) Glutaminsäure für Nr. 119 und Leucin für Nr. 196,
(41) Glycin für Nr. 123,
(42) Serin für Nr. 152,
(43) Alanin für Nr. 201 und
(44) Serin für Nr. 225 und Arginin für Nr. 264,

eingebracht in die Aminosäuresequenz, die durch SEQ ID NO: 1 im Sequenzprotokoll repräsentiert wird.

**11.** Polypeptid gemäß Anspruch 9 oder 10, wobei das organische Chlorid ein Chlorketon ist.

**12.** Polypeptid gemäß Anspruch 11, wobei das Chlorketon entweder Chloraceton oder 3-Chlor-1-(2-thienyl)propan-1-on ist.

**13.** Polypeptid gemäß Anspruch 1 oder 2, welches zusätzlich zu den Eigenschaften (a) bis (c) Eigenschaft (d) aufweist, welche eine größere Affinität zu einem Coenzym ist als die einer Carbonylreduktase, bestehend aus der Aminosäuresequenz, die durch SEQ ID NO: 1 im Sequenzprotokoll repräsentiert wird.

**14.** Polypeptid gemäß Anspruch 13, wobei eine oder mehrere Aminosäuren, ausgewählt aus der Gruppe, bestehend aus den folgenden:

Aminosäuren Nr. 2, 46, 68 und 95,
in der Aminosäuresequenz, die durch SEQ ID NO: 1 im Sequenzprotokoll repräsentiert wird, substituiert ist/sind.

**15.** Polypeptid gemäß Anspruch 14, wobei eine oder mehrere Aminosäuresubstitutionen der Aminosäuresequenz, die durch SEQ ID NO: 1 im Sequenzprotokoll repräsentiert wird, eingebracht ist/sind, ausgewählt aus der Gruppe, bestehend aus den folgenden:

Isoleucin für Nr. 2, Methionin für Nr. 46, Serin für Nr. 68 und Glycin für Nr. 95.

**16.** Polynukleotid, welches das Polypeptid gemäß mindestens einem der Ansprüche 1 bis 15 codiert.

**17.** Vektor, welcher das Polynukleotid gemäß Anspruch 16 umfasst.

**18.** Vektor gemäß Anspruch 17, welcher ebenfalls ein Polynukleotid umfasst, welches ein Polypeptid codiert, das reduktive Coenzymregenerationsfähigkeit aufweist.

**19.** Transformante, erhalten durch Transformieren von Wirtszellen mit dem Vektor gemäß mindestens einem der Ansprüche 17 bis 18.

**20.** Verfahren zum Herstellen einer Alkoholverbindung, wobei das Polypeptid gemäß mindestens einem der Ansprüche 1 bis 15 oder die Transformante gemäß Anspruch 19 und/oder ein behandeltes Produkt davon auf eine Carbonylverbindung angewandt wird.

**21.** Herstellungsverfahren gemäß Anspruch 20, wobei die Carbonylverbindung ein asymmetrisches Keton ist und das

Produkt ein optisch aktiver Alkohol ist.

**22.** Herstellungsverfahren gemäß Anspruch 21, wobei die eine Carbonylgruppe aufweisende Verbindung ein asymmetrisches Keton ist, repräsentiert durch die folgende Formel (1):

[Chem. 1]

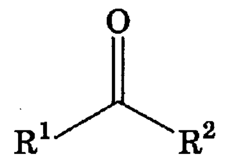

(1)

(worin $R^1$ und $R^2$ Wasserstoffatome, Halogenatome, optional substituierte Alkylgruppen, optional substituierte Aralkylgruppen, optional substituierte Arylgruppen, optional substituierte Alkoxygruppen, Aminogruppen oder Nitrogruppen sind, oder ansonsten $R^1$ und $R^2$ unter Bildung eines Rings aneinander binden; jedoch $R^1$ und $R^2$ unterschiedliche Strukturen aufweisen), und das Produkt ein optisch aktiver Alkohol ist, repräsentiert durch die folgende Formel (2):

[Chem. 2]

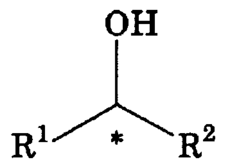

(2)

(worin $R^1$ und $R^2$ wie oben beschrieben sind und * einen asymmetrischen Kohlenstoff repräsentiert).

**23.** Herstellungsverfahren gemäß Anspruch 21, wobei die eine Carbonylgruppe aufweisende Verbindung 3-Chinuclidinon oder ein Salz davon, 3-Chlor-1-(2-thienyl)propan-1-on oder 3-Chlor-1-(2-phenyl)propan-1-on ist.

**24.** Verfahren zum Herstellen eines Bulk-Pharmazeutikums, wobei das Verfahren einen Schritt umfasst, bei dem das Polypeptid gemäß mindestens einem der Ansprüche 1 bis 15 oder die Transformante gemäß Anspruch 19 und/oder das behandelte Produkt davon auf eine Carbonylverbindung angewandt wird, um einen optisch aktiven Alkohol zu produzieren.

**Revendications**

**1.** Polypeptide possédant les propriétés (a) à (c) suivantes :

(a) au moins 97 % d'identité de séquence avec une séquence d'acides aminés représentée par SEQ ID NO : 1 dans la liste des séquences ;
(b) production de (R)-3-quinuclidinol par réduction de 3-quinuclidinone ou production de (S)-3-chloro-1-(2-thiényl)propan-1-ol par réduction de 3-chloro-1-(2-thiényl)propan-1-one ; et
(c) réactivité supérieure à des composés de carbonyle en présence d'un atome d'halogène comparativement à une carbonyl-réductase constituée de la séquence d'acides aminés représentée par SEQ ID NO : 1 dans la liste des séquences.

**2.** Polypeptide selon la revendication 1, dans lequel l'atome d'halogène est un atome de chlore.

**3.** Polypeptide selon la revendication 1 ou 2, dans lequel un ou plusieurs acides aminés choisis dans le groupe constitué des acides aminés No. 2, 5, 8, 11, 23, 26, 28, 29, 30, 32, 33, 34, 40, 45, 46, 48, 61, 62, 64, 65, 66, 68, 71, 72, 75, 78, 79, 80, 81, 83, 88, 90, 95, 96, 100, 103, 105, 108, 115, 116, 119, 123, 128, 130, 133, 137, 141, 146, 152, 164, 168, 169, 170, 176, 177, 180, 189, 191, 196, 200, 201, 203, 211, 218, 225, 226, 229, 230, 231, 234, 240, 242 et 262 est/sont substitué(s), et/ou un ou plusieurs acides aminés est/sont ajouté(s) à l'une ou l'autre extrémité N ou C dans la séquence d'acides aminés représentée par SEQ ID NO : 1 dans la liste des séquences.

**4.** Polypeptide selon la revendication 3, dans lequel les une ou plusieurs substitutions d'acides aminés et/ou additions d'acides aminés sont choisies dans le groupe constitué de :

la substitution d'isoleucine ou d'alanine au No. 2, d'arginine au No. 5, d'alanine au No. 8, de valine ou de thréonine au No. 11, de thréonine au No. 23, de tyrosine au No. 26, d'histidine ou de leucine au No. 28, de leucine au No. 29, de sérine au No. 30, d'histidine au No. 32, de leucine au No. 33, de méthionine au No. 34, d'arginine au No. 40, de sérine, de thréonine, de valine ou de proline au No. 45, de méthionine ou d'isoleucine au No. 46, de thréonine au No. 48, de glycine au No. 61, d'alanine ou de thréonine au No. 62, de thréonine au No. 64, de thréonine au No. 65, de valine au No. 66, de sérine ou de leucine au No. 68, de glutamine au No. 71, de phénylalanine au No. 72, de cystéine ou d'histidine au No. 75, d'histidine au No. 78, d'alanine au No. 79, de tyrosine au No. 80, de thréonine au No. 81, d'alanine au No. 83, de sérine au No. 88, de sérine au No. 90, de glycine au No. 95, d'acide aspartique ou de lysine au No. 96, de thréonine au No. 100, d'histidine au No. 103, de thréonine ou de valine au No. 105, de thréonine au No. 108, d'isoleucine au No. 115, de thréonine au No. 116, d'acide glutamique au No. 119, de glycine au No. 123, d'histidine au No. 128, d'asparagine au No. 130, d'acide aspartique au No. 133, de thréonine au No. 137, de valine au No. 141, d'alanine au No. 146, de sérine au No. 152, de thréonine au No. 164, d'arginine au No. 168, de sérine au No. 169, d'arginine au No. 170, de valine au No. 176, d'alanine au No. 177, de cystéine au No. 180, de thréonine au No. 189, d'isoleucine au No. 191, de leucine au No. 196, de valine au No. 200, d'alanine au No. 201, de glycine au No. 203, de valine au No. 211, de valine au No. 218, de sérine au No. 225, de thréonine ou de valine au No. 226, de valine au No. 229, d'isoleucine au No. 230, de phénylalanine au No. 231, de leucine au No. 234, d'alanine au No. 240, de thréonine au No. 242 et de méthionine au No. 262 et l'addition du tryptophane et de l'arginine à l'extrémité C introduites dans la séquence d'acides aminés représentée par SEQ ID NO : 1 dans la liste des séquences.

**5.** Polypeptide selon la revendication 4, comprenant une ou plusieurs substitutions d'acides aminés choisies dans le groupe constitué de : la substitution d'isoleucine à l'acide aminé No. 2, de tyrosine au No. 26, d'histidine au No. 28, de méthionine au No. 46, de glycine au No. 61, de sérine au No. 68, de tyrosine au No. 80, de thréonine au No. 81, d'alanine au No. 83, de glycine au No. 95, de lysine au No. 96, d'acide glutamique au No. 119, de glycine au No. 123, d'asparagine au No. 130, de sérine au No. 152, de leucine au No. 196, de valine au No. 200, d'isoleucine au No. 230 et de leucine au No. 234 introduite(s) dans la séquence d'acides aminés représentée par SEQ ID NO : 1 dans la liste des séquences, et présentant une stabilité améliorée en présence d'atomes de chlorure comparativement à une carbonyl-réductase constituée de la séquence d'acides aminés représentée par SEQ ID NO : 1 dans la liste des séquences.

**6.** Polypeptide selon la revendication 5, comprenant une substitution ou des substitutions d'acide(s) aminé(s) choisies parmi les (1) à (17) suivantes :

(1) d'isoleucine au No. 2 et de méthionine au No. 46,
(2) d'arginine au No. 5, de thréonine au No. 23, de thréonine au No. 100 et de lysine au No. 201,
(3) de tyrosine au No. 26,
(4) d'histidine au No. 28, de glycine au No. 61 et de leucine au No. 234,
(5) de sérine au No. 68,
(6) de tyrosine au No. 80 et d'asparagine au No. 130,
(7) de thréonine au No. 81,
(8) d'alanine au No. 83,
(9) de sérine au No. 88,
(10) de glycine au No. 95,
(11) de lysine au No. 96,
(12) de thréonine au No. 105 et de valine au No. 226,
(13) d'acide glutamique au No. 119 et de leucine au No. 196,
(14) de glycine au No. 123,
(15) de sérine au No. 152,
(16) de valine au No. 200 et
(17) d'isoleucine au No. 230

Introduite(s) dans la séquence d'acides aminés représentée par SEQ ID NO : 1 dans la liste des séquences.

**7.** Polypeptide selon la revendication 4, comprenant une ou plusieurs substitutions d'acides aminés et/ou additions d'acides aminés choisies dans le groupe constitué de :

la substitution d'isoleucine ou d'alanine au No. 2, d'arginine au No. 5, d'alanine au No. 8, de thréonine au No. 23, de tyrosine au No. 26, d'histidine ou de leucine au No. 28, de leucine au No. 29, de sérine au No. 30, d'histidine au No. 32, de leucine au No. 33, de méthionine au No. 34, d'arginine au No. 40, de sérine, de thréonine, de valine ou de proline au No. 45, de méthionine ou d'isoleucine au No. 46, de thréonine au No. 48, de glycine au No. 61, d'alanine ou de thréonine au No. 62, de thréonine au No. 65, de valine au No. 66, de sérine ou de leucine au No. 68, de glutamine au No. 71, de phénylalanine au No. 72, de cystéine ou d'histidine au No. 75, d'histidine au No. 78, d'alanine au No. 79, de tyrosine au No. 80, de thréonine au No. 81, de sérine au No. 88, de sérine au No. 90, de glycine au No. 95, d'acide aspartique ou de lysine au No. 96, de thréonine au No. 100, d'histidine au No. 103, de valine au No. 105, de thréonine au No. 108, d'isoleucine au No. 115, de thréonine au No. 116, de glycine au No. 123, d'histidine au No. 128, d'asparagine au No. 130, d'acide aspartique au No. 133, de thréonine au No. 137, de valine au No. 141, d'alanine au No. 146, de sérine au No. 152, de thréonine au No. 164, d'arginine au No. 168, de sérine au No. 169, d'arginine au No. 170, de valine au No. 176, d'alanine au No. 177, de cystéine au No. 180, de thréonine au No. 189, d'isoleucine au No. 191, de valine au No. 200, de glycine au No. 203, de valine au No. 211, de valine au No. 218, de sérine au No. 225, de thréonine au No. 226, de valine au No. 229, d'isoleucine au No. 230, de phénylalanine au No. 231, de leucine au No. 234, d'alanine au No. 240, de thréonine au No. 242 et de méthionine au No. 262 et l'addition du tryptophane et de l'arginine à l'extrémité C, introduites dans la séquence d'acides aminés représentée par SEQ ID NO : 1 dans la liste des séquences, et présentant une résistance améliorée à l'inhibition de réaction par des ions chlorure comparativement à une carbonyl-réductase constituée de la séquence d'acides aminés représentée par SEQ ID NO : 1 dans la liste des séquences.

**8.** Polypeptide selon la revendication 7, comprenant une addition d'acide aminé et/ou une substitution ou des substitutions d'acides aminés choisies parmi les (1) à (55) suivantes :

(1) d'alanine au No. 2, d'arginine au No. 40 et de valine au No. 229,
(2) d'isoleucine au No. 2,
(3) d'isoleucine au No. 2, de valine au No. 11, de méthionine au No. 46 et de thréonine au No. 64,
(4) d'isoleucine au No. 2, de thréonine au No. 11 et de méthionine au No. 46,
(5) d'isoleucine au No. 2, de leucine au No. 28 et de méthionine au No. 46,
(6) d'isoleucine au No. 2, de leucine au No. 29, d'histidine au No. 32 et de méthionine au No. 46,
(7) d'isoleucine au No. 2, d'histidine au No. 32 et de méthionine au No. 46,
(8) d'isoleucine au No. 2, de thréonine au No. 45 et de méthionine au No. 46,
(9) d'isoleucine au No. 2, de valine au No. 45 et de méthionine au No. 46,
(10) d'isoleucine au No. 2, de proline au No. 45, de méthionine au No. 46 et d'arginine au No. 168,
(11) d'isoleucine au No. 2, de sérine au No. 45, de méthionine au No. 46 et de sérine au No. 90,
(12) d'isoleucine au No. 2 et d'isoleucine au No. 46,
(13) d'isoleucine au No. 2 et de méthionine au No. 46,
(14) d'isoleucine au No. 2, de méthionine au No. 46 et de thréonine au No. 48,
(15) d'isoleucine au No. 2, de méthionine au No. 46 et de thréonine au No. 62,
(16) d'isoleucine au No. 2, de méthionine au No. 46 et de thréonine au No. 65,
(17) d'isoleucine au No. 2, de méthionine au No. 46 et de valine au No. 66,
(18) d'isoleucine au No. 2, de méthionine au No. 46 et de sérine au No. 68,
(19) d'isoleucine au No. 2, de méthionine au No. 46 et d'histidine au No. 78,
(20) d'isoleucine au No. 2, de méthionine au No. 46 et d'alanine au No. 177,
(21) d'arginine au No. 5, de thréonine au No. 23, de thréonine au No. 100 et de lysine au No. 201,
(22) d'alanine au No. 8,
(23) d'histidine au No. 28, de glycine au No. 61 et de leucine au No. 234,
(24) d'histidine au No. 28, d'alanine au No. 62, d'alanine au No. 79 et de thréonine au No. 164,
(25) de cystéine au No. 29,
(26) de sérine au No. 30 et de phénylalanine au No. 231,
(27) de leucine au No. 33,
(28) de méthionine au No. 34,
(29) de méthionine au No. 46,
(30) de méthionine au No. 46 et de sérine au No. 68,
(31) d'alanine au No. 62, d'isoleucine au No. 115, de valine au No. 141, de sérine au No. 169, de cystéine au No. 180, de thréonine au No. 242 et de méthionine au No. 262,
(32) de sérine au No. 68,
(33) de leucine au No. 68 et de valine au No. 105,

(34) de glutamine au No. 71,
(35) de phénylalanine au No. 72 et d'acide aspartique au No. 133,
(36) de cystéine au No. 75, de thréonine au No. 137 et de glycine au No. 203,
(37) d'histidine au No. 75 et de thréonine au No. 226,
(38) de tyrosine au No. 80 et d'asparagine au No. 130,
(39) de thréonine au No. 81,
(40) d'alanine au No. 83,
(41) d'alanine au No. 83 et de thréonine au No. 176,
(42) de sérine au No. 88,
(43) de sérine au No. 90,
(44) de glycine au No. 95,
(45) d'acide aspartique au No. 96 et d'arginine au No. 170,
(46) d'histidine au No. 103, de thréonine au No. 189 et d'isoleucine au No. 191,
(47) de thréonine au No. 108, d'alanine au No. 146, de thréonine au No. 164 et de valine au No. 218,
(48) de thréonine au No. 116 et d'histidine au No. 128,
(49) d'acide glutamique au No. 119 et de leucine au No. 196,
(50) de glycine au No. 123,
(51) de sérine au No. 152,
(52) d'alanine au No. 201,
(53) de valine au No. 211 et de tryptophane au No. 264,
(54) de sérine au No. 225 et d'arginine au No. 264 et
(55) d'alanine au No. 240,

introduites dans la séquence d'acides aminés représentée par SEQ ID NO : 1 dans la liste des séquences.

**9.** Polypeptide selon la revendication 4, comprenant une ou plusieurs substitutions d'acides aminés et/ou additions d'acides aminés choisies dans le groupe suivant :

la substitution d'isoleucine au No. 2, d'alanine au No. 8, de valine ou de thréonine au No. 11, de tyrosine au No. 26, d'histidine au No. 28, d'histidine au No. 32, de leucine au No. 33, de méthionine au No. 34, de sérine ou de thréonine au No. 45, de méthionine ou d'isoleucine au No. 46, de thréonine au No. 48, de glycine au No. 61, de thréonine au No. 62, de thréonine au No. 64, de thréonine au No. 65, de valine au No. 66, de sérine au No. 68, de glutamine au No. 71, de cystéine ou d'histidine au No. 75, d'histidine au No. 78, d'alanine au No. 79, de tyrosine au No. 80, de thréonine au No. 81, d'alanine au No. 83, de sérine au No. 88, de sérine au No. 90, de glycine au No. 95, d'acide aspartique ou de lysine au No. 96, de thréonine au No. 116, d'acide glutamique au No. 119, de glycine au No. 123, d'histidine au No. 128, d'asparagine au No. 130, de thréonine au No. 137, de sérine au No. 152, d'arginine au No. 170, de valine au No. 176, d'alanine au No. 177, de leucine au No. 196, de valine au No. 200, de glycine au No. 203, de sérine au No. 225, de thréonine au No. 226, d'isoleucine au No. 230 et de leucine au No. 234, introduite(s) dans la séquence d'acides aminés représentée par SEQ ID NO : 1 dans la liste des séquences, et présentant une stabilité améliorée en présence de chlorures organiques comparativement à une carbonyl-réductase constituée de la séquence d'acides aminés représentée par SEQ ID NO : 1 dans la liste des séquences.

**10.** Polypeptide selon la revendication 9, comprenant une substitution ou des substitutions d'acides aminés choisies parmi les (1) à (44) suivantes :

(1) d'alanine au No. 2, d'arginine au No. 40 et de valine au No. 229,
(2) d'isoleucine au No. 2, de valine au No. 11, de méthionine au No. 46 et de thréonine au No. 64,
(3) d'isoleucine au No. 2, de thréonine au No. 11 et de méthionine au No. 46,
(4) d'isoleucine au No. 2, de leucine au No. 28 et de méthionine au No. 46,
(5) d'isoleucine au No. 2, de leucine au No. 29, d'histidine au No. 32 et de méthionine au No. 46,
(6) d'isoleucine au No. 2, d'histidine au No. 32 et de méthionine au No. 46,
(7) d'isoleucine au No. 2, de thréonine au No. 45 et de méthionine au No. 46,
(8) d'isoleucine au No. 2, de valine au No. 45 et de méthionine au No. 46,
(9) d'isoleucine au No. 2, de proline au No. 45, de méthionine au No. 46 et d'arginine au No. 168,
(10) d'isoleucine au No. 2, de sérine au No. 45, de méthionine au No. 46 et de sérine au No. 90,
(11) d'isoleucine au No. 2 et d'isoleucine au No. 46,
(12) d'isoleucine au No. 2 et de méthionine au No. 46,

(13) d'isoleucine au No. 2, de méthionine au No. 46 et de thréonine au No. 48,
(14) d'isoleucine au No. 2, de méthionine au No. 46 et de thréonine au No. 62,
(15) d'isoleucine au No. 2, de méthionine au No. 46 et de thréonine au No. 65,
(16) d'isoleucine au No. 2, de méthionine au No. 46 et de valine au No. 66,
(17) d'isoleucine au No. 2, de méthionine au No. 46 et de sérine au No. 68,
(18) d'isoleucine au No. 2, de méthionine au No. 46 et d'histidine au No. 78,
(19) d'isoleucine au No. 2, de méthionine au No. 46 et d'alanine au No. 177,
(20) d'alanine au No. 8,
(21) d'histidine au No. 28, de glycine au No. 61 et de leucine au No. 234,
(22) d'histidine au No. 28, d'alanine au No. 62, d'alanine au No. 79 et de thréonine au No. 164,
(23) de cystéine au No. 29,
(24) de leucine au No. 33,
(25) de méthionine au No. 34,
(26) de méthionine au No. 46 et de sérine au No. 68,
(27) de sérine au No. 68,
(28) de glutamine au No. 71,
(29) de cystéine au No. 75, de thréonine au No. 137 et de glycine au No. 203,
(30) d'histidine au No. 75 et de thréonine au No. 226,
(31) de tyrosine au No. 80 et d'asparagine au No. 130,
(32) de thréonine au No. 81,
(33) d'alanine au No. 83,
(34) d'alanine au No. 83 et de valine au No. 176,
(35) de sérine au No. 88,
(36) de sérine au No. 90,
(37) de glycine au No. 95,
(38) d'acide aspartique au No. 96 et d'arginine au No. 170,
(39) de thréonine au No. 116 et d'histidine au No. 128,
(40) d'acide glutamique au No. 119 et de leucine au No. 196,
(41) de glycine au No. 123,
(42) de sérine au No. 152,
(43) d'alanine au No. 201 et
(44) de sérine au No. 225 et d'arginine au No. 264,

Introduite(s) dans la séquence d'acides aminés représentée par SEQ ID NO : 1 dans la liste des séquences.

**11.** Polypeptide selon la revendication 9 ou 10, dans lequel le chlorure organique est une chlorocétone.

**12.** Polypeptide selon la revendication 11, dans lequel la chlorocétone est soit la chlorocétone soit la 3-chloro-1-(2-thiényl)propan-1-one.

**13.** Polypeptide selon la revendication 1 ou 2, possédant, en plus des propriétés (a) à (c), la propriété (d) qui est une affinité supérieure avec une coenzyme par rapport à une carbonyl-réductase constituée de la séquence d'acides aminés représentée par SEQ ID NO : 1 dans la liste des séquences.

**14.** Polypeptide selon la revendication 13, dans lequel un ou plusieurs acides aminés choisis dans le groupe constitué des suivants :

les acides aminés No. 2, 46, 68 et 95,
est/ont substitué(s) dans la séquence d'acides aminés représentée par SEQ ID NO : 1 dans la liste des séquences.

**15.** Polypeptide selon la revendication 14, dans lequel une ou plusieurs substitutions d'acides aminés choisies dans le groupe constitué des suivantes :

d'isoleucine au No. 2, de méthionine au No. 46, de sérine au No. 68 et de glycine au No. 95,
est/sont introduite(s) dans la séquence d'acides aminés représentée par SEQ ID NO : 1 dans la liste des séquences.

**16.** Polynucléotide codant pour le polypeptide selon l'une quelconque des revendications 1 à 15.

**17.** Vecteur comprenant le polynucléotide selon la revendication 16.

**18.** Vecteur selon la revendication 17, comprenant également un polynucléotide codant pour un polypeptide possédant une capacité de régénération de coenzyme réductrice.

**19.** Transformant obtenu par transformation de cellules hôtes avec le vecteur selon l'une quelconque des revendications 17 à 18.

**20.** Procédé de fabrication d'un composé d'alcool, dans lequel le polypeptide selon l'une quelconque des revendications 1 à 15 ou le transformant selon la revendication 19 et/ou l'un de leurs produits traités est appliqué à un composé de carbonyle.

**21.** Procédé de fabrication selon la revendication 20, dans lequel le composé de carbonyle est une cétone asymétrique, et le produit est un alcool optiquement actif.

**22.** Procédé de fabrication selon la revendication 21, dans lequel le composé comportant un groupe carbonyle est une cétone asymétrique représentée par la formule (1) suivante :

[Chem. 1]

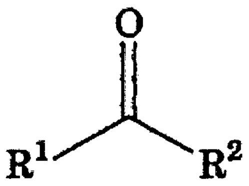

(1)

(dans laquelle $R^1$ et $R^2$ représentent des atomes d'hydrogène, des atomes d'halogène, des groupes alkyle éventuellement substitués, des groupes aralkyle éventuellement substitués, des groupes aryle éventuellement substitués, des groupes alcoxy éventuellement substitués, des groupes amino ou des groupes nitro, ou sinon $R^1$ et $R^2$ se lient l'un à l'autre pour former un cycle ; mais $R^1$ et $R^2$ ont des structures différentes), et le produit est un alcool optiquement actif représenté par la formule (2) suivante :

[Chem. 2]

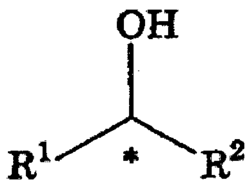

(2)

(où $R^1$ et $R^2$ sont tels que décrits ci-dessus, et * représente un atome de carbone asymétrique.

**23.** Procédé de fabrication selon la revendication 21, dans lequel le composé comportant un groupe carbonyle est la 3-quinuclidinone ou l'un de ses sels, la 3-chloro-1-(2-thiényl)propan-1-one, ou la 3-chloro-1-(2-phényl)propan-1-one.

**24.** Procédé de fabrication d'un produit pharmaceutique en vrac, le procédé comprenant une étape dans laquelle le polypeptide selon l'une quelconque des revendications 1 à 15 ou le transformant selon la revendication 19 et/ou leur produit traité est appliqué à un composé de carbonyle pour produire un alcool optiquement actif.

FIG.1

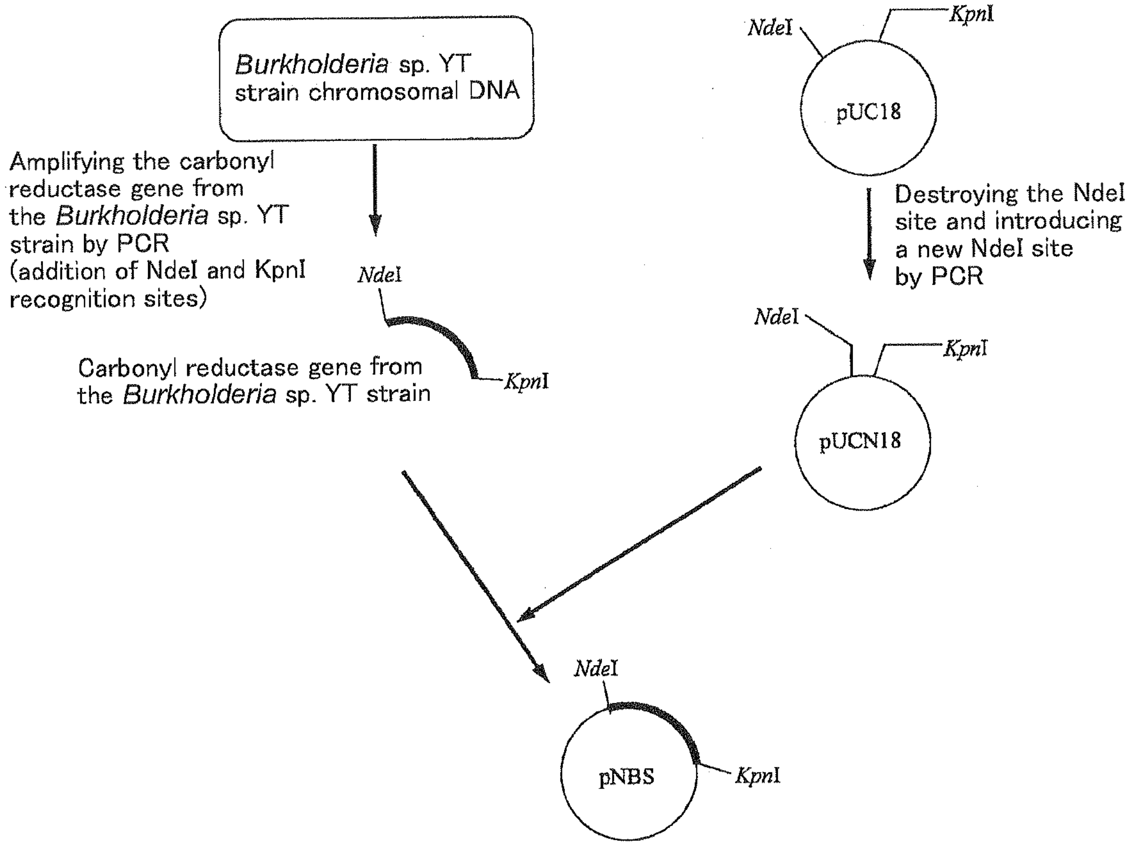
Burkholderia sp. YT strain chromosomal DNA
Amplifying the carbonyl reductase gene from the Burkholderia sp. YT strain by PCR (addition of NdeI and KpnI recognition sites)
NdeI
Carbonyl reductase gene from the Burkholderia sp. YT strain
KpnI
NdeI
KpnI
pUC18
Destroying the NdeI site and introducing a new NdeI site by PCR
NdeI
KpnI
pUCN18
NdeI
pNBS
KpnI

FIG.2

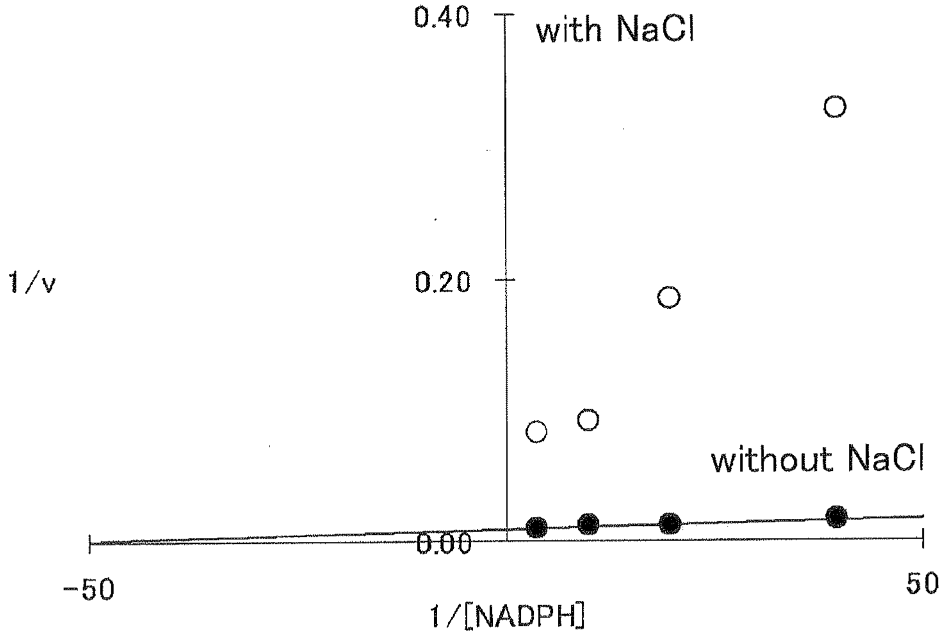
0.40
with NaCl
1/v
0.20
without NaCl
0.00
-50
50
1/[NADPH]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006061111 A
- WO 9403613 A
- JP 2010098698 A


### Non-patent literature cited in the description


- **UZURA A. et al.** *APPL MICROBIOL BIOTECHNOL.,* 21 February 2009, vol. 83, 617-626
- *J. Biosci. Bioeng.,* 2007, vol. 104, 416-419
- **JOSEPH SAMBROOK.** Molecular Cloning. Cold Spring Harbor Laboratory Press, 1989
- **LEUNG et al.** *Technique,* 1989, vol. 1, 11-15
- **FREDERICK M. AUSUBEL.** Current Protocols in Molecular Biology. Greene Publishing Associates and Wiley-Interscience, 1989
- **OLFERT LANDT et al.** *Gene,* 1990, vol. 96, 125-128
- **SMITH et al.** Genetic Engineering. Plenum Press, vol. 3, 1
- **VLASUK et al.** Experimental Manipulation of Gene Expression. Academic Press
- **HOS. N. HUN et al.** *Gene,* 1989, vol. 77, 51
- **TADAHIKO ANDO.** Biseibutsugaku Kiso Koza. Kyoritsu Publishing, 1987
- *Nature,* 1985, vol. 315, 592-594
- *J. Med. Chem.,* 2005, vol. 48, 6597-6606
- *J. Label. Compd. Radiopharm.,* 1995, vol. 34, 213-223
- **MURRAY et al.** *Nucl. Acids Res.,* 1980, vol. 8, 4321
- *Eur. J. Biochem.,* 1989, vol. 186, 389